# EUROPEAN PATENT APPLICATION

(11) **EP 4 386 081 A2**
(43) Date of publication of application: **19.06.2024**
(21) Application number: 24169043.7
(22) Date of filing: 20.05.2020
(51) Int. Cl.: C12N 5/071

(54) **ENDOCRINE DIFFERENTIATION-INDUCING MOLECULE**

(30) Priority: 21.05.2019 US 201962851085 P
(62) Divisional of application: 20810819.1
(71) Applicant: President and Fellows of Harvard College, Cambridge, MA 02138 (US)
(72) Inventor: SHARON, Nadav, Cambridge, 02138 (US); MELTON, Douglas, A., Lexington, 02421 (US)
(74) Representative: Lock, Graham James

(57) **Abstract**

Disclosed herein are methods for directing a differentiation protocol to produce cells of a desired cell fate, such as SC-β and/or SC-α cells.

## Description

### RELATED APPLICATIONS

This application claims the benefit of U.S. Provisional Application No. 62/851,085, filed on May 21, 2019, the contents of which are hereby incorporated by reference in its entirety.

### BACKGROUND OF THE INVENTION

Type 1 diabetes (T1D) is caused by autoimmune destruction of the insulin-producing β-cells in the pancreatic islets. Although transplantation of cadaveric islets can cure the disease¹, donor scarcity and the high cost of islet transplantation limits its feasibility. In an attempt to develop a ready supply of β cells for transplantation, several protocols for the *in vitro* differentiation of pluripotent cells into β cells were developed in recent years²⁻⁴. A protocol directs differentiation of human embryonic stem cells (hESCs) into β cells that resemble cadaveric β cells in both gene expression and function, including the ability to secrete Insulin in response to changing glucose levels³. Still, under these *in vitro* conditions, only about 30% of the generated cells are in fact β cells, and finding ways to increase the efficiency of the differentiation will be valuable.

An obstacle to protocol improvement is an incomplete understanding of the complex process of β cell differentiation. During normal embryonic development, the nascent pancreas contains a network of monolayered tubules comprised of epithelial progenitors, called epithelial cords (Pan and Wright, 2011). As cells in the cords divide, some turn on NEUROG3 and form "peninsulas" - bud like structures that grow and develop to become the islets (Sharon et al., 2019). Current protocols aim to recapitulate embryonic islet development by stepwise application of defined factors.

### SUMMARY OF THE INVENTION

*In vitro* differentiation of pluripotent cells into β cells is a promising alternative to cadaveric-islet transplantation as a cure for type 1diabetes (T1D). During the directed differentiation of human embryonic stem cells (hESCS) by exogenous factors, numerous genes that affect the differentiation process are turned on and off autonomously. Manipulating these reactions could increase the efficiency of differentiation and provide a more complete control over the final composition of cell populations. Here single cell RNA sequencing (scRNA-seq) is used to characterize the cell populations that appear during the *in vitro* differentiation process and identify pathways that affect β cell yield.

Disclosed herein are methods of increasing differentiation of pluripotent cells into pancreatic endocrine cells. The methods comprise contacting a population of stem cells with at least one WNT pathway inhibitor.

Also disclosed herein are methods of increasing differentiated endocrine cell yield. The methods comprise contacting a population of stem cells with at least one WNT pathway inhibitor.

Also disclosed herein are methods of directing differentiation of a population of cells. The methods comprise contacting a population of stem cells with at least one WNT pathway inhibitor.

In some embodiments, the WNT pathway inhibitor comprises a tankyrase inhibitor (e.g., IWR1-endo, XAV939, AZ6102, JW55, MN64, TC-E5001, WIKI4, or combinations thereof). In some embodiments, the population of stem cells comprises pancreatic bud cells.

In some embodiments, the population of stem cells is contacted with the WNT pathway inhibitor at stage 4 of a differentiation protocol. In some embodiments, the contacting of the population of stem cells with the WNT pathway inhibitor at stage 4 increases the number of CHGA⁺ cells by about 20%. In some embodiments, the contacting of the population of stem cells with the WNT pathway inhibitor at stage 4 decreases the number of PDX⁺NKX6.1⁺ progenitor cells by more than 30%. In some embodiments, the contacting of the population of stem cells with the WNT pathway inhibitor at stage 4 increases the yield of stem cell-derived alpha cells.

In some embodiments, the population of stem cells is contacted with the WNT pathway inhibitor at stage 5 of a differentiation protocol. In some embodiments, the contacting of the population of stem cells with the WNT pathway inhibitor at stage 5 increases the number of CHGA⁺ cells by about 50%. In some embodiments, the contacting of the population of stem cells with the WNT pathway inhibitor at stage 5 increases the number of C-PEP⁺ cells by more than 50%. The C-PEP⁺ cells may be C-PEP⁺/NKX6.1⁺ cells. In some embodiments, the contacting of the population of stem cells with the WNT pathway inhibitor at stage 5 increases the yield of stem cell-derived beta cells. In some embodiments, the population of stem cells is contacted with the WNT pathway inhibitor at stages 4 and 5 of a differentiation protocol.

In some embodiments, the methods further comprise contacting the population of stem cells with a BMP pathway inhibitor. In some embodiments, the population of stem cells are contacted with the BMP pathway inhibitor at stage 4 of a differentiation protocol. In some embodiments, the population of stem cells are contacted with the BMP pathway inhibitor at stage 5 of a differentiation protocol. In some embodiments, the population of stem cells are contacted with the BMP pathway inhibitor at stages 4 and 5 of a differentiation protocol.

Disclosed herein are methods of increasing differentiation of pluripotent cells into epithelial progenitor cells. The methods comprise contacting a population of stem cells with at least one BMP pathway activator.

Also disclosed herein are methods of increasing differentiated epithelial progenitor cell yield. The methods comprise contacting a population of stem cells with at least one BMP pathway activator.

Also disclosed herein are methods of directing differentiation of a population of cells comprising contacting a population of stem cells with at least one BMP pathway activator.

In some embodiments, the BMP pathway activator is selected from BMP2, BMP7, BMP9, GDF5, BMP2/6, BMP2/7, BMP4/7, or combinations thereof. In some embodiments, the population of stem cells comprises pancreatic bud cells.

In some embodiments, the population of stem cells is contacted with the BMP pathway activator at stage 4 of a differentiation protocol. In some embodiments, the contacting of the population of stem cells with the BMP pathway activator at stage 4 decreases the number of CHGA⁺ cells by about two fold. In some embodiments, the contacting of the population of stem cells with the BMP pathway activator at stage 4 increases the number of PDX⁺NKX6.1⁺ progenitor cells by more than two fold. In some embodiments, the contacting of the population of stem cells with the BMP pathway activator at stage 4 increases the yield of epithelial progenitor cells.

In some embodiments, the methods further comprise contacting the population of stem cells with a WNT pathway activator.

### BRIEF DESCRIPTION OF THE DRAWINGS

The patent or application file contains at least one drawing executed in color. Copies of this patent or patent application publication with color drawings will be provided by the Office upon request and payment of the necessary fee.

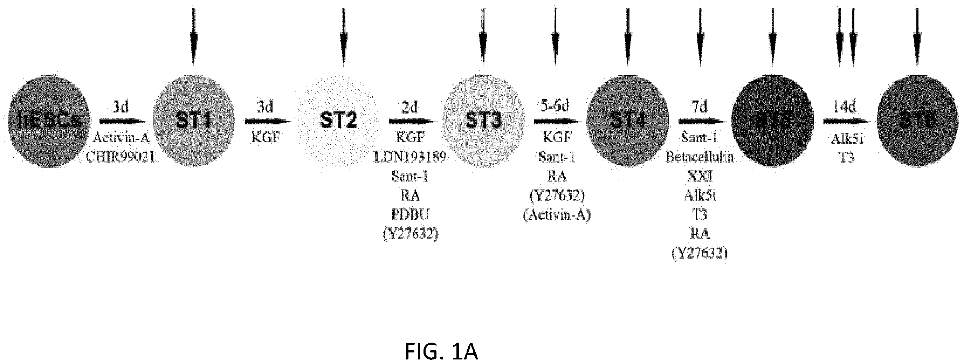

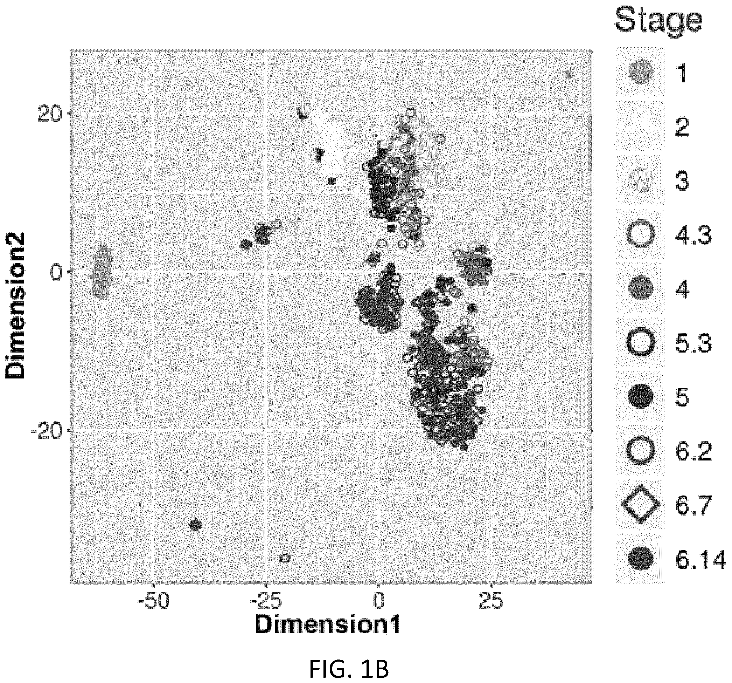

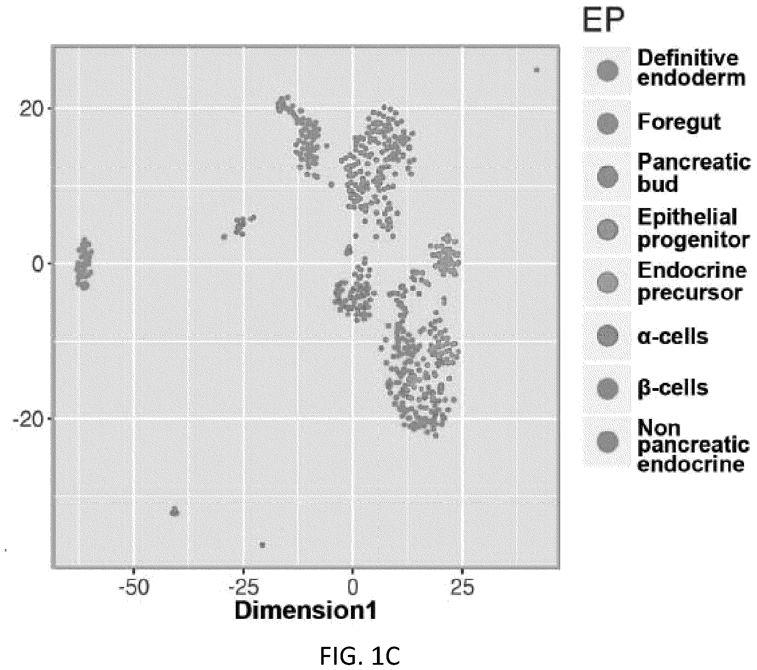

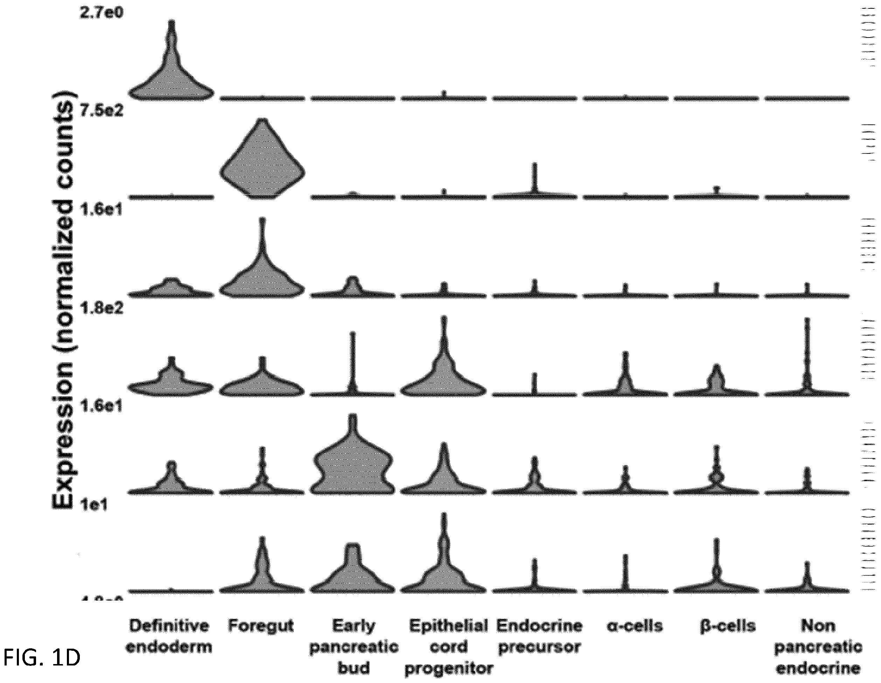

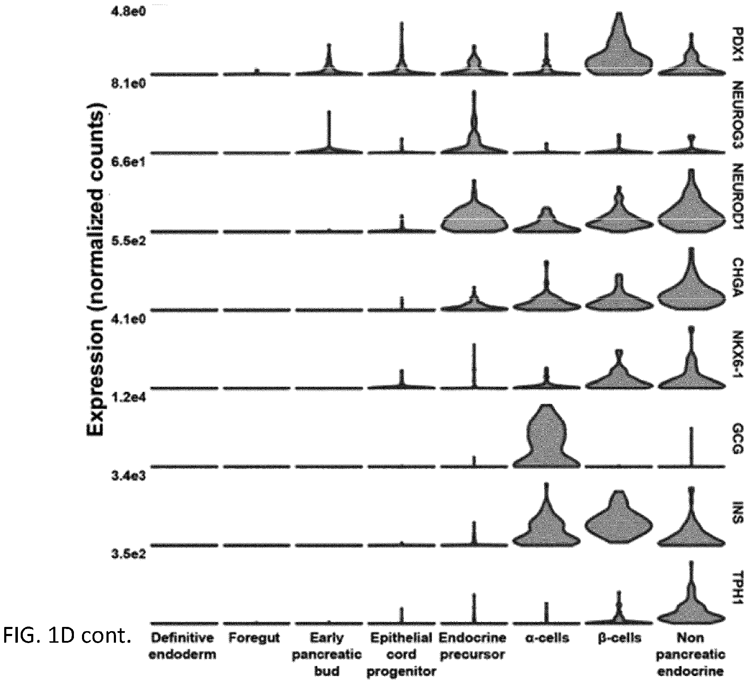

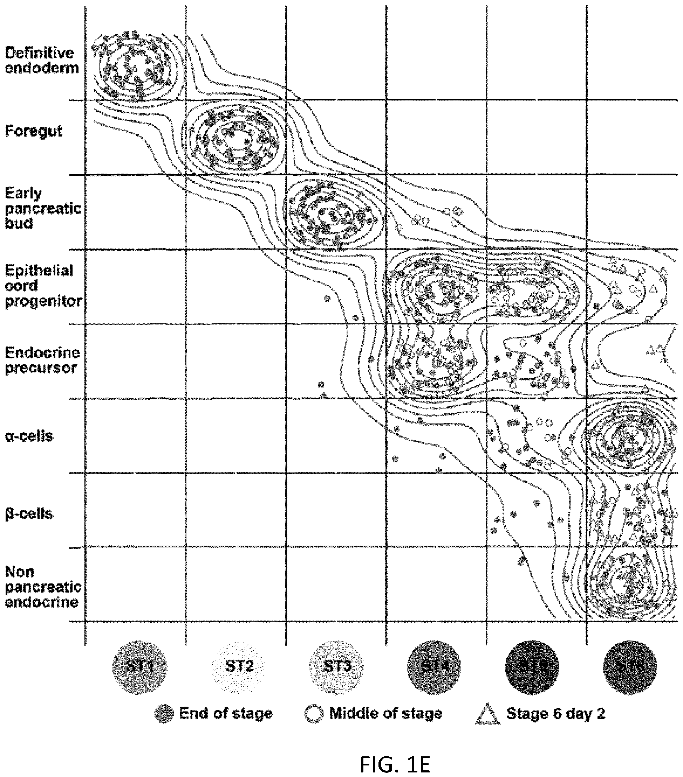

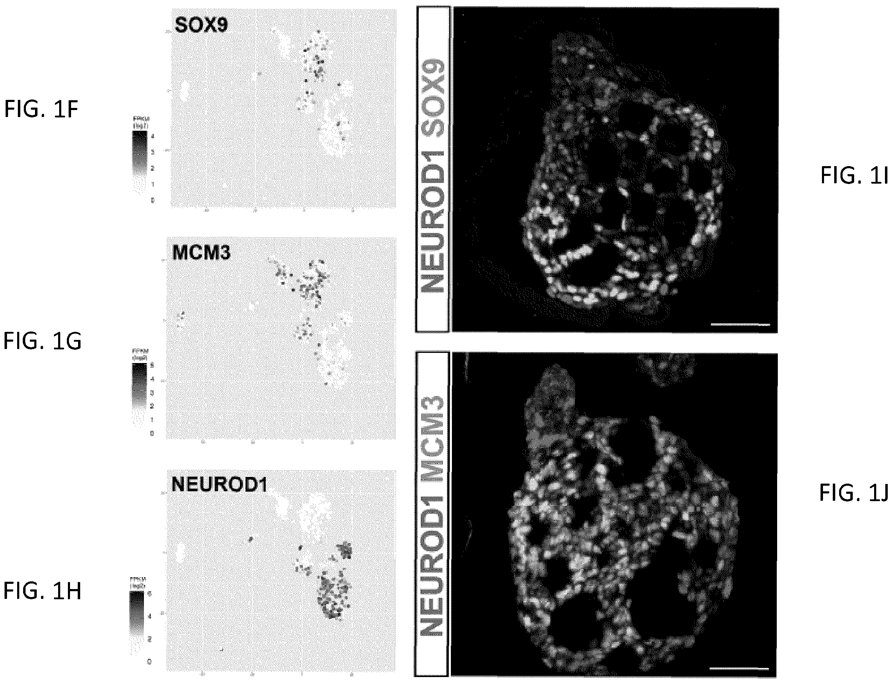

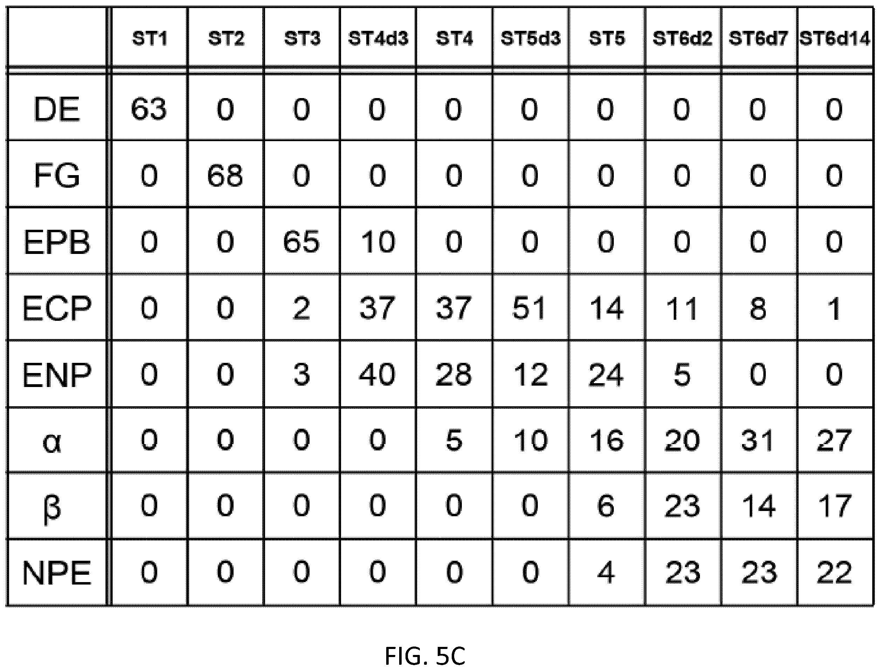

FIGS. 1A-1J demonstrate single cell RNA-seq of analysis of the directed differentiation of β cells *in vitro.* FIG. 1A provides an overview of the protocol for the directed differentiation of hESCs into β cells (Pagliuca et al., 2014). Cells were collected from undifferentiated hESCs , at the end of stages 1 through 5, on the 3^{rd} day of stages 4 and 5, and on days 2,7 and 14 of stage 6. FIG. 1B provides SIMLR visualization of the 720 differentiating cells analyzed (excluding hESCs). Colors represent time of collection. FIG. 1C provides a SIMLR plot, with colors representing the most highly used cell-type EP. FIG. 1D provides violin plots representing gene expression distributions within the cell-groups defined by cell-type EPs. FIG. 1E provides a density based topographic map overlaid on top of a bins-table to emphasize the changes that occur in the identity of the cells during *in vitro* differentiation. Cells are binned based on stage of collection (columns) and developmental identity (rows), and randomly dispersed within each bin. For a numerical summary of the bins-table, see FIG. 5C. FIGS. 1F-1H provide a SIMLR map overlaid with the relative expression level of the indicated gene in each cell. FIGS. 1I-1J show immunofluorescent staining of clusters at the end of stage 4. Scale bar: 50µm.

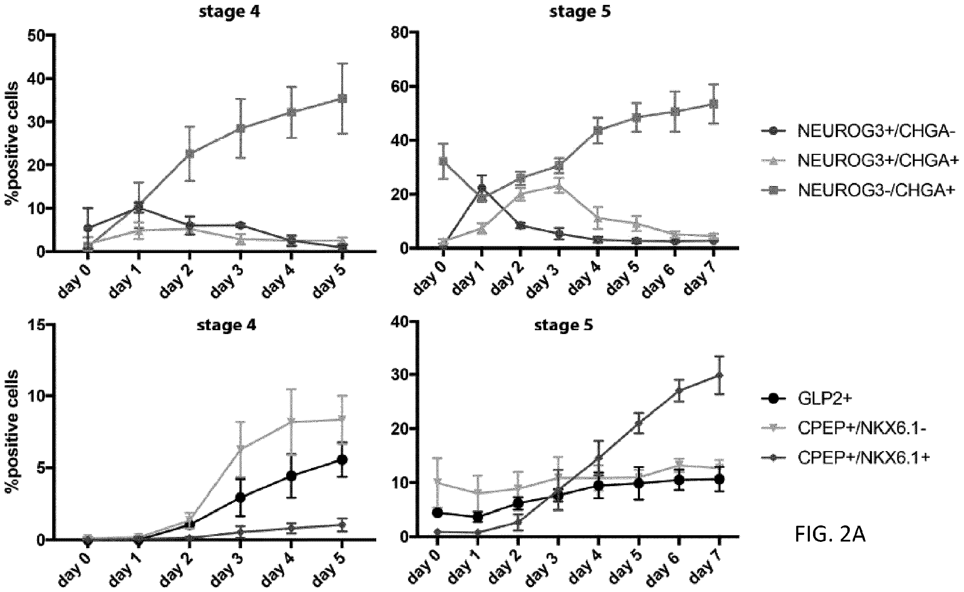

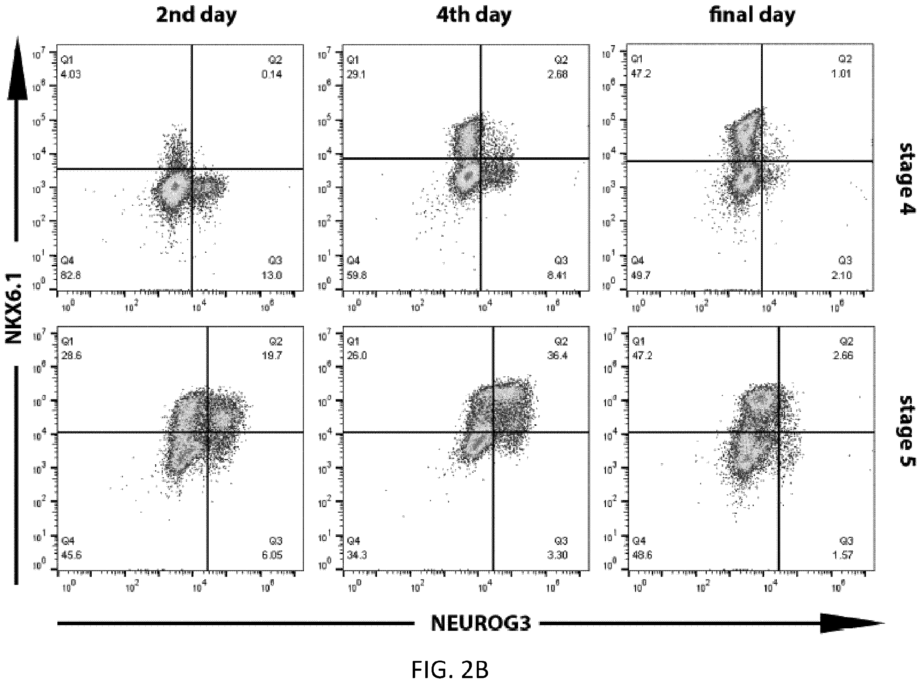

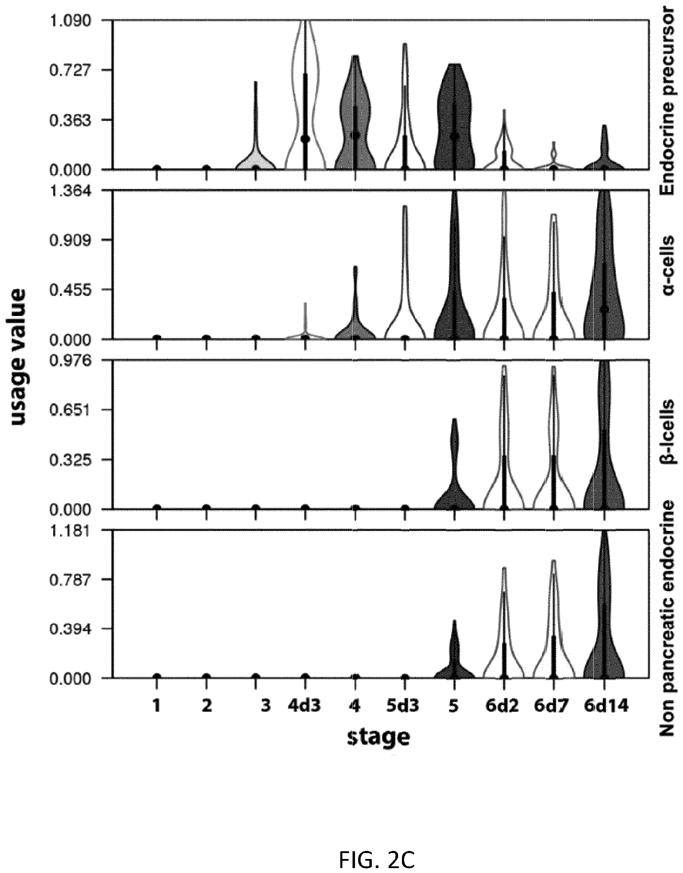

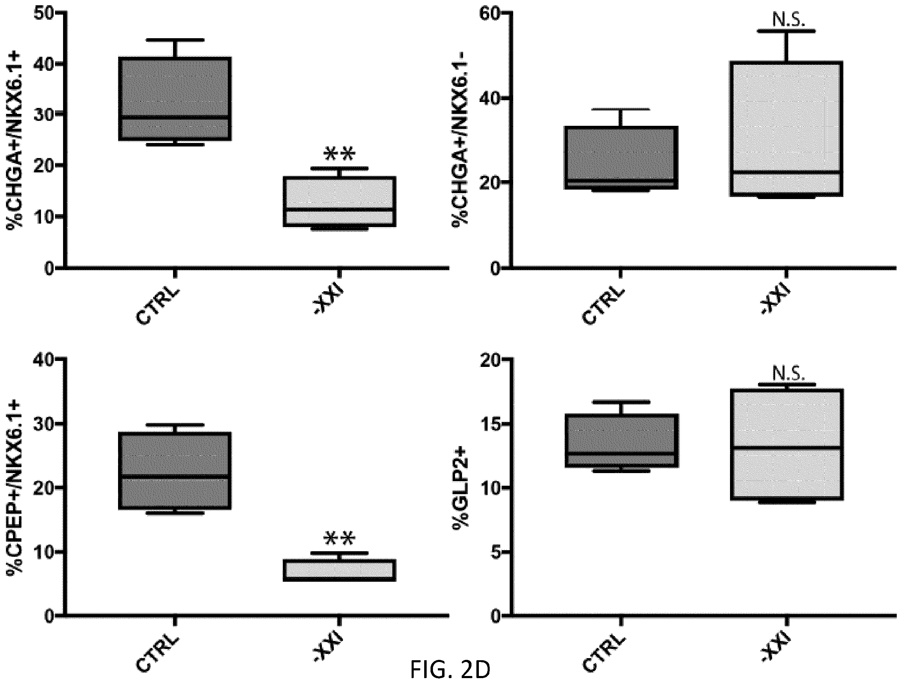

FIGS. 2A-2D demonstrate stages 4 and 5 produce different endocrine cell types. FIG. 2A provides daily flow cytometry analysis of endocrine induction during stages 4 and 5. n=3, data points represent mean±SD. FIG. 2B provides representative flow cytometry plots show the relationship between NKX6.1 and NEUROG3⁺ expression during stages 4 and 5. FIG. 2C provides violin plots that present the usage value distributions of endocrine EPs in cells collected in different stages. FIG. 2D provides flow cytometry analysis on cells at the end of stage 5, with (control) or without XXI. n=4, p-values from top left, clockwise: 0.0022, 0.3933, 0.1848, 0.0066, Student's T test.

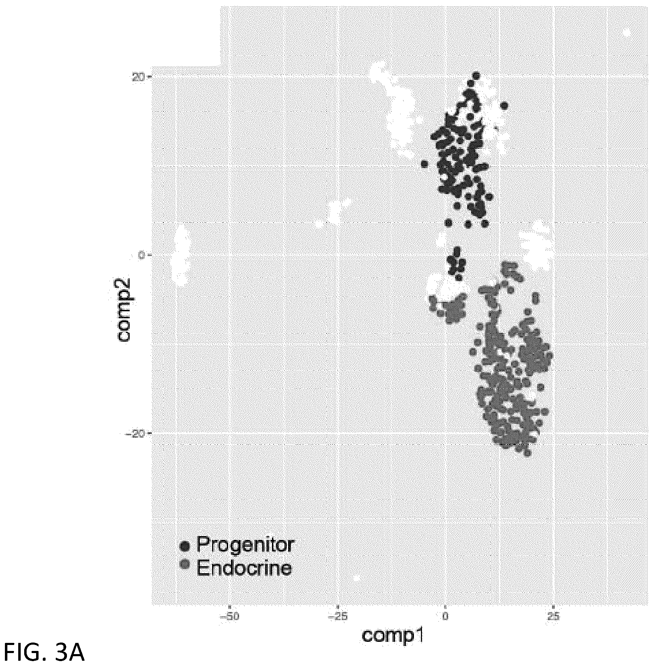

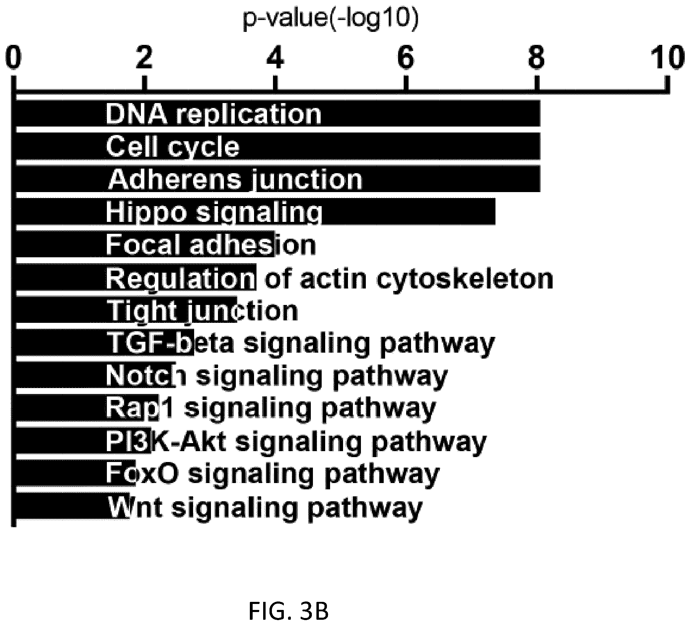

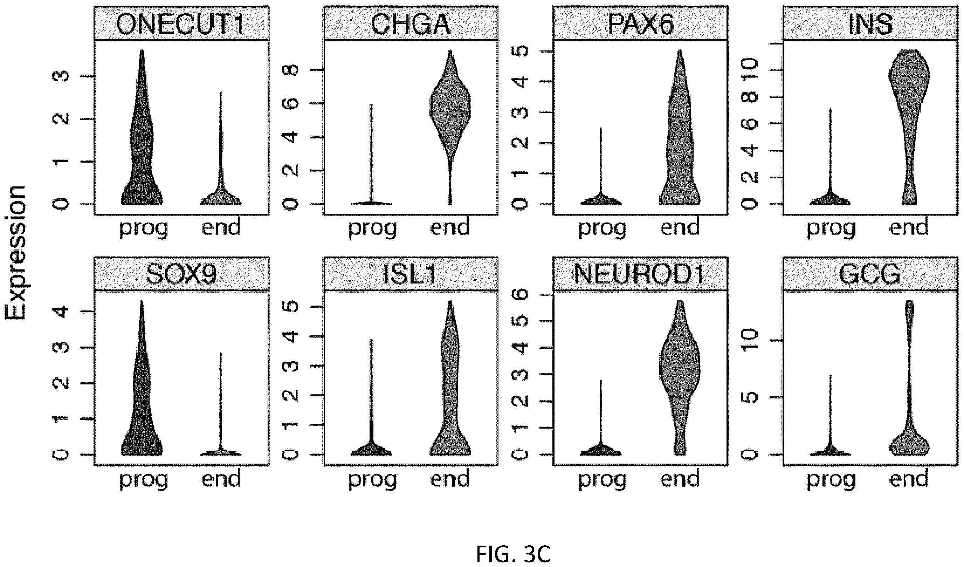

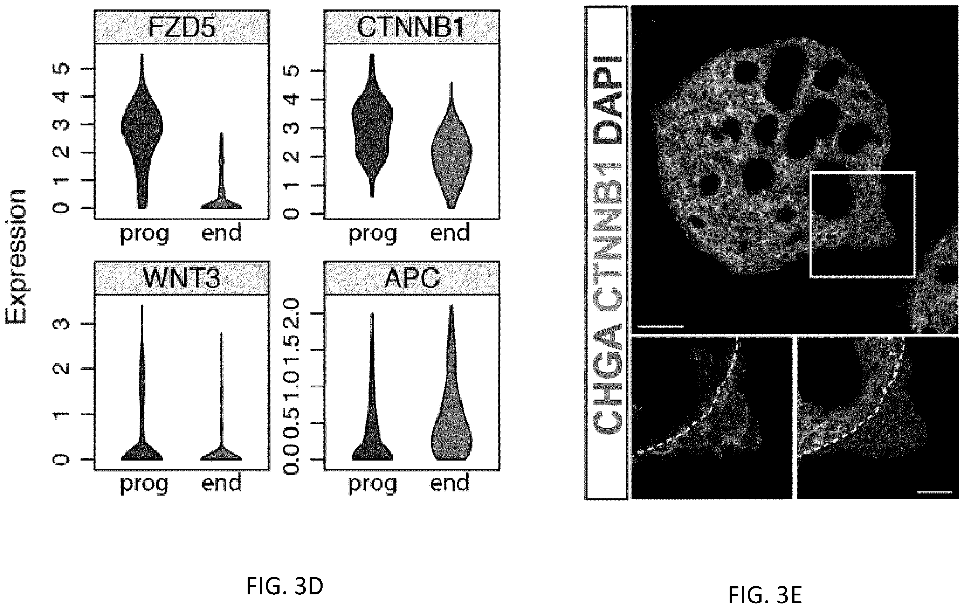

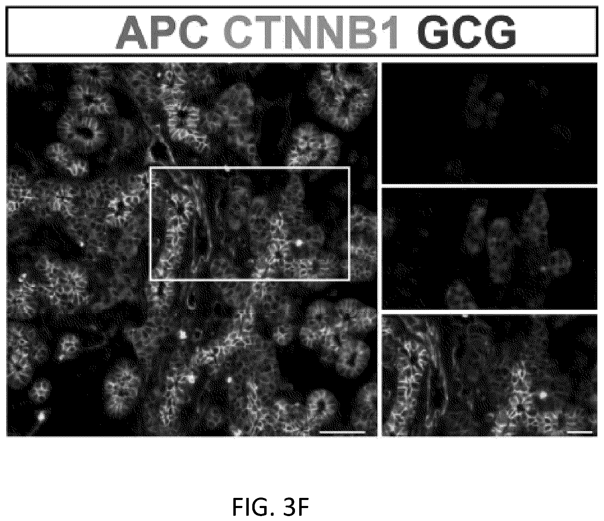

FIGS. 3A-3F demonstrate identification of genetic pathways that separate progenitors and endocrine cells. FIG. 3A provides a SIMLR map presenting the cells used for differential expression analysis. Cells with transitory intermediate characteristics were omitted to obtain clearer distinction between the populations. FIG. 3B shows representative gene categories enriched in the progenitor compartment. FIGS. 3C-3D provide violin plots showing the expression distributions of genes in progenitor (prog) and in endocrine (end) cells. FIG. 3E shows immunofluorescent staining of clusters at the end of stage 4. Dashed line marks the base of the endocrine bud. scale bar: 50µM. FIG. 3F shows immunofluorescent staining of a mouse embryonic pancreas at E15.5 scale bar: 25µm.

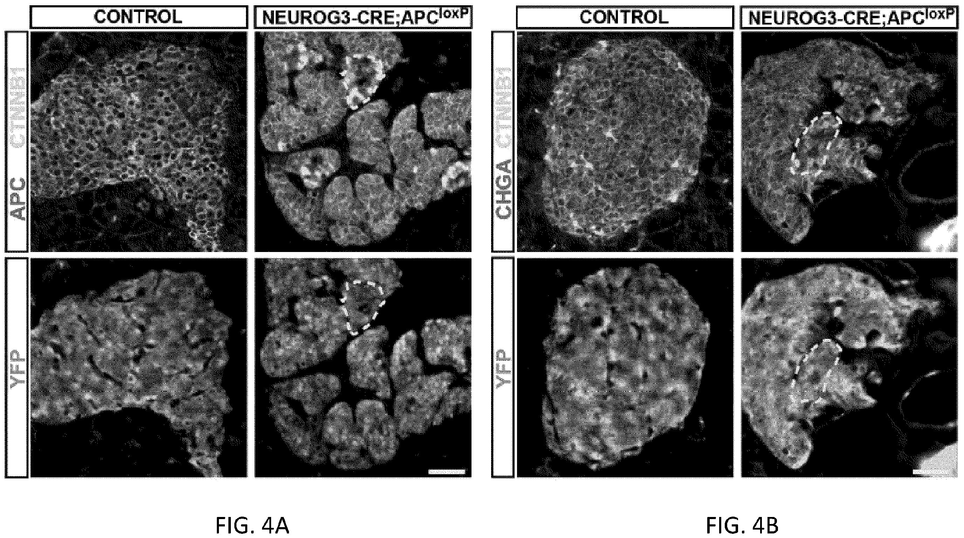

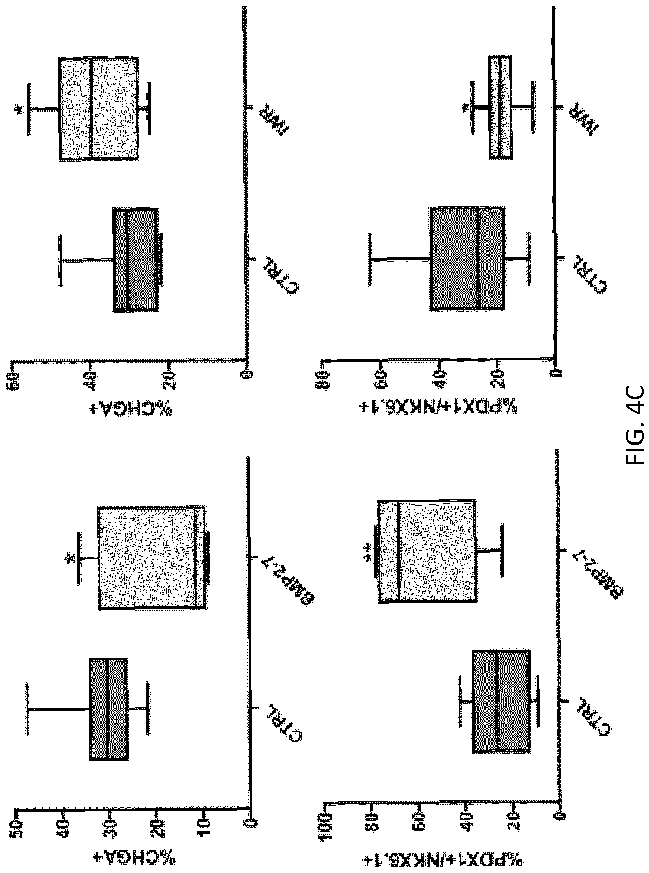

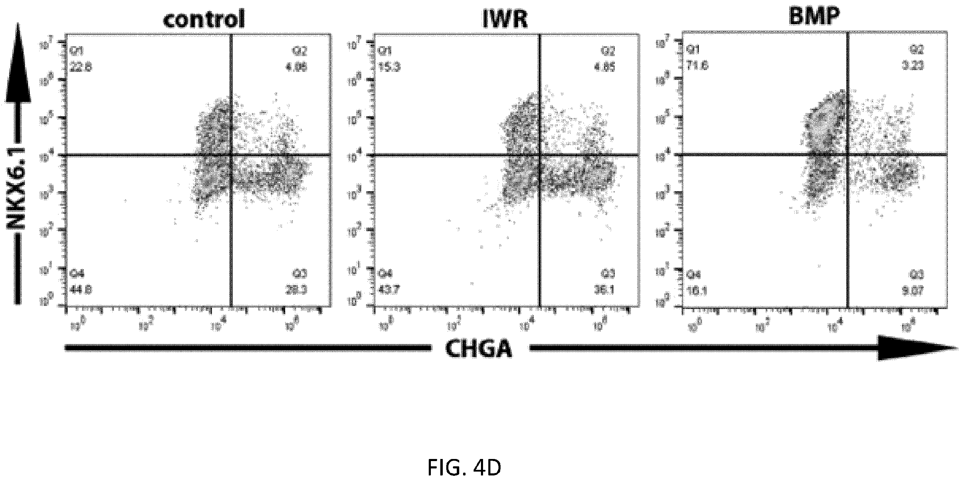

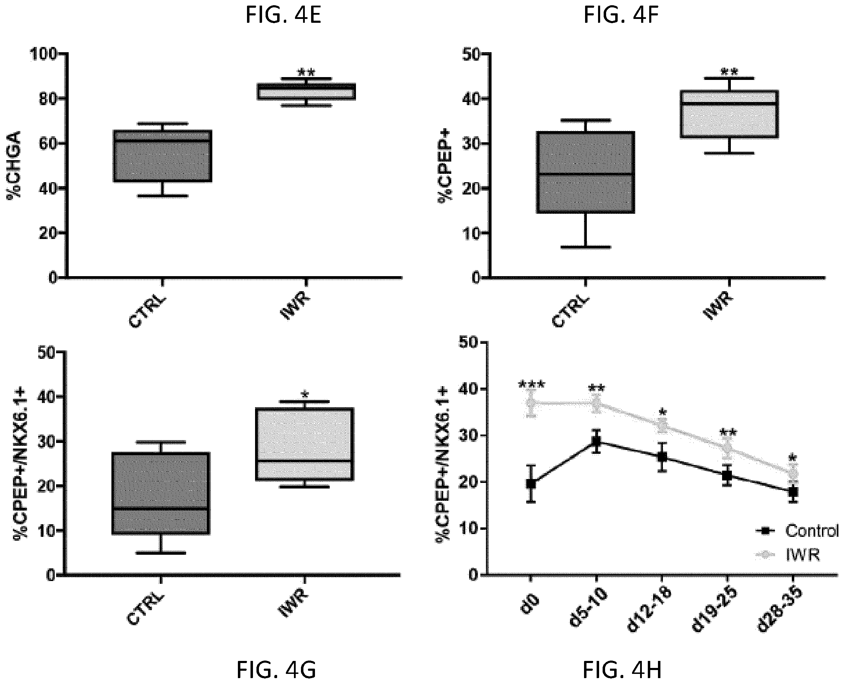

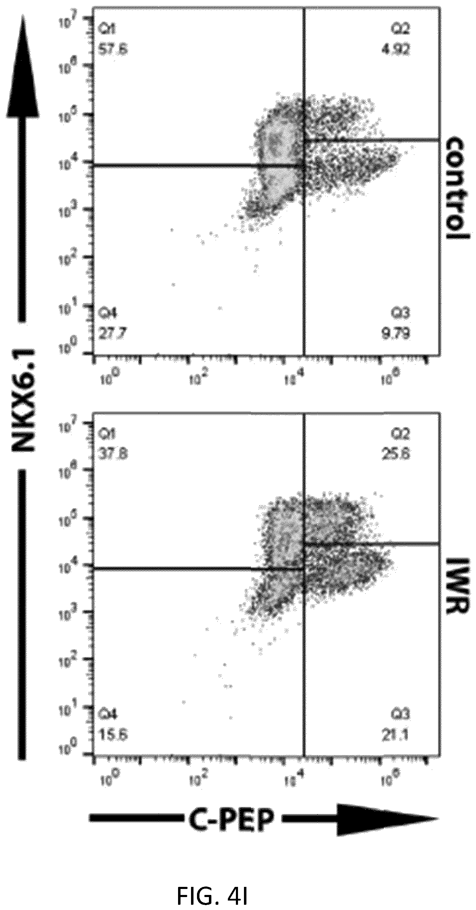

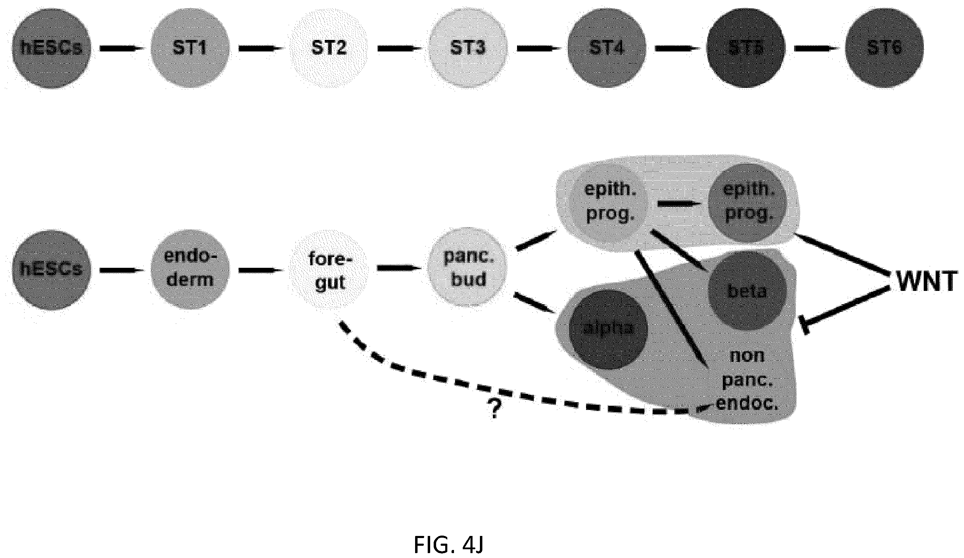

FIGS. 4A-4J demonstrate WNT pathway inhibition in endocrine differentiation. FIGS. 4A-4B shows immunofluorescent staining of pancreata from 1 month old Neurog3-cre female mouse (control) and 2 month old Neurog3-CRE;APC^{loxP/loxP} (APC-endocrine-KO) female mouse. Both mice bear a loxP-stop-loxP-YFP sequence in their ROSA26 locus, to trace cells that activated CRE. Notice that APC was not deleted in all YFP⁺ cells. Scale bar: 25µm. FIG. 4C provides flow cytometry analysis at the end of stage 4 following treatment with BMP2-7. n=7. Top: p-value=0.0235. Bottom: p-value=0.0098, Student's T test (left side) and following treatment with IWR1-endo. n=9. Top: p-value=0.0078. Bottom: p-value=0.0314, Student's T test (right side). FIG. 4D provides representative flow cytometry plots at the end of stage 4. FIGS. 4E-4G provide flow cytometry analysis at the end of stage 5 following treatment with IWR1-endo. (FIG. 4E) n=5, p-value=0.0045. (FIG. 4F) n=5, p-value=0.0037. (FIG. 4G) n=5, p-value=0.0138. Student's T test. FIG. 4H provides flow cytometry analysis of stage 6 cells, following treatment with IWR1-endo during stage 5. Values present mean±SEM. d - days into stage 6, d0 - last day of stage 5. 2-way ANOVA: p-value<10⁻⁴ for change in β cell proportion over days, and for overall difference in β cell proportion between conditions. Time-point-specific differences: d0: n=8, p-value=1.6×10⁻⁴. d5-10: n=7, p-value=0.002. d12-18: n=7, p-value=0.03. d19-25: n=7, p-value=0.001. d28-35: n=7, p-value=0.03. Student's t-test. FIG. 4I provides representative flow cytometry plots at the end of stage 5. FIG. 4J shows an overview of the protocol (top) and the actual dynamics of cell differentiation *in vitro* (bottom).

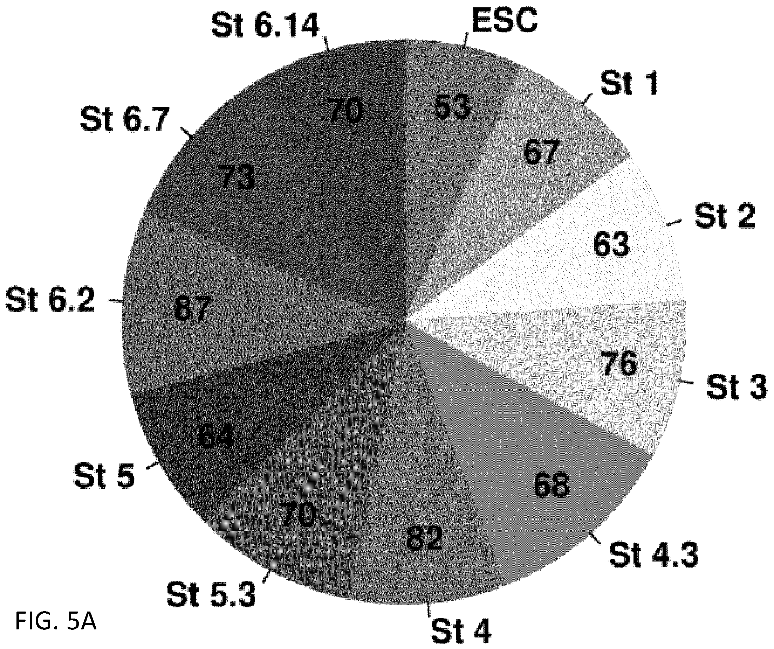

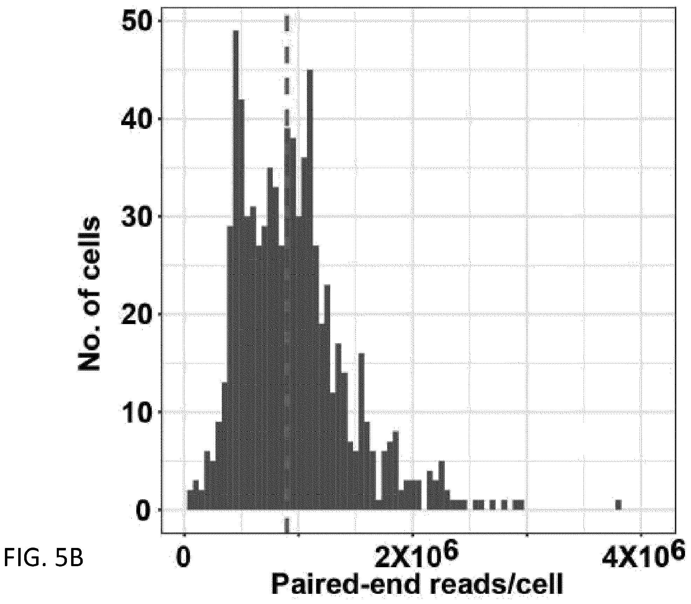

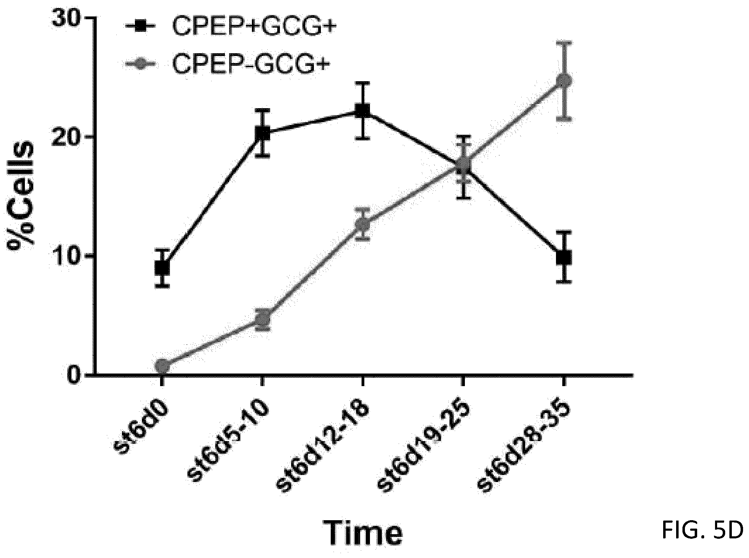

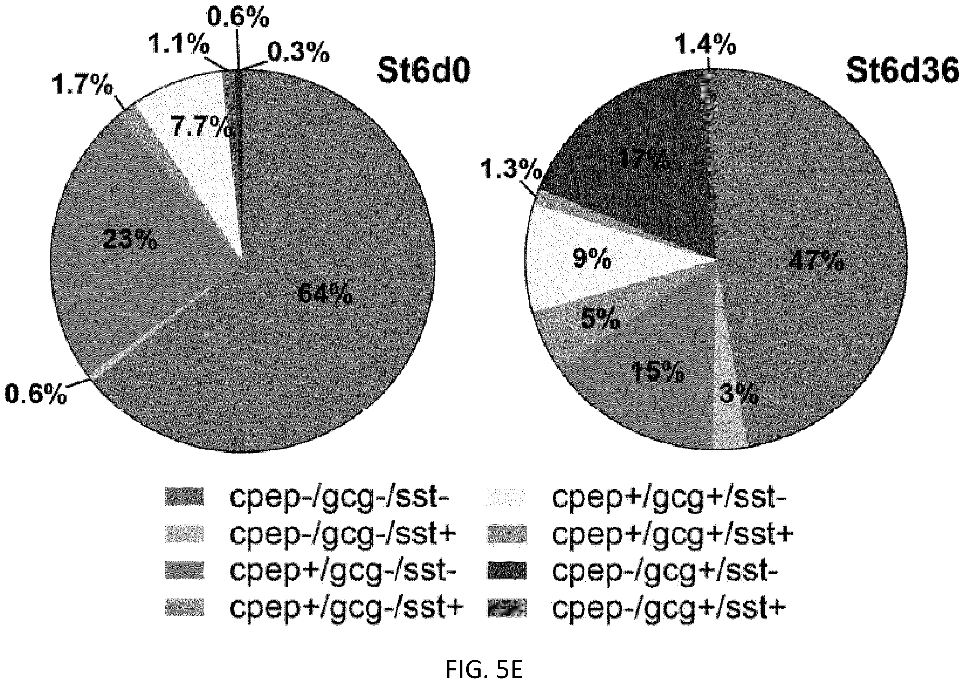

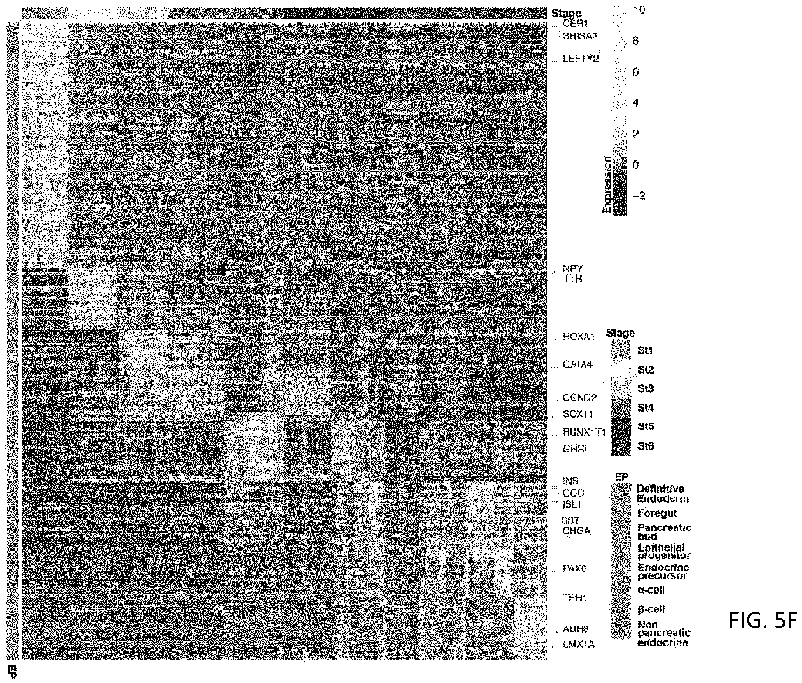

FIGS. 5A-5F demonstrate single cell RNA-seq of analysis of the directed differentiation of β cells. FIG. 5A shows the number of single cells analyzed at each time point. Altogether, 773 cells were analyzed. FIG. 5B shows distribution of usable reads. Median = 898,228 paired-end reds per cell. FIG. 5C provides a numerical summary of the plot presented in FIG. 1E, showing the number of cells collected at each stage and their classification into various cell types. DE - definitive endoderm; FG - Foregut; EPB - early pancreatic bud; ECP - epithelial cord progenitor; ENP - endocrine precursor; NPE - non-pancreatic endocrine. FIG. 5D provides flow cytometry analysis at consecutive time intervals during stage 6 of the protocol showing that after an initial increase in their proportion, CPEP⁺GCG⁺ cells decline whereas CPEP⁻GCG⁺ cell proportions rise constantly. Values present mean±SEM. st6d0 (last day of stage 5) n=8, all other time intervals n=7. FIG. 5E shows representative distribution of hormone co-expression as determined using flow cytometry at the end of stage 5 (St6d0) and following 5 weeks in stage 6 medium (St6d36). The plots are from two independent differentiation batches. FIG. 5F shows relative expression levels of the top genes in each of the major 8 expression profiles (rows), across all cells (columns). Columns are grouped according to differentiation stages. Rows are grouped according to EPs, and every gene appears once, in the EP with the highest relevance value.

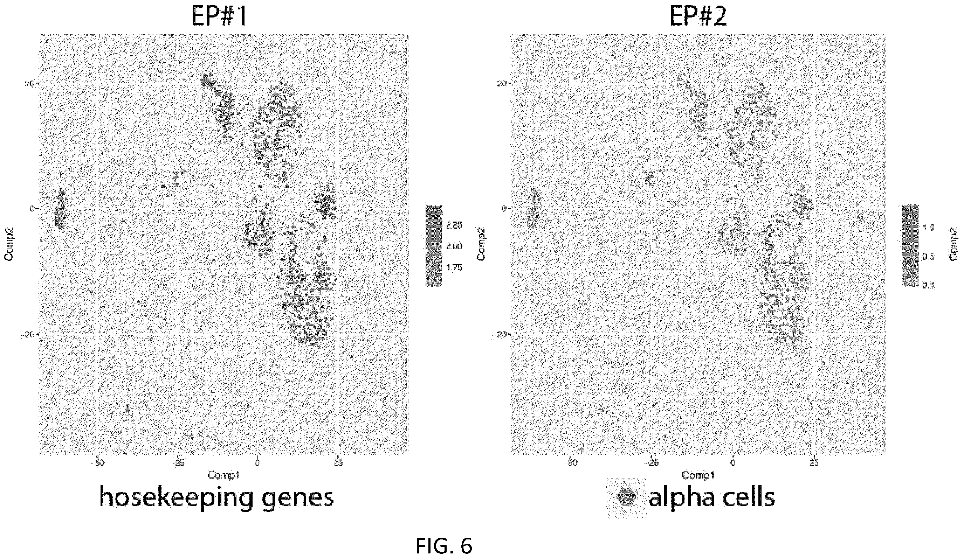

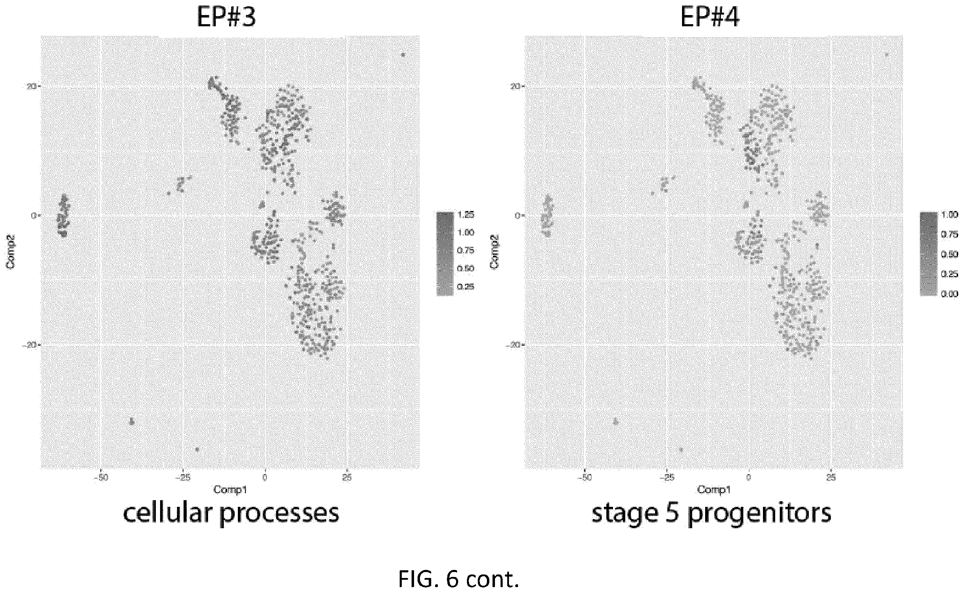

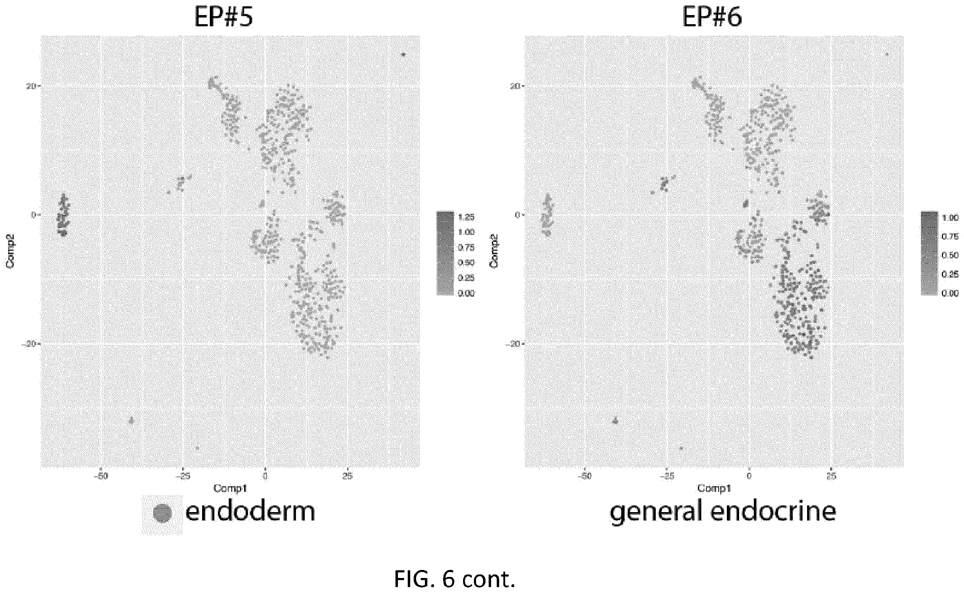

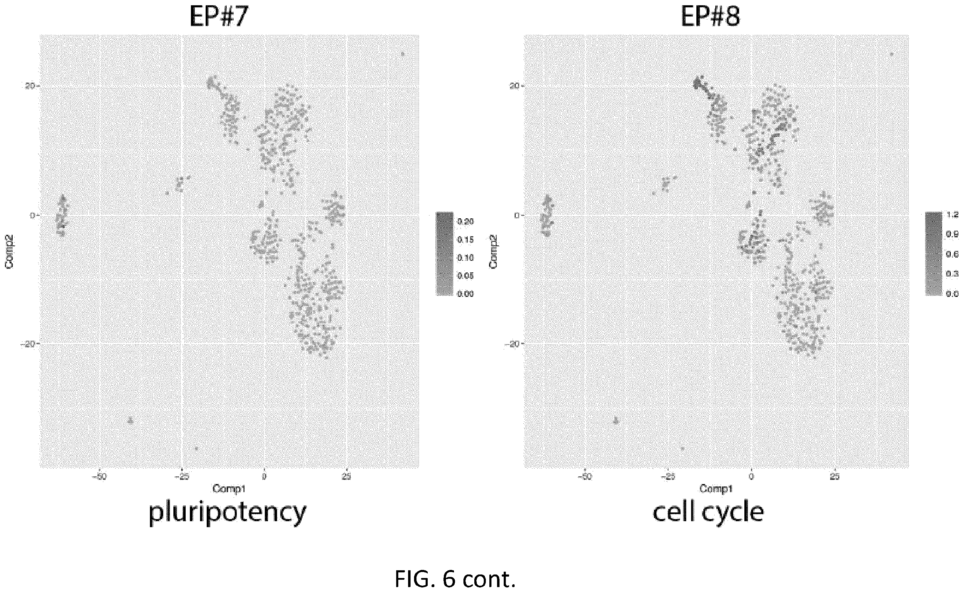

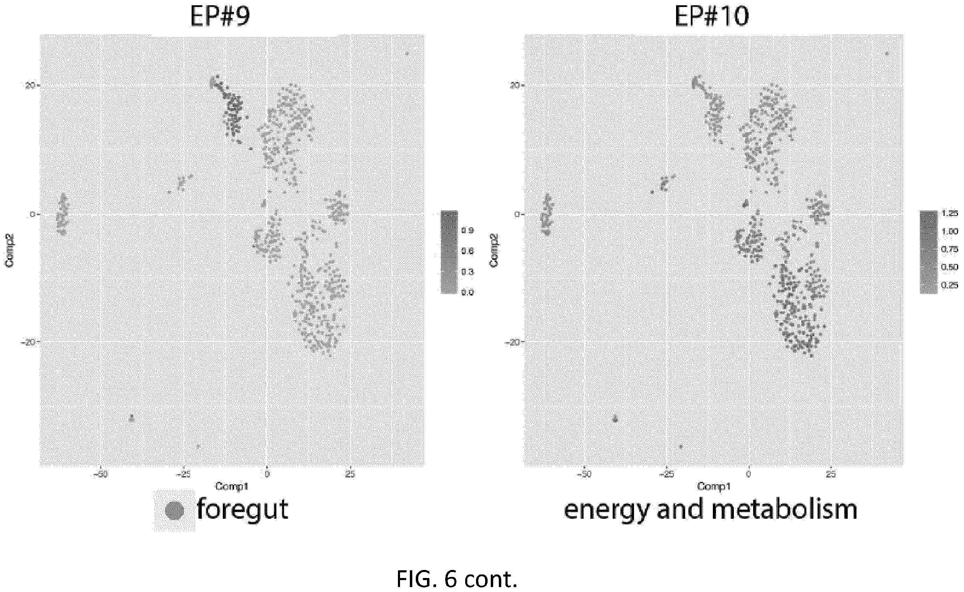

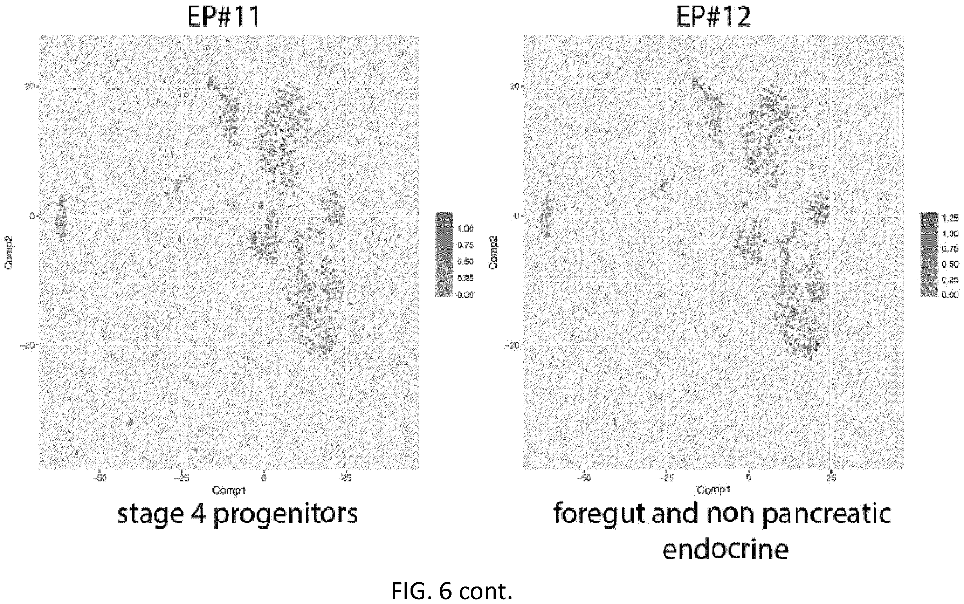

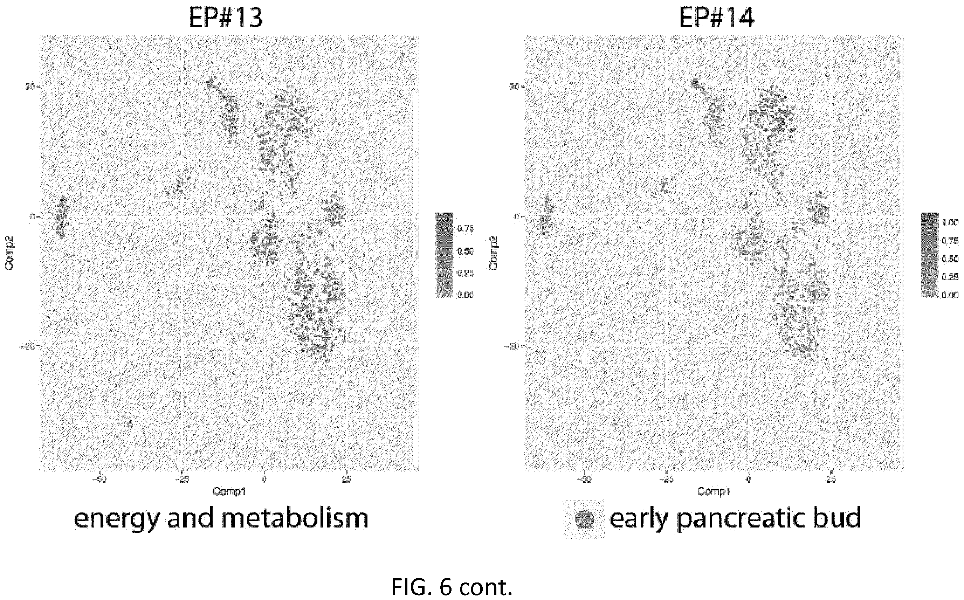

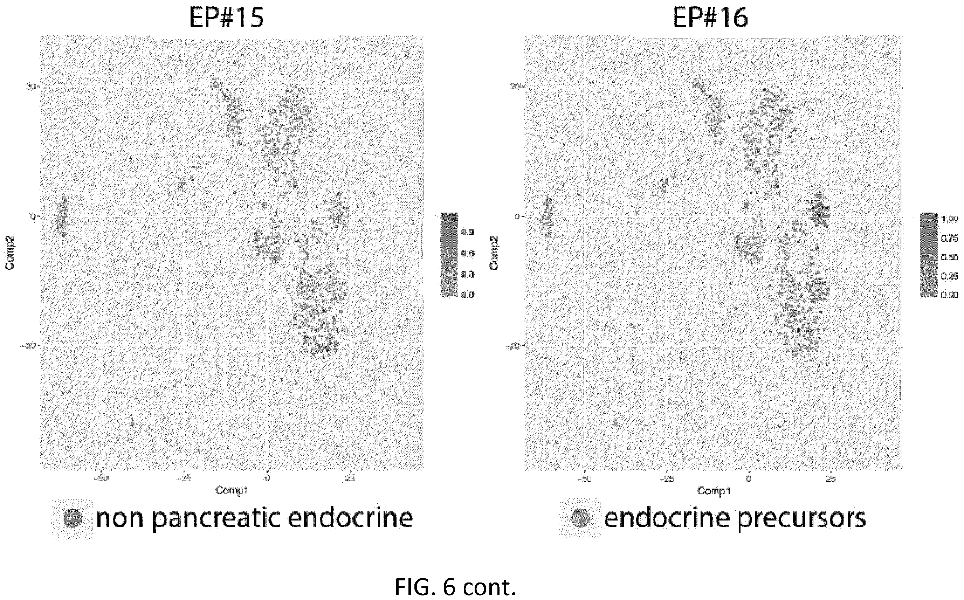

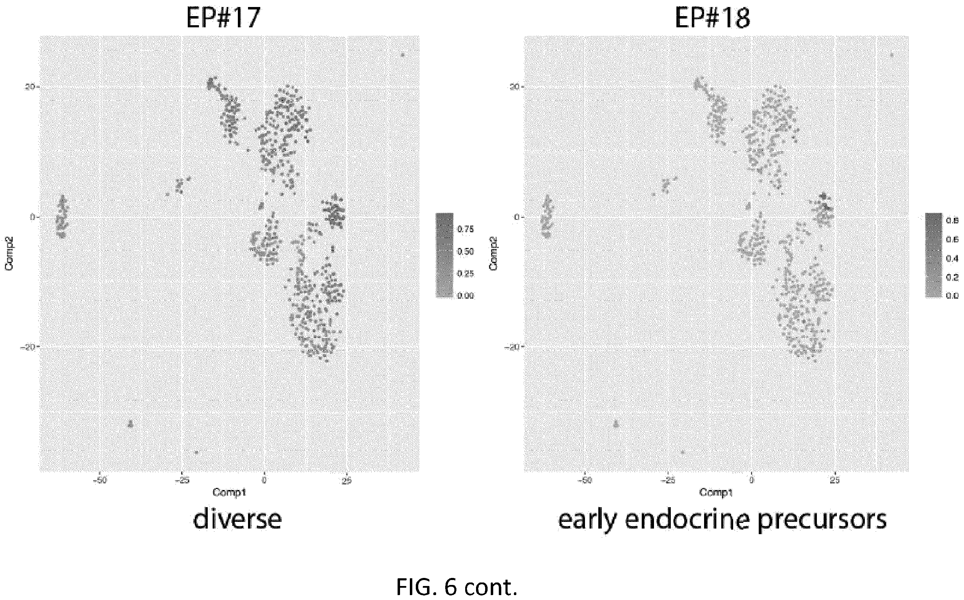

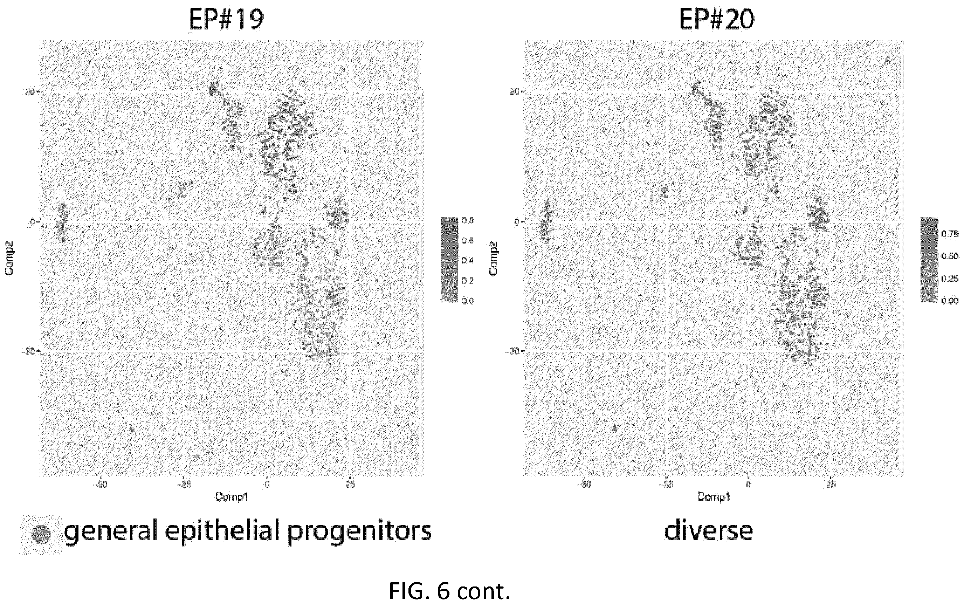

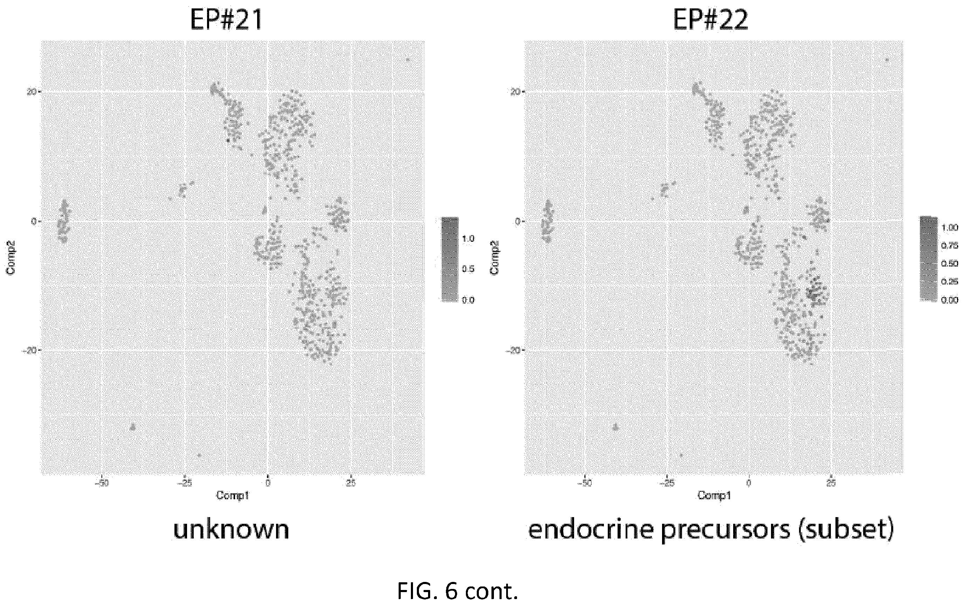

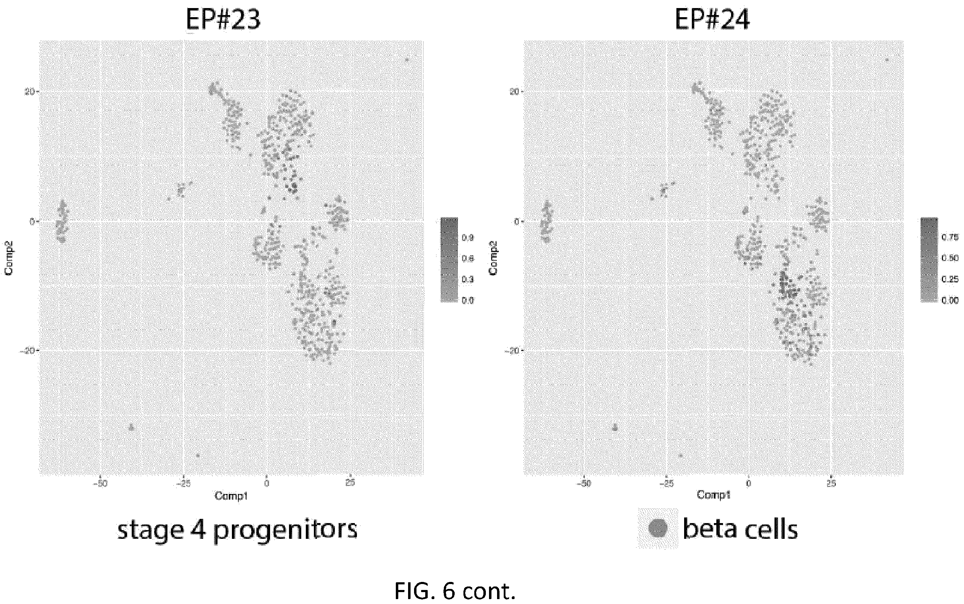

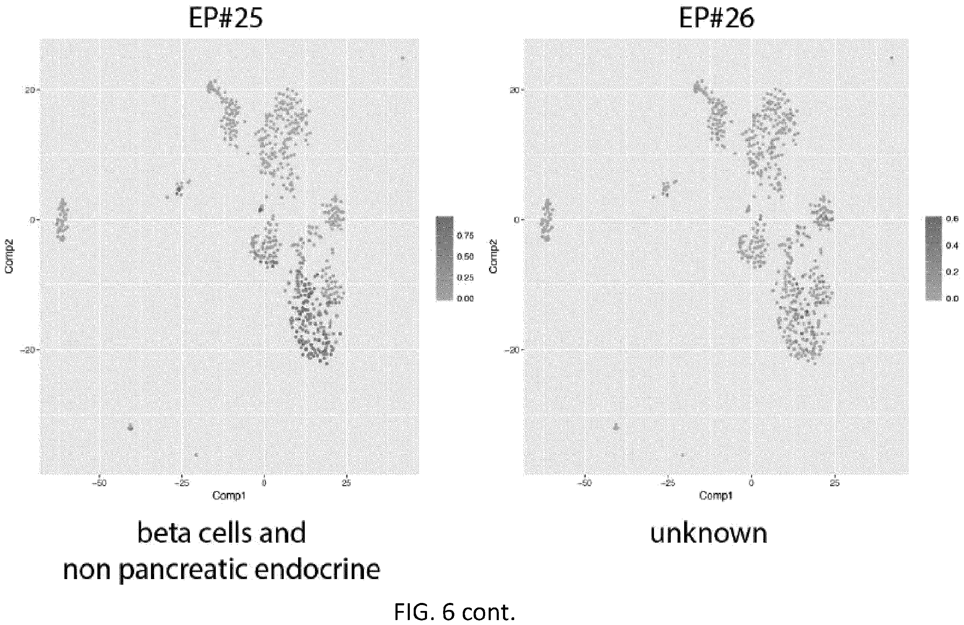

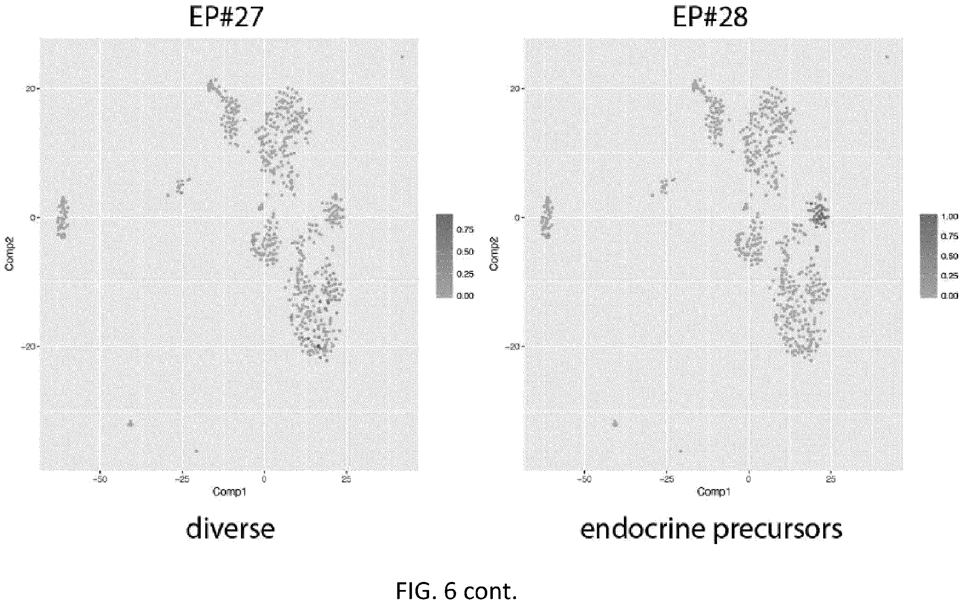

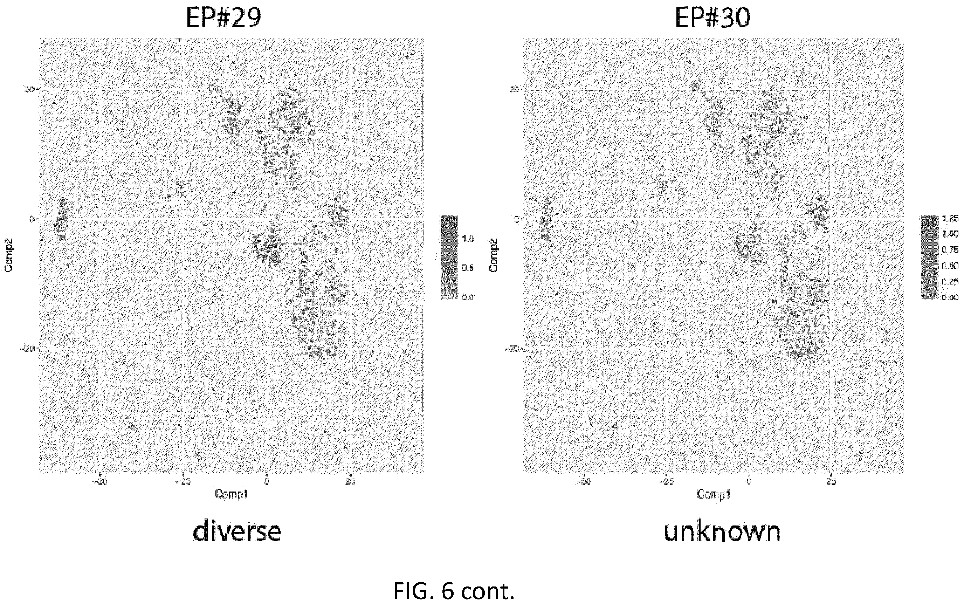

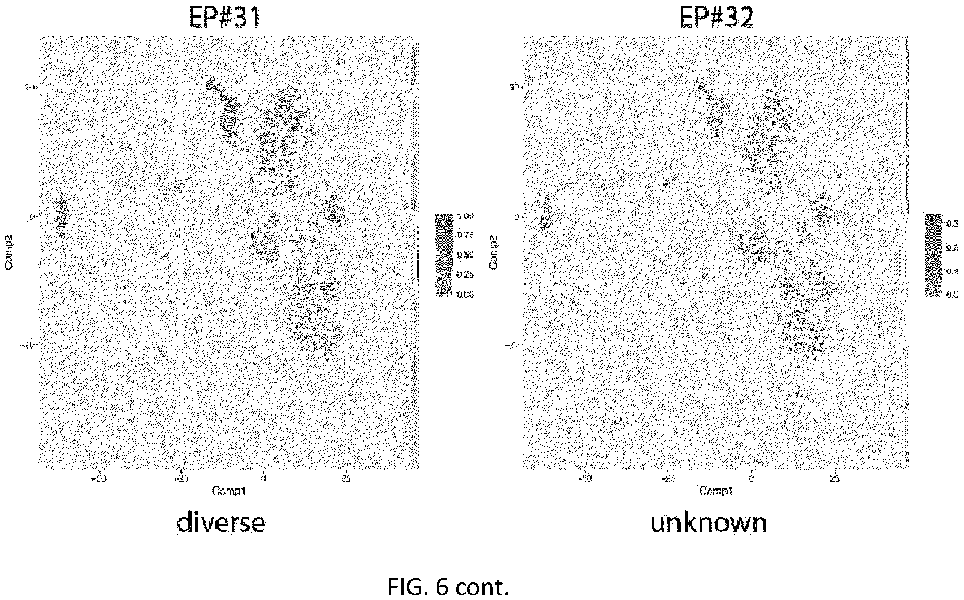

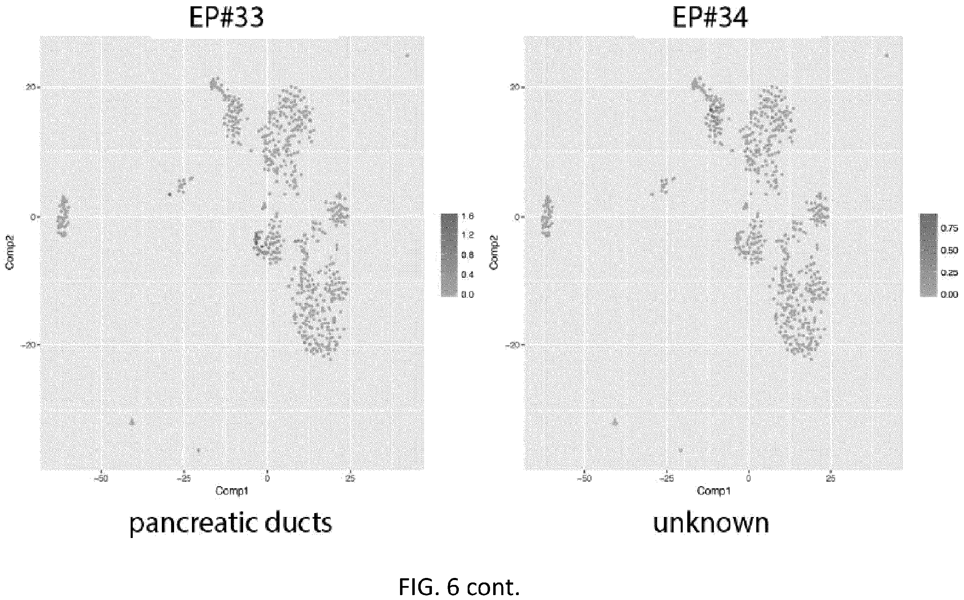

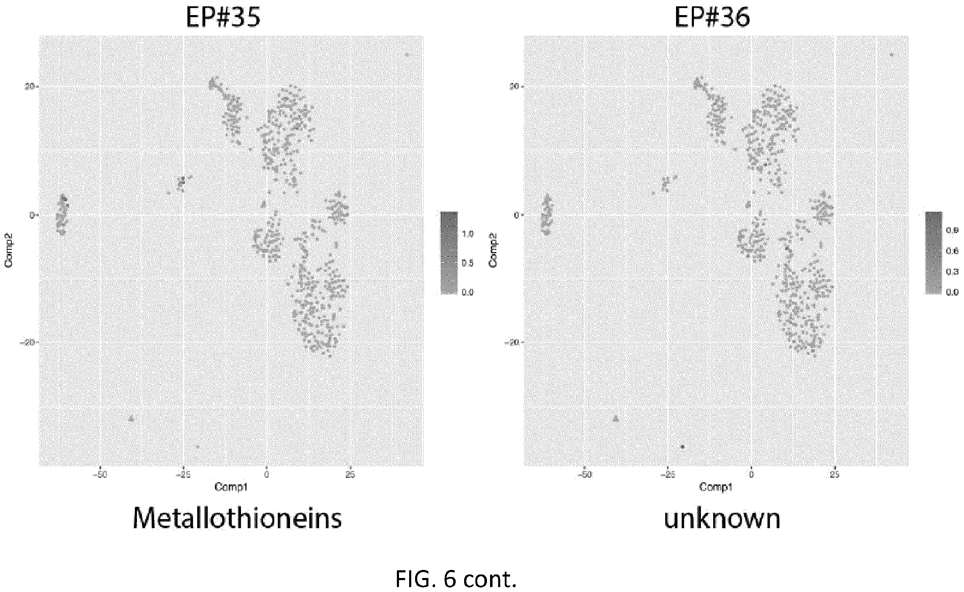

FIG. 6 provides per-cell usage values of all EPs identified, overlaid on top of a SIMLR 2D map. Each EP is annotated according to marker genes with high relevance values. "Unknown" EPs are EPs in which the participating genes were insufficient to establish a characteristic biological identity. The major 8 cell-type specific EPs are color labeled.

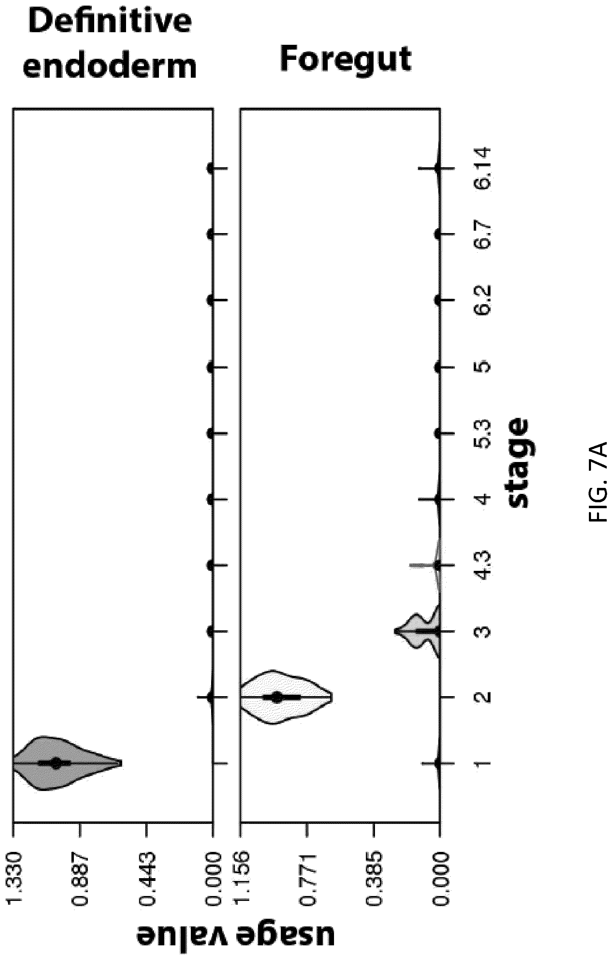

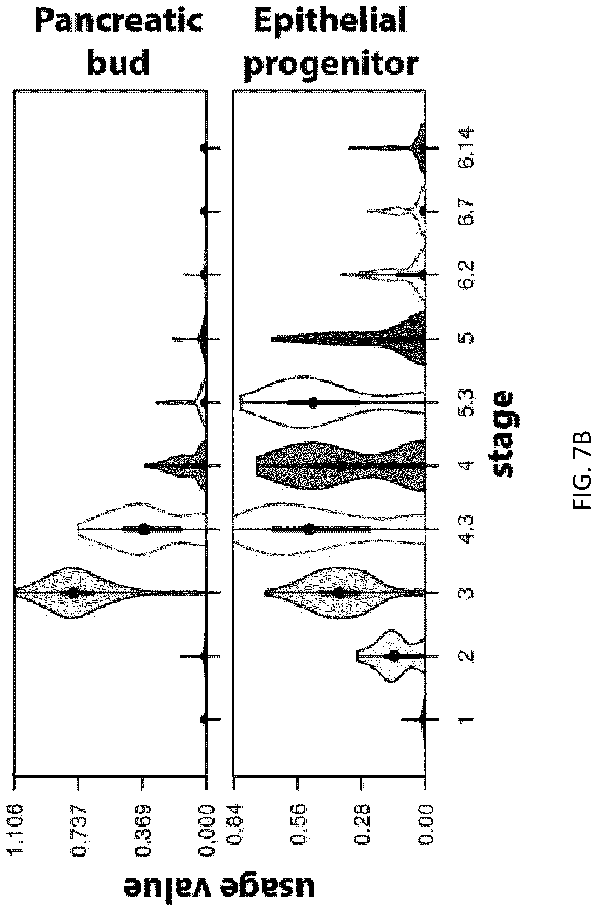

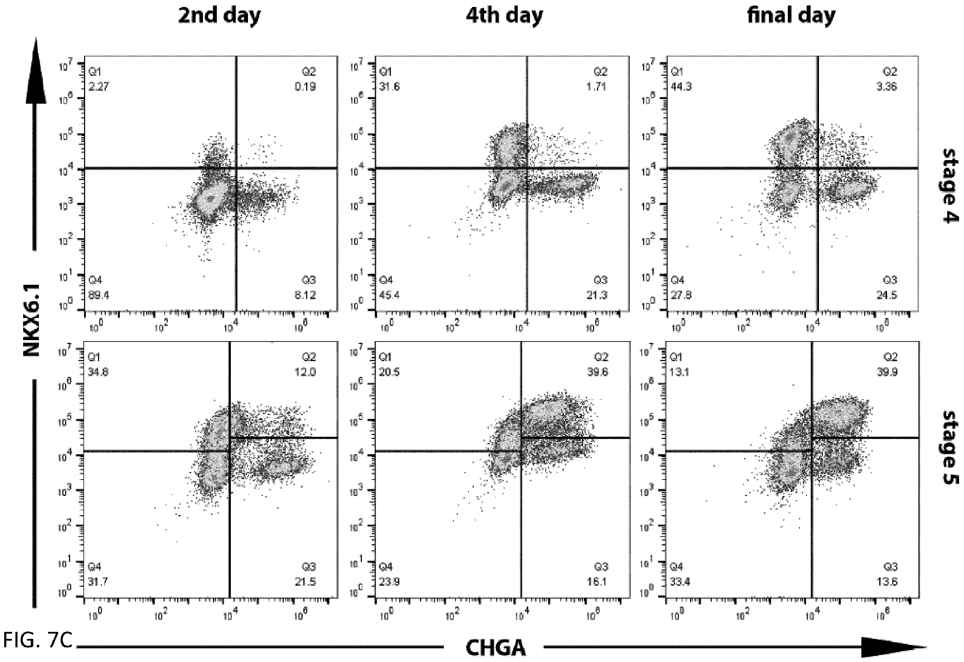

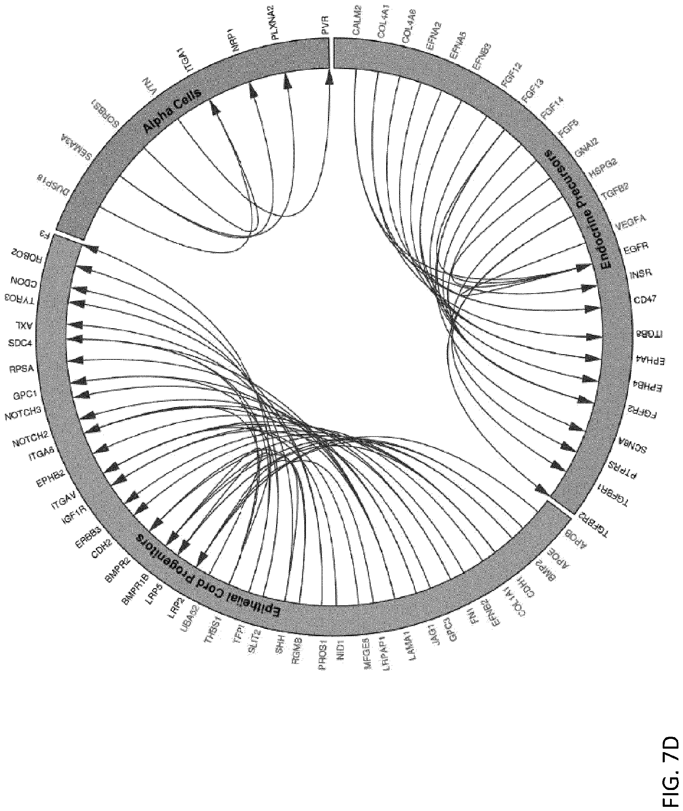

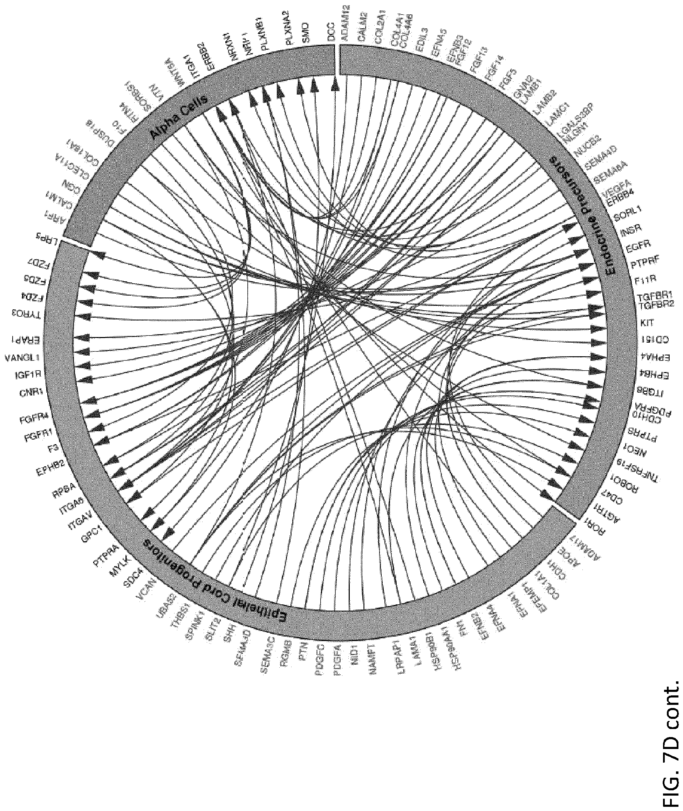

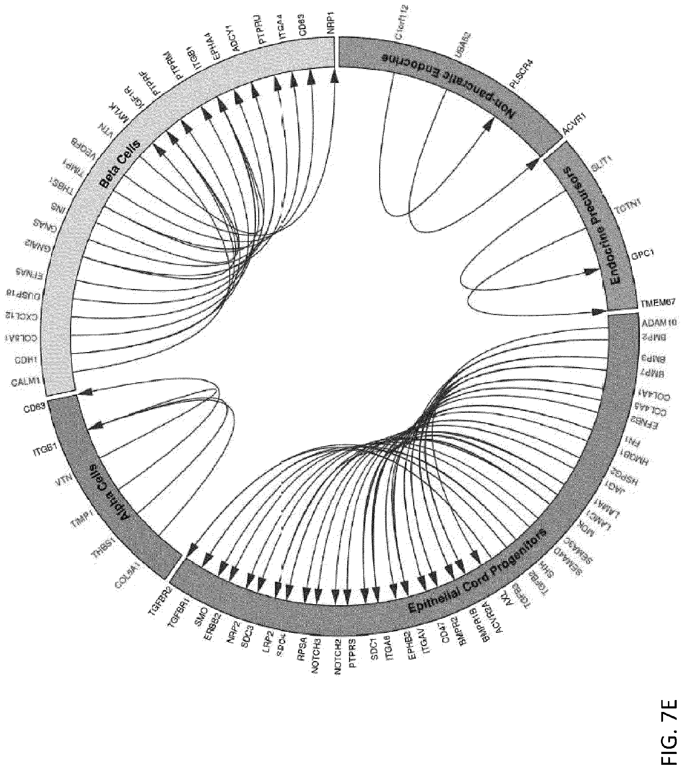

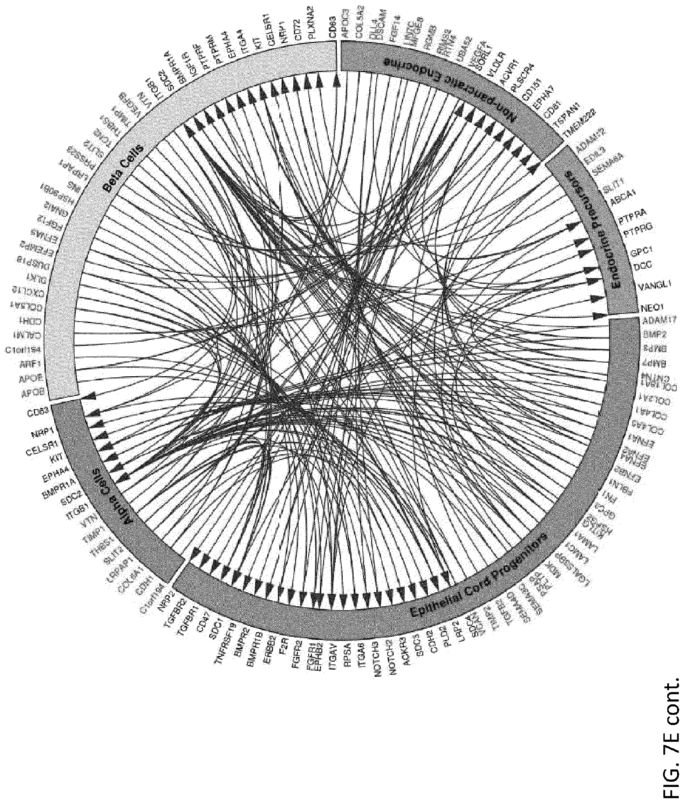

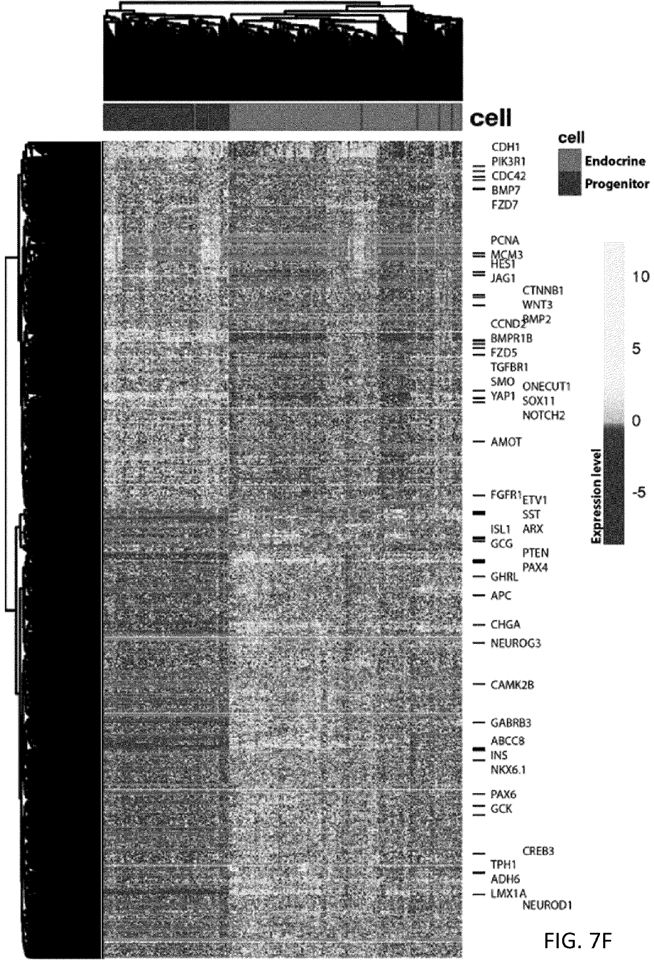

FIGS. 7A-7F demonstrate temporal effect on cell fate and differential expression of genes between progenitor and endocrine cells. FIG. 7A shows usage value distributions of the definitive endoderm and foregut EPs by cells collected at different stages of the protocol. FIG. 7B shows usage value distributions of the pancreatic bud and epithelial progenitor EPs by cells collected at different stages of the protocol. FIG. 7C provides representative flow cytometry plots show that CHGA⁺ endocrine cells rarely express NKX6.1 during stage 4, whereas at stage 5 most CHGA⁺ cells are also NKX6.1⁺. FIG. 7D provides ligand-receptor analysis in stage 4 cells. (FIG. 7D) potential autoregulatory LR pairs. (FIG. 7D cont.) potential cross-regulatory LR pairs. FIG. 7E provides ligand-receptor analysis in stage 5 cells. (FIG. 7E) potential autoregulatory LR pairs. (FIG. 7E cont.) potential cross-regulatory LR pairs. FIG. 7F provides a heatmap presenting the relative gene expression level for 4863 genes that are differentially expressed between epithelial-progenitor and endocrine populations, with FDR of less or equal to 0.05.

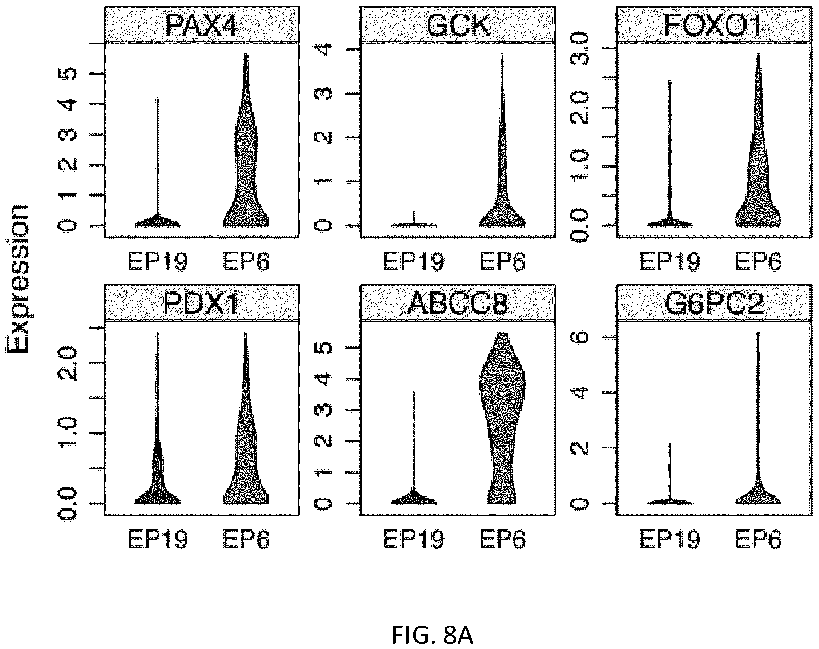

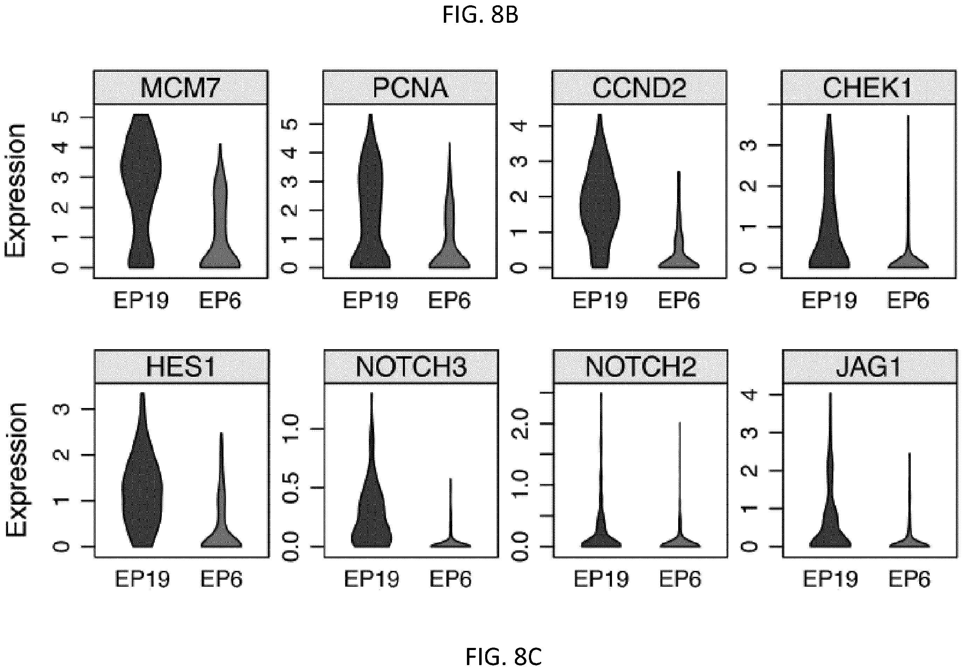

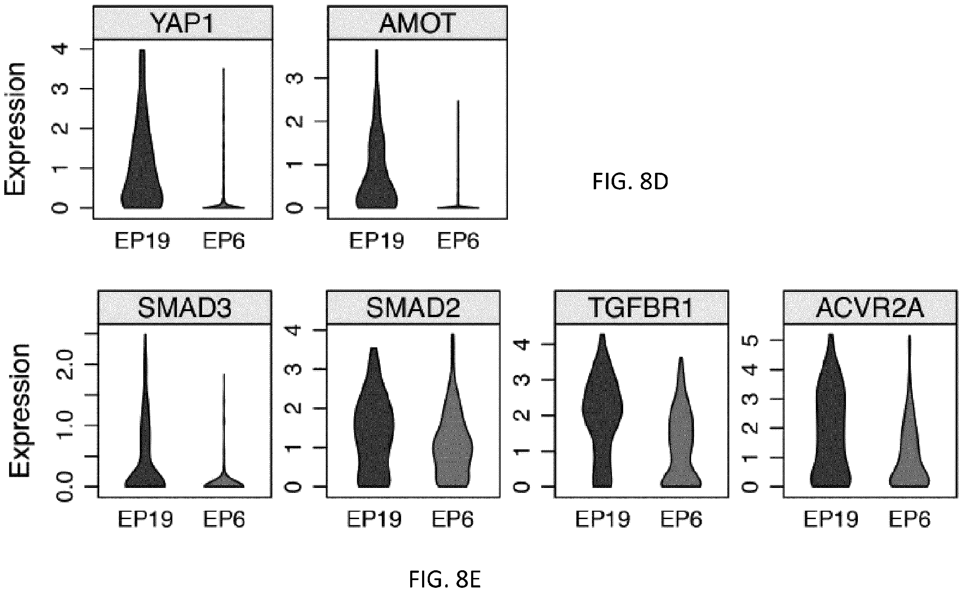

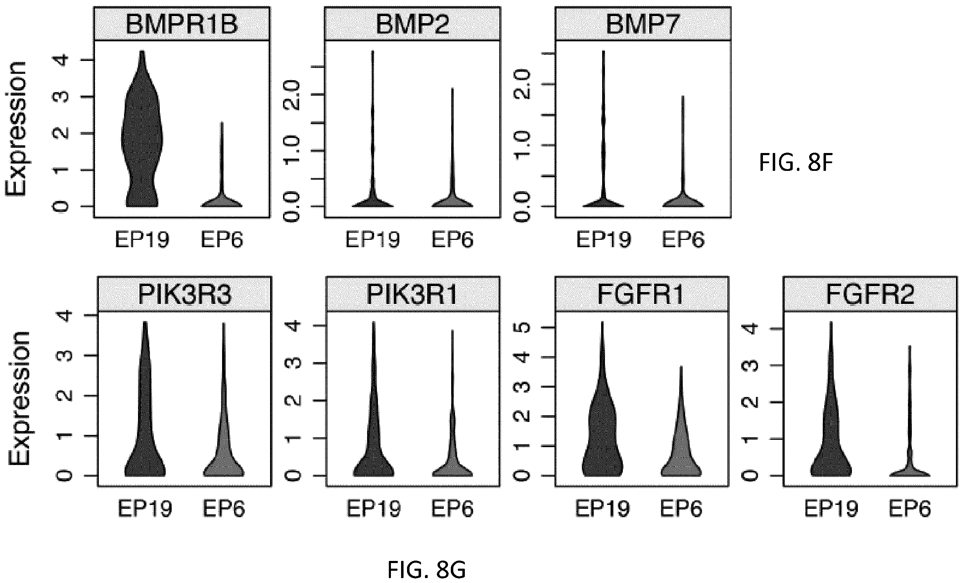

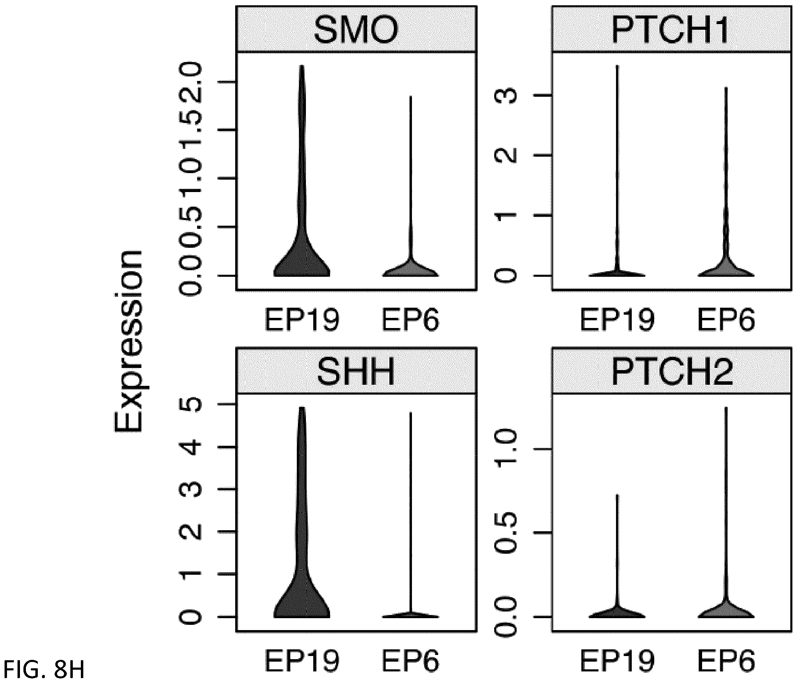

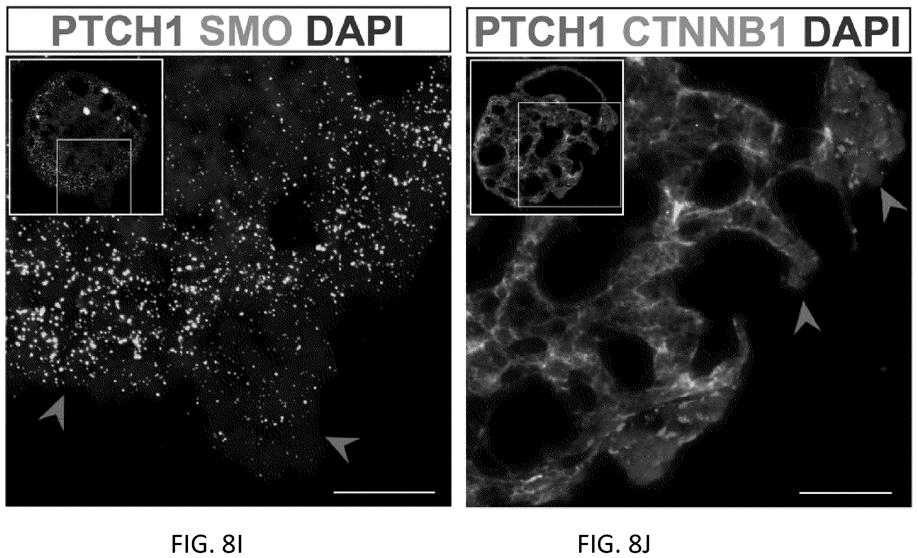

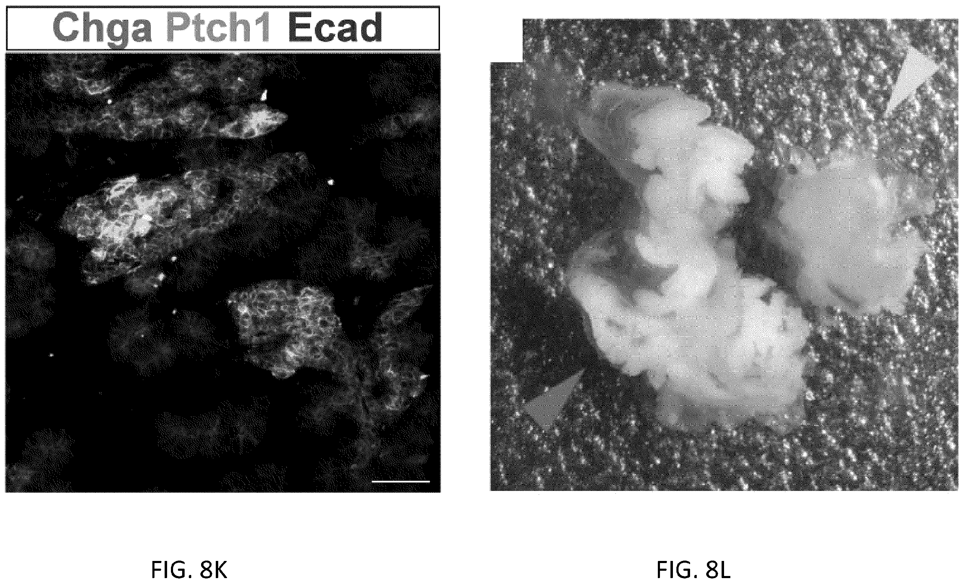

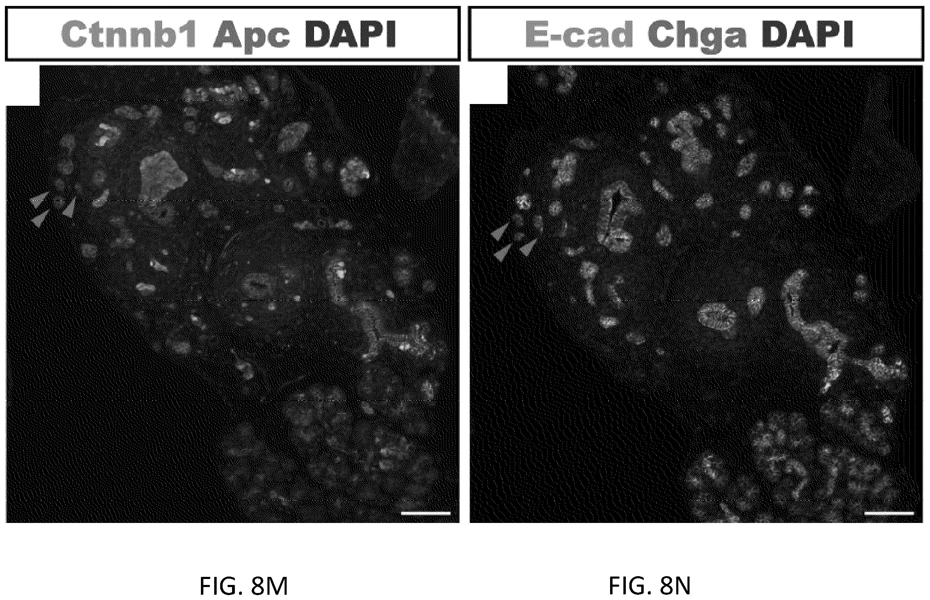

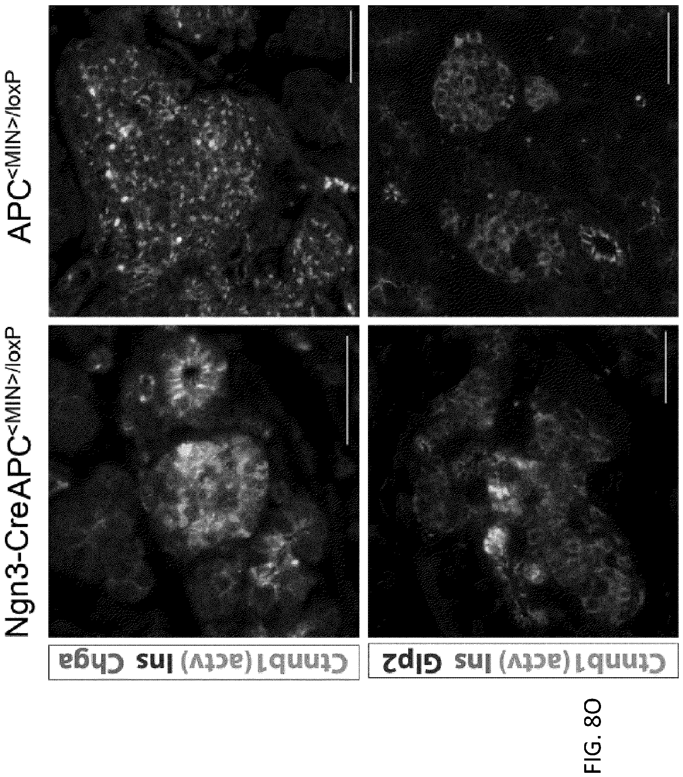

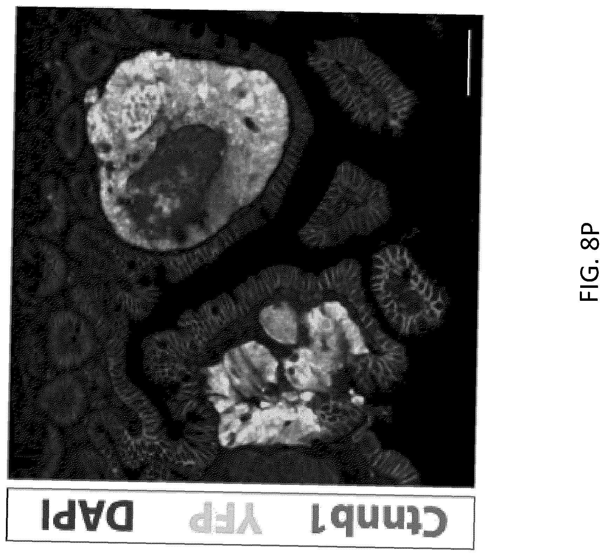

FIGS. 8A-8P demonstrates differentially expressed genes between progenitor and endocrine cells and that the Hedgehog pathway is differentially active between the progenitor and endocrine compartments. FIGS. 8A-8H shows groups of genes that are differentially expressed between epithelial progenitors and endocrine cells. Genes related with MODY or endocrine cell function (FIG. 8A); proliferation markers (FIG. 8B); Notch pathway genes (FIG. 8C); Hippo pathway genes (FIG. 8D); TGFβ pathway genes related with Activin (FIG. 8E); BMP pathway genes (FIG. 8F); PI3K and FGF pathway related genes (FIG. 8G). FIG. 8H shows activating components of the hedgehog pathway (SHH, SMO) are enriched in the progenitors, but no significant enrichment of PTCH transcripts is observed in either compartment. FIG. 8I shows fluorescent in-situ hybridization of clusters at the end of stage 4 showing PTCH1 transcripts are uniformly distributed in the epithelial compartment (green arrowhead) and the endocrine bud (red arrowhead), whereas SMO transcript density is low in the endocrine bud. scale bar: 50µM. FIG. 8J shows immunofluorescent staining of clusters at the end of stage 4 detects PTCH1 protein only in the endocrine buds. Scale bar: 50µM. FIG. 8K shows immunostaining of a cross section through mouse embryonic pancreas at e16.5 shows that Ptch1 protein expression is confined to the Chga⁺ developing islets. FIG. 8L shows pancreata of newborn (P0) Apc^{loxP/WT};no-cre mouse (left, red arrowhead) and Apc^{loxP/MIN};Pdx1-cre mouse (right, blue arrowhead) show the absence of Apc in the pancreas results in the formation of a rudimentary pancreas. FIGS. 8M-8N provide a cross section through a pancreas of 1 day old pup (P1) of Apc^{loxP/MIN};Pdx1-cre mouse showing that the endocrine cells that did form (for example, red arrowheads) all maintain Apc expression, indicating cre recombinase escape. Scale bar: 100µM. FIG. 8O shows a mouse bearing an allele with a mutated form of Apc (Apc^{MIN}) and an allele with loxP sites flanking the gene (Apc^{loxP}) was crossed with a mouse expressing Cre under the Ngn3 promoter. This yielded Ngn3-Cre;Apc^{MIN/loxp} mice in which Apc is deleted specifically in endocrine progenitors. In P1 mice lacking Apc in endocrine progenitors, no Chga or hormones are detected in cells that show nuclear accumulation of Ctnnb1. Scale bar: 50µm. FIG. 8P shows hyperplastic lesions observed in the gut of a 2-month old Ngn3-Cre;Apc^{loxP/loxP};ROSA-YFP^{loxp-stop-loxP} mouse. Scale bar: 50µm.

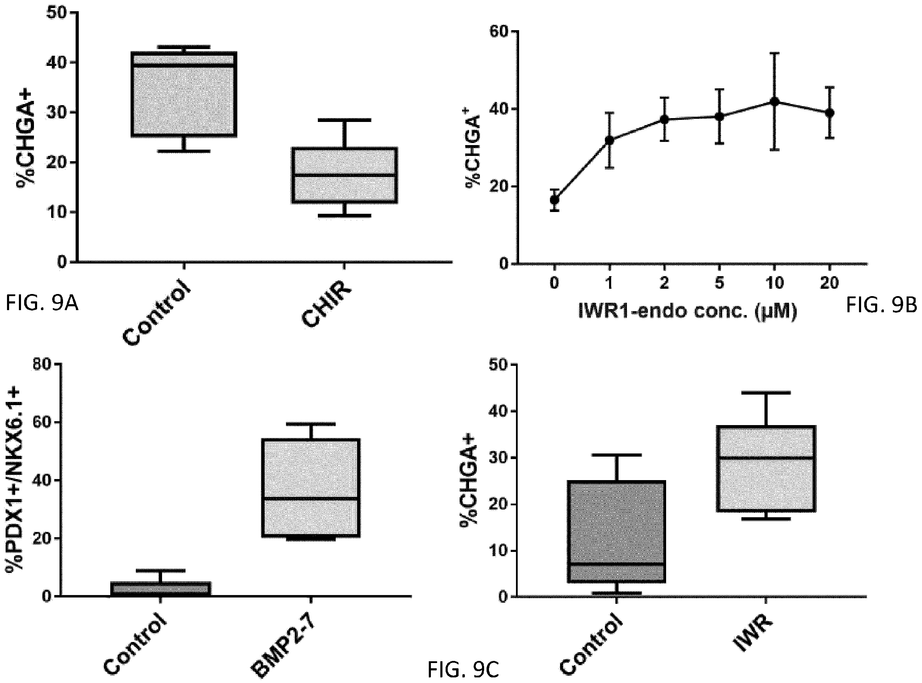

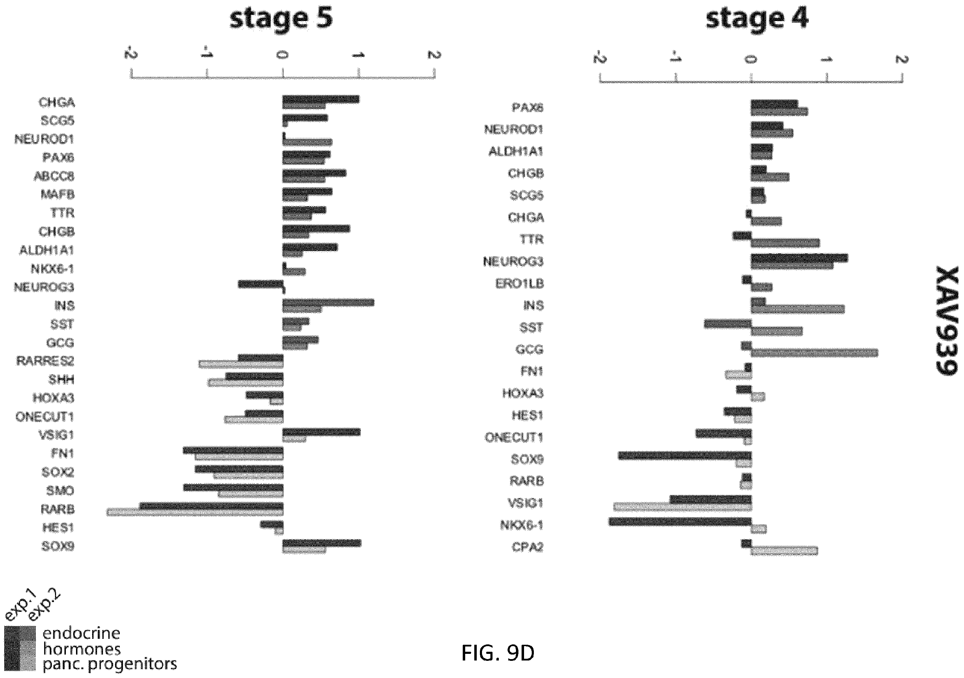

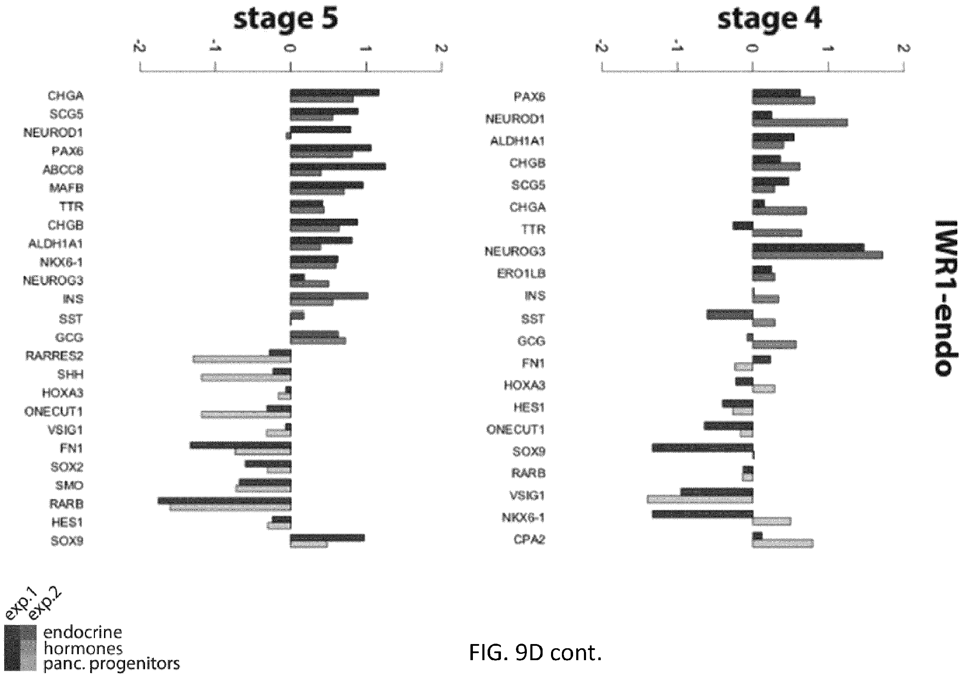

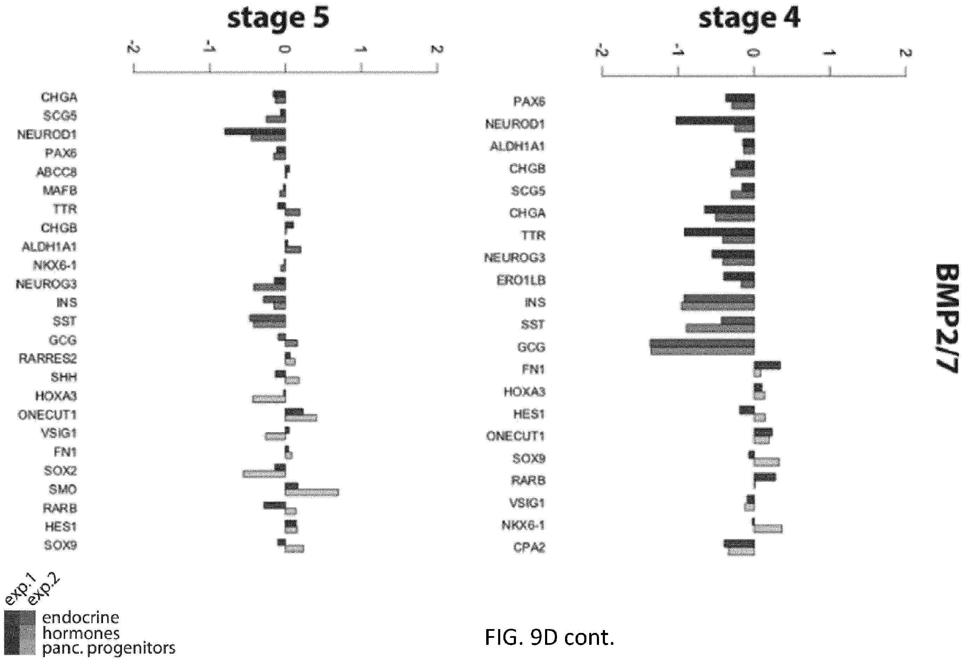

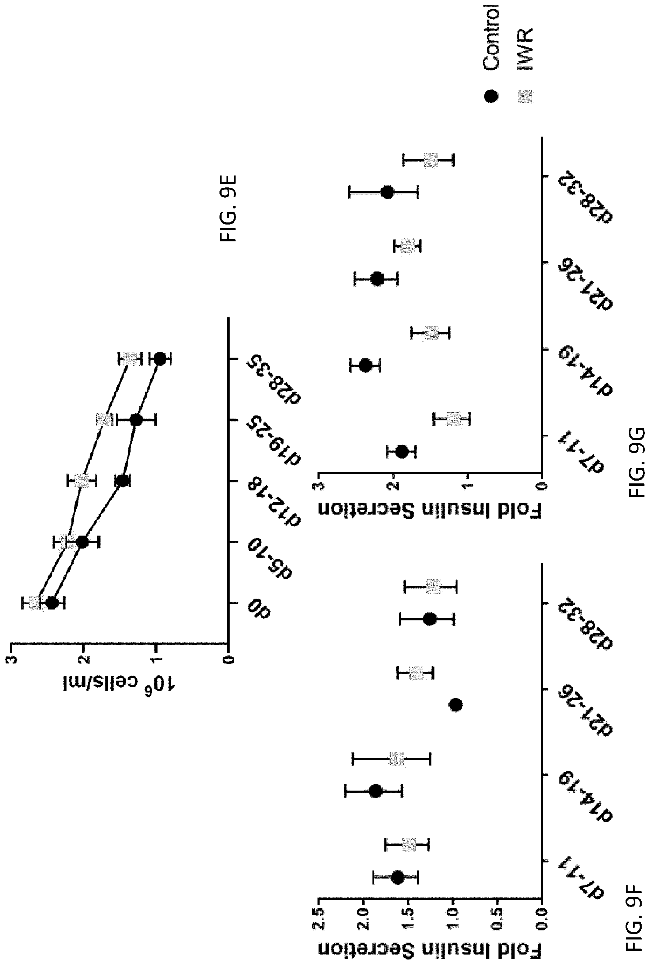

FIGS. 9A-9G demonstrate WNT pathway inhibition in endocrine differentiation. FIG. 9A shows the proportion of CHGA⁺ cells at the end of stage 4, with and without administration of CHIR99021, was measured using flow cytometry. n=5, p-value= 0.004, Student's t-test. FIG. 9B shows flow cytometry was used to determine the proportion of CHGA⁺ cells at the end of stage 4, following administration of IWR1-endo at various concentrations. n=3, cells were grown together until the end of stage 3 and then split into 3 parallel differentiations. Plot shows mean±S.E. FIG. 9C provides flow cytometry showing that recombinant BMP2-7 increases the proportion of hiPSC-1016 derived PDX⁺NKX6.1⁺ cells at stage 4. n=5, p-value= 0.007, Student's t-test (left side) and flow cytometry showing that IWR1-endo increases the proportion of hiPSC-1016 derived CHGA⁺ cells at stage 5. n=5, p-value= 0.02, Student's t-test (right side). FIG. 9D shows nanostring was used to measure mRNA expression levels of various endocrine markers (red), hormones (purple) and pancreatic-epithelial-progenitor markers (blue) after administration of recombinant BMP2-7 or the WNT inhibitors IWR1-endo and XAV939. The relative changes in gene expression relative to control conditions are presented in log values, for two independent experiments. FIG. 9E provides cell concentration in the differentiation batches analyzed in FIG. 4H. Cell concentration during stage 6 of the differentiation protocol is slightly increased following IWR1-endo administration during stage 5 (p-value=0.002, 2-way ANOVA). Notice that the decrease in cell concentration observed throughout stage 6 (p-value<10⁻⁴, 2-way ANOVA) is at least partly caused by consecutive sample collection from the differentiation container. Bars show the average±S.E. n, from left to right: 8 (7 for IWR), 6, 8, 7, 6. FIGS. 9F-9G shows glucose stimulated insulin secretion (GSIS) was measured at consecutive intervals during stage 6 of the differentiation protocol. The bars show geometric mean±S.E. of the fold change in insulin secretion following the transition from low (2.8mM) to high (20mM) glucose levels. FIG. 9F provides fold change in insulin secretion after one stimulation with high glucose. From left to right: n=5,6,6,3; 2-way ANOVA on log(Fold insulin secretion): p-value=0.83 and 0.16 for differences caused by the treatment or the day of analysis, respectively. FIG. 9G provides fold change in insulin secretion after a second stimulation with high glucose. From left to right: n=5,6,6,3; 2-way ANOVA on log(Fold insulin secretion): p-value=0.005 and 0.32 for differences caused by the treatment or the day of analysis, respectively.

The following Tables 1-3 are submitted herewith as Appendices 1-3, respectively:
Table 1 - provides EPs identified during *in vitro* differentiation of hESCs into β cells. The table presents the EPs identified through TM analysis, with the genes in each EP and the relevance value of each gene to the EP. GeneProgram Topic-Modeling Results from 773 cells, and 33088 nonzero-expression genes. The FPKM values of each gene are discretized into 4 levels. The Table is related to FIG. 1.
Table 2 - provides differentially enriched gene groups between epithelial progenitors and endocrine cells. The table presents the results of a gene enrichment analysis which compares the progenitor and endocrine populations presented in FIG. 3.
Table 3 - provides a ligand-receptor interaction analysis. The table presents the potential ligand-receptor pairs presented in FIG. 7D. Pairs are classified according to the stage in the protocol of the analyzed cells, and according to the potential for either auto- or inter- cell type interactions.

The material submitted herewith in electronic (.txt) form and comprising Appendices 1-3 (Tables 1-3, respectively) is incorporated herein by reference, specifically:
Appendix 1 (file name: Table 1.pdf; date created: May 9, 2019; and file size: 531,839 bytes);
Appendix 2 (file name: Table 2.pdf; date created: May 9, 2019; and file size: 46,643 bytes); and
Appendix 3 (file name: Table 3.pdf; date created: May 9, 2019; and file size: 21,352 bytes).

### DETAILED DESCRIPTION OF THE INVENTION

Aspects of the disclosure relate to alternative protocols for producing stem cell-derived beta (SC-β) cells and/or stem cell-derived alpha (SC-α) cells. Other aspects of the disclosure relate to methods of directing the differentiation of cells with multiple potential differentiation outcomes toward or away from particular differentiation outcomes, such as by targeting one or more pathways.

### Definitions

For convenience, certain terms employed herein, in the specification, examples and appended claims are collected here. Unless otherwise defined, all technical and scientific terms used herein have the same meaning as commonly understood by one of ordinary skill in the art to which this invention belongs.

The term "differentiated cell" is meant any primary cell that is not, in its native form, pluripotent as that term is defined herein. Stated another way, the term "differentiated cell" refers to a cell of a more specialized cell type derived from a cell of a less specialized cell type (e.g., a stem cell such as an induced pluripotent stem cell) in a cellular differentiation process. Without wishing to be limited to theory, a pluripotent stem cell in the course of normal ontogeny can differentiate first to an endoderm cell that is capable of forming pancreas cells and other endoderm cell types. Further differentiation of an endoderm cell leads to the pancreatic pathway, where ~98% of the cells become exocrine, ductular, or matrix cells, and ~2% become endocrine cells.

As used herein, the term "somatic cell" refers to any cells forming the body of an organism, as opposed to germline cells. In mammals, germline cells (also known as "gametes") are the spermatozoa and ova which fuse during fertilization to produce a cell called a zygote, from which the entire mammalian embryo develops. Every other cell type in the mammalian body-apart from the sperm and ova, the cells from which they are made (gametocytes) and undifferentiated stem cells-is a somatic cell: internal organs, skin, bones, blood, and connective tissue are all made up of somatic cells. In some embodiments the somatic cell is a "non-embryonic somatic cell", by which is meant a somatic cell that is not present in or obtained from an embryo and does not result from proliferation of such a cell *in vitro.* In some embodiments the somatic cell is an "adult somatic cell", by which is meant a cell that is present in or obtained from an organism other than an embryo or a fetus or results from proliferation of such a cell *in vitro.* Unless otherwise indicated the methods described herein can be performed both *in vivo* and *in vitro.*

As used herein, the term "adult cell" refers to a cell found throughout the body after embryonic development.

The term "endoderm cell" as used herein refers to a cell which is from one of the three primary germ cell layers in the very early embryo (the other two germ cell layers are the mesoderm and ectoderm). The endoderm is the innermost of the three layers. An endoderm cell differentiates to give rise first to the embryonic gut and then to the linings of respiratory and digestive tracts (e.g. the intestine), the liver and the pancreas.

The term "a cell of endoderm origin" as used herein refers to any cell which has developed or differentiated from an endoderm cell. For example, a cell of endoderm origin includes cells of the liver, lung, pancreas, thymus, intestine, stomach and thyroid. Without wishing to be bound by theory, liver and pancreas progenitors (also referred to as pancreatic progenitors) develop from endoderm cells in the embryonic foregut. Shortly after their specification, liver and pancreas progenitors rapidly acquire markedly different cellular functions and regenerative capacities. These changes are elicited by inductive signals and genetic regulatory factors that are highly conserved among vertebrates.

The term "pancreatic progenitor" or "pancreatic precursor" are used interchangeably herein and refer to a stem cell which is capable of forming any of; pancreatic endocrine cells, pancreatic exocrine cells, or pancreatic duct cells. The term "pdx1-positive pancreatic progenitor" as used herein refers to a cell which is a pancreatic endoderm (PE) cell. A Pdx1-positive pancreatic progenitor expresses the marker Pdx1. Other markers include, but are not limited to Cdcp1, or Ptf1a, or HNF6 or NRx2.2. The expression of Pdx 1 may be assessed by any method known by the skilled person such as immunochemistry using an anti-Pdx1 antibody or quantitative RT-PCR. The term "pdx1-positive, NKX6-1-positive pancreatic progenitor" as used herein refers to a cell which is a pancreatic endoderm (PE) cell. A pdx1-positive, NKX6-1-positive pancreatic progenitor expresses the markers Pdx1 and NKX6-1. Other markers include, but are not limited to Cdcp1, or Ptf1a, or HNF6 or NRx2.2. The expression of NKX6-1 may be assessed by any method known by the skilled person such as immunochemistry using an anti-NKX6-1 antibody or quantitative RT-PCR.

The terms "stem cell-derived β cell", "SC-β cell", and "mature SC-β cell" refer to cells (e.g., pancreatic β cells) that display at least one marker indicative of a pancreatic β cell, express insulin, and display a GSIS response characteristic of an endogenous mature β cell. In some embodiments, the "SC-β cell" comprises a mature pancreatic β cells. It is to be understood that the SC-β cell need not be derived (e.g., directly) from stem cells, as the methods of the disclosure are capable of deriving SC-β cells from any insulin-positive endocrine cell or precursor thereof using any cell as a starting point (e.g., one can use embryonic stem cells, induced-pluripotent stem cells, progenitor cells, partially reprogrammed somatic cells (e.g., a somatic cell which has been partially reprogrammed to an intermediate state between an induced pluripotent stem cell and the somatic cell from which it was derived), multipotent cells, totipotent cells, a transdifferentiated version of any of the foregoing cells, etc, as the invention is not intended to be limited in this manner). Examples of SC-β cells, and methods of obtaining such SC-β cells, are described in WO 2015/002724 and WO 2014/201167, both of which are incorporated herein by reference in their entirety.

The terms "stem cell-derived α cell", "SC-α cell", and "mature SC-α cell" refer to cells (e.g., pancreatic α cells) that display at least one marker indicative of a pancreatic α cell, express and secrete glucagon, and display an ultrastructure similar to cadaveric alpha cells. In some embodiments, the "SC- α cell" comprises a mature pancreatic α cell. It is to be understood that the SC- α cells need not be derived (e.g., directly) from stem cells, as the methods of the disclosure are capable of deriving SC-α cells from any insulin-positive endocrine cell or precursor thereof using any cell as a starting point (e.g., one can use embryonic stem cells, induced-pluripotent stem cells, progenitor cells, partially reprogrammed somatic cells (e.g., a somatic cell which has been partially reprogrammed to an intermediate state between an induced pluripotent stem cell and the somatic cell from which it was derived), multipotent cells, totipotent cells, a transdifferentiated version of any of the foregoing cells, etc, as the invention is not intended to be limited in this manner). Moreover it should be understood that an SC- α cell is a non-native, i.e., non-naturally occurring, non-endogenous, cell and has at least one characteristic that is different from a native/naturally-occurring/endogenous cell.

The term "exocrine cell" as used herein refers to a cell of an exocrine gland, i.e. a gland that discharges its secretion via a duct. In particular embodiments, an exocrine cell refers to a pancreatic exocrine cell, which is a pancreatic cell that produces enzymes that are secreted into the small intestine. These enzymes help digest food as it passes through the gastrointestinal tract. Pancreatic exocrine cells are also known as islets of Langerhans that secrete two hormones, insulin and glucagon.

The term "phenotype" refers to one or a number of total biological characteristics that define the cell or organism under a particular set of environmental conditions and factors, regardless of the actual genotype.

The term "pluripotent" as used herein refers to a cell with the capacity, under different conditions, to differentiate to more than one differentiated cell type, and preferably to differentiate to cell types characteristic of all three germ cell layers. Pluripotent cells are characterized primarily by their ability to differentiate to more than one cell type, preferably to all three germ layers, using, for example, a nude mouse teratoma formation assay. Pluripotency is also evidenced by the expression of embryonic stem (ES) cell markers, although the preferred test for pluripotency is the demonstration of the capacity to differentiate into cells of each of the three germ layers. It should be noted that simply culturing such cells does not, on its own, render them pluripotent. Reprogrammed pluripotent cells (e.g. iPS cells as that term is defined herein) also have the characteristic of the capacity of extended passaging without loss of growth potential, relative to primary cell parents, which generally have capacity for only a limited number of divisions in culture.

As used herein, the terms "iPS cell" and "induced pluripotent stem cell" are used interchangeably and refers to a pluripotent stem cell artificially derived (e.g., induced or by complete reversal) from a non-pluripotent cell, typically an adult somatic cell, for example, by inducing a forced expression of one or more genes.

The term "progenitor" or "precursor" cell are used interchangeably herein and refer to cells that have a cellular phenotype that is more primitive (i.e., is at an earlier step along a developmental pathway or progression than is a fully differentiated cell) relative to a cell which it can give rise to by differentiation. Often, progenitor cells also have significant or very high proliferative potential. Progenitor cells can give rise to multiple distinct differentiated cell types or to a single differentiated cell type, depending on the developmental pathway and on the environment in which the cells develop and differentiate.

The term "stem cell" as used herein, refers to an undifferentiated cell which is capable of proliferation and giving rise to more progenitor cells having the ability to generate a large number of mother cells that can in turn give rise to differentiated, or differentiable daughter cells. The daughter cells themselves can be induced to proliferate and produce progeny that subsequently differentiate into one or more mature cell types, while also retaining one or more cells with parental developmental potential. The term "stem cell" refers to a subset of progenitors that have the capacity or potential, under particular circumstances, to differentiate to a more specialized or differentiated phenotype, and which retains the capacity, under certain circumstances, to proliferate without substantially differentiating. In one embodiment, the term stem cell refers generally to a naturally occurring mother cell whose descendants (progeny) specialize, often in different directions, by differentiation, e.g., by acquiring completely individual characters, as occurs in progressive diversification of embryonic cells and tissues. Cellular differentiation is a complex process typically occurring through many cell divisions. A differentiated cell may derive from a multipotent cell which itself is derived from a multipotent cell, and so on. While each of these multipotent cells may be considered stem cells, the range of cell types each can give rise to may vary considerably. Some differentiated cells also have the capacity to give rise to cells of greater developmental potential. Such capacity may be natural or may be induced artificially upon treatment with various factors. In many biological instances, stem cells are also "multipotent" because they can produce progeny of more than one distinct cell type, but this is not required for "stem-ness." Self-renewal is the other classical part of the stem cell definition, and it is essential as used in this document. In theory, self-renewal can occur by either of two major mechanisms. Stem cells may divide asymmetrically, with one daughter retaining the stem state and the other daughter expressing some distinct other specific function and phenotype. Alternatively, some of the stem cells in a population can divide symmetrically into two stems, thus maintaining some stem cells in the population as a whole, while other cells in the population give rise to differentiated progeny only. Formally, it is possible that cells that begin as stem cells might proceed toward a differentiated phenotype, but then "reverse" and re-express the stem cell phenotype, a term often referred to as "dedifferentiation" or "reprogramming" or "retrodifferentiation" by persons of ordinary skill in the art. As used herein, the term "pluripotent stem cell" includes embryonic stem cells, induced pluripotent stem cells, placental stem cells, etc.

In the context of cell ontogeny, the adjective "differentiated", or "differentiating" is a relative term meaning a "differentiated cell" is a cell that has progressed further down the developmental pathway than the cell it is being compared with. Thus, stem cells can differentiate to lineage-restricted precursor cells (such as a mesodermal stem cell), which in turn can differentiate into other types of precursor cells further down the pathway (such as a cardiomyocyte precursor), and then to an end-stage differentiated cell, which plays a characteristic role in a certain tissue type, and may or may not retain the capacity to proliferate further.

The term "embryonic stem cell" is used to refer to the pluripotent stem cells of the inner cell mass of the embryonic blastocyst (see U.S. Pat. Nos. 5,843,780, 6,200,806). Such cells can similarly be obtained from the inner cell mass of blastocysts derived from somatic cell nuclear transfer (see, for example, U.S. Pat. Nos. 5,945,577, 5,994,619, 6,235,970). The distinguishing characteristics of an embryonic stem cell define an embryonic stem cell phenotype. Accordingly, a cell has the phenotype of an embryonic stem cell if it possesses one or more of the unique characteristics of an embryonic stem cell such that that cell can be distinguished from other cells. Exemplary distinguishing embryonic stem cell characteristics include, without limitation, gene expression profile, proliferative capacity, differentiation capacity, karyotype, responsiveness to particular culture conditions, and the like.

The term "adult stem cell" or "ASC" is used to refer to any multipotent stem cell derived from non-embryonic tissue, including fetal, juvenile, and adult tissue. Stem cells have been isolated from a wide variety of adult tissues including blood, bone marrow, brain, olfactory epithelium, skin, pancreas, skeletal muscle, and cardiac muscle. Each of these stem cells can be characterized based on gene expression, factor responsiveness, and morphology in culture. Exemplary adult stem cells include neural stem cells, neural crest stem cells, mesenchymal stem cells, hematopoietic stem cells, and pancreatic stem cells. As indicated above, stem cells have been found resident in virtually every tissue. Accordingly, the present invention appreciates that stem cell populations can be isolated from virtually any animal tissue.

The term "reprogramming" as used herein refers to the process that alters or reverses the differentiation state of a somatic cell. The cell can either be partially or terminally differentiated prior to the reprogramming. Reprogramming encompasses complete reversion of the differentiation state of a somatic cell to a pluripotent cell. Such complete reversal of differentiation produces an induced pluripotent (iPS) cell. Reprogramming as used herein also encompasses partial reversion of a cells differentiation state, for example to a multipotent state or to a somatic cell that is neither pluripotent or multipotent, but is a cell that has lost one or more specific characteristics of the differentiated cell from which it arises, e.g. direct reprogramming of a differentiated cell to a different somatic cell type. Reprogramming generally involves alteration, e.g., reversal, of at least some of the heritable patterns of nucleic acid modification (e.g., methylation), chromatin condensation, epigenetic changes, genomic imprinting, etc., that occur during cellular differentiation as a zygote develops into an adult.

The term "agent" as used herein means any compound or substance such as, but not limited to, a small molecule, nucleic acid, polypeptide, peptide, drug, ion, etc. An "agent" can be any chemical, entity or moiety, including without limitation synthetic and naturally-occurring proteinaceous and non-proteinaceous entities. In some embodiments, an agent is nucleic acid, nucleic acid analogues, proteins, antibodies, peptides, aptamers, oligomer of nucleic acids, amino acids, or carbohydrates including without limitation proteins, oligonucleotides, ribozymes, DNAzymes, glycoproteins, siRNAs, lipoproteins, aptamers, and modifications and combinations thereof etc. In certain embodiments, agents are small molecule having a chemical moiety. For example, chemical moieties included unsubstituted or substituted alkyl, aromatic, or heterocyclyl moieties including macrolides, leptomycins and related natural products or analogues thereof. Compounds can be known to have a desired activity and/or property, or can be selected from a library of diverse compounds.

As used herein, the term "contacting" (i.e., contacting at least one endocrine cell or a precursor thereof with a maturation factor, or combination of maturation factors) is intended to include incubating the maturation factor and the cell together *in vitro* (e.g., adding the maturation factors to cells in culture). In some embodiments, the term "contacting" is not intended to include the *in vivo* exposure of cells to the compounds as disclosed herein that may occur naturally in a subject (i.e., exposure that may occur as a result of a natural physiological process). The step of contacting at least one endocrine cell or a precursor thereof with a maturation factor as in the embodiments described herein can be conducted in any suitable manner. For example, the cells may be treated in adherent culture, or in suspension culture. In some embodiments, the cells are treated in conditions that promote cell clustering. The disclosure contemplates any conditions which promote cell clustering. Examples of conditions that promote cell clustering include, without limitation, suspension culture in low attachment tissue culture plates, spinner flasks, or aggrewell plates. In some embodiments, the inventors have observed that clusters have remained stable in media containing 10% serum. In some embodiments, the conditions that promote clustering include a low serum medium.

It is understood that the cells contacted with a maturation factor can also be simultaneously or subsequently contacted with another agent, such as a growth factor or other differentiation agent or environments to stabilize the cells, or to differentiate the cells further.

The term "cell culture medium" (also referred to herein as a "culture medium" or "medium") as referred to herein is a medium for culturing cells containing nutrients that maintain cell viability and support proliferation. The cell culture medium may contain any of the following in an appropriate combination: salt(s), buffer(s), amino acids, glucose or other sugar(s), antibiotics, serum or serum replacement, and other components such as peptide growth factors, etc. Cell culture media ordinarily used for particular cell types are known to those skilled in the art.

The term "cell line" refers to a population of largely or substantially identical cells that has typically been derived from a single ancestor cell or from a defined and/or substantially identical population of ancestor cells. The cell line may have been or may be capable of being maintained in culture for an extended period (e.g., months, years, for an unlimited period of time). It may have undergone a spontaneous or induced process of transformation conferring an unlimited culture lifespan on the cells. Cell lines include all those cell lines recognized in the art as such. It will be appreciated that cells acquire mutations and possibly epigenetic changes over time such that at least some properties of individual cells of a cell line may differ with respect to each other. In some embodiments, a cell line comprises a stem cell derived cell described herein.

The term "exogenous" refers to a substance present in a cell or organism other than its native source. For example, the terms "exogenous nucleic acid" or "exogenous protein" refer to a nucleic acid or protein that has been introduced by a process involving the hand of man into a biological system such as a cell or organism in which it is not normally found or in which it is found in lower amounts. A substance will be considered exogenous if it is introduced into a cell or an ancestor of the cell that inherits the substance. In contrast, the term "endogenous" refers to a substance that is native to the biological system.

The term "expression" refers to the cellular processes involved in producing RNA and proteins and as appropriate, secreting proteins, including where applicable, but not limited to, for example, transcription, translation, folding, modification and processing. "Expression products" include RNA transcribed from a gene and polypeptides obtained by translation of mRNA transcribed from a gene.

The terms "genetically modified" or "engineered" cell as used herein refers to a cell into which an exogenous nucleic acid has been introduced by a process involving the hand of man (or a descendant of such a cell that has inherited at least a portion of the nucleic acid). The nucleic acid may for example contain a sequence that is exogenous to the cell, it may contain native sequences (i.e., sequences naturally found in the cells) but in a non-naturally occurring arrangement (e.g., a coding region linked to a promoter from a different gene), or altered versions of native sequences, etc. The process of transferring the nucleic acid into the cell can be achieved by any suitable technique. Suitable techniques include calcium phosphate or lipid-mediated transfection, electroporation, and transduction or infection using a viral vector. In some embodiments the polynucleotide or a portion thereof is integrated into the genome of the cell. The nucleic acid may have subsequently been removed or excised from the genome, provided that such removal or excision results in a detectable alteration in the cell relative to an unmodified but otherwise equivalent cell. It should be appreciated that the term genetically modified is intended to include the introduction of a modified RNA directly into a cell (e.g., a synthetic, modified RNA). Such synthetic modified RNAs include modifications to prevent rapid degradation by endo- and exo-nucleases and to avoid or reduce the cell's innate immune or interferon response to the RNA. Modifications include, but are not limited to, for example, (a) end modifications, e.g., 5' end modifications (phosphorylation dephosphorylation, conjugation, inverted linkages, etc.), 3' end modifications (conjugation, DNA nucleotides, inverted linkages, etc.), (b) base modifications, e.g., replacement with modified bases, stabilizing bases, destabilizing bases, or bases that base pair with an expanded repertoire of partners, or conjugated bases, (c) sugar modifications (e.g., at the 2' position or 4' position) or replacement of the sugar, as well as (d) internucleoside linkage modifications, including modification or replacement of the phosphodiester linkages. To the extent that such modifications interfere with translation (i.e., results in a reduction of 50% or more in translation relative to the lack of the modification-e.g., in a rabbit reticulocyte *in vitro* translation assay), the modification is not suitable for the methods and compositions described herein.

The term "identity" as used herein refers to the extent to which the sequence of two or more nucleic acids or polypeptides is the same. The percent identity between a sequence of interest and a second sequence over a window of evaluation, e.g., over the length of the sequence of interest, may be computed by aligning the sequences, determining the number of residues (nucleotides or amino acids) within the window of evaluation that are opposite an identical residue allowing the introduction of gaps to maximize identity, dividing by the total number of residues of the sequence of interest or the second sequence (whichever is greater) that fall within the window, and multiplying by 100. When computing the number of identical residues needed to achieve a particular percent identity, fractions are to be rounded to the nearest whole number. Percent identity can be calculated with the use of a variety of computer programs known in the art. For example, computer programs such as BLAST2, BLASTN, BLASTP, Gapped BLAST, etc., generate alignments and provide percent identity between sequences of interest. The algorithm of Karlin and Altschul (Karlin and Altschul, Proc. Natl. Acad. Sci. USA 87:22264-2268, 1990) modified as in Karlin and Altschul, Proc. Natl. Acad. ScL USA 90:5873-5877, 1993 is incorporated into the NBLAST and XBLAST programs of Altschul et al. (Altschul, et al., J. MoI. Biol. 215:403-410, 1990). To obtain gapped alignments for comparison purposes, Gapped BLAST is utilized as described in Altschul et al. (Altschul, et al. Nucleic Acids Res. 25: 3389-3402, 1997). When utilizing BLAST and Gapped BLAST programs, the default parameters of the respective programs may be used. A PAM250 or BLOSUM62 matrix may be used. Software for performing BLAST analyses is publicly available through the National Center for Biotechnology Information (NCBI). See the Web site having URL world-wide web address of: "ncbi.nlm nih.gov" for these programs. In a specific embodiment, percent identity is calculated using BLAST2 with default parameters as provided by the NCBI.

The term "isolated" or "partially purified" as used herein refers, in the case of a nucleic acid or polypeptide, to a nucleic acid or polypeptide separated from at least one other component (e.g., nucleic acid or polypeptide) that is present with the nucleic acid or polypeptide as found in its natural source and/or that would be present with the nucleic acid or polypeptide when expressed by a cell, or secreted in the case of secreted polypeptides. A chemically synthesized nucleic acid or polypeptide or one synthesized using *in vitro* transcription/translation is considered "isolated".

The term "isolated cell" as used herein refers to a cell that has been removed from an organism in which it was originally found or a descendant of such a cell. Optionally the cell has been cultured *in vitro,* e.g., in the presence of other cells. Optionally the cell is later introduced into a second organism or re-introduced into the organism from which it (or the cell from which it is descended) was isolated.

The term "isolated population" with respect to an isolated population of cells as used herein refers to a population of cells that has been removed and separated from a mixed or heterogeneous population of cells. In some embodiments, an isolated population is a substantially pure population of cells as compared to the heterogeneous population from which the cells were isolated or enriched from.

The term "substantially pure", with respect to a particular cell population, refers to a population of cells that is at least about 75%, preferably at least about 85%, more preferably at least about 90%, and most preferably at least about 95% pure, with respect to the cells making up a total cell population.

The terms "enriching" or "enriched" are used interchangeably herein and mean that the yield (fraction) of cells of one type is increased by at least 10% over the fraction of cells of that type in the starting culture or preparation.

The terms "renewal" or "self-renewal" or "proliferation" are used interchangeably herein, and are used to refer to the ability of stem cells to renew themselves by dividing into the same non-specialized cell type over long periods, and/or many months to years. In some instances, proliferation refers to the expansion of cells by the repeated division of single cells into two identical daughter cells.

The term "lineages" as used herein describes a cell with a common ancestry or cells with a common developmental fate. For example, in the context of a cell that is of endoderm origin or is "endodermal linage" this means the cell was derived from an endoderm cell and can differentiate along the endoderm lineage restricted pathways, such as one or more developmental lineage pathways which give rise to definitive endoderm cells, which in turn can differentiate into liver cells, thymus, pancreas, lung and intestine.

As used herein, the term "xenogeneic" refers to cells that are derived from different species.

A "marker" as used herein is used to describe the characteristics and/or phenotype of a cell. Markers can be used for selection of cells comprising characteristics of interests. Markers will vary with specific cells. Markers are characteristics, whether morphological, functional or biochemical (enzymatic) characteristics of the cell of a particular cell type, or molecules expressed by the cell type. Preferably, such markers are proteins, and more preferably, possess an epitope for antibodies or other binding molecules available in the art. However, a marker may consist of any molecule found in a cell including, but not limited to, proteins (peptides and polypeptides), lipids, polysaccharides, nucleic acids and steroids. Examples of morphological characteristics or traits include, but are not limited to, shape, size, and nuclear to cytoplasmic ratio. Examples of functional characteristics or traits include, but are not limited to, the ability to adhere to particular substrates, ability to incorporate or exclude particular dyes, ability to migrate under particular conditions, and the ability to differentiate along particular lineages. Markers may be detected by any method available to one of skill in the art. Markers can also be the absence of a morphological characteristic or absence of proteins, lipids etc. Markers can be a combination of a panel of unique characteristics of the presence and absence of polypeptides and other morphological characteristics.

The term "modulate" is used consistently with its use in the art, i.e., meaning to cause or facilitate a qualitative or quantitative change, alteration, or modification in a process, pathway, or phenomenon of interest. Without limitation, such change may be an increase, decrease, or change in relative strength or activity of different components or branches of the process, pathway, or phenomenon. A "modulator" is an agent that causes or facilitates a qualitative or quantitative change, alteration, or modification in a process, pathway, or phenomenon of interest.

As used herein, the term "DNA" is defined as deoxyribonucleic acid.

The term "polynucleotide" is used herein interchangeably with "nucleic acid" to indicate a polymer of nucleosides. Typically a polynucleotide of this invention is composed of nucleosides that are naturally found in DNA or RNA (e.g., adenosine, thymidine, guanosine, cytidine, uridine, deoxyadenosine, deoxythymidine, deoxyguanosine, and deoxycytidine) joined by phosphodiester bonds. However the term encompasses molecules comprising nucleosides or nucleoside analogs containing chemically or biologically modified bases, modified backbones, etc., whether or not found in naturally occurring nucleic acids, and such molecules may be preferred for certain applications. Where this application refers to a polynucleotide it is understood that both DNA, RNA, and in each case both single- and double-stranded forms (and complements of each single-stranded molecule) are provided. "Polynucleotide sequence" as used herein can refer to the polynucleotide material itself and/or to the sequence information (i.e. the succession of letters used as abbreviations for bases) that biochemically characterizes a specific nucleic acid. A polynucleotide sequence presented herein is presented in a 5' to 3' direction unless otherwise indicated.

The terms "polypeptide" as used herein refers to a polymer of amino acids. The terms "protein" and "polypeptide" are used interchangeably herein. A peptide is a relatively short polypeptide, typically between about 2 and 60 amino acids in length. Polypeptides used herein typically contain amino acids such as the 20 L-amino acids that are most commonly found in proteins. However, other amino acids and/or amino acid analogs known in the art can be used. One or more of the amino acids in a polypeptide may be modified, for example, by the addition of a chemical entity such as a carbohydrate group, a phosphate group, a fatty acid group, a linker for conjugation, functionalization, etc. A polypeptide that has a non-polypeptide moiety covalently or non-covalently associated therewith is still considered a "polypeptide". Exemplary modifications include glycosylation and palmitoylation. Polypeptides may be purified from natural sources, produced using recombinant DNA technology, synthesized through chemical means such as conventional solid phase peptide synthesis, etc. The term "polypeptide sequence" or "amino acid sequence" as used herein can refer to the polypeptide material itself and/or to the sequence information (i.e., the succession of letters or three letter codes used as abbreviations for amino acid names) that biochemically characterizes a polypeptide. A polypeptide sequence presented herein is presented in an N-terminal to C-terminal direction unless otherwise indicated.

The term a "variant" in referring to a polypeptide could be, e.g., a polypeptide at least 80%, 85%, 90%, 95%, 98%, or 99% identical to full length polypeptide. The variant could be a fragment of full length polypeptide. The variant could be a naturally occurring splice variant. The variant could be a polypeptide at least 80%, 85%, 90%, 95%, 98%, or 99% identical to a fragment of the polypeptide, wherein the fragment is at least 50%, 60%, 70%, 80%, 85%, 90%, 95%, 98%, or 99% as long as the full length wild type polypeptide or a domain thereof having an activity of interest. In some embodiments the domain is at least 100, 200, 300, or 400 amino acids in length, beginning at any amino acid position in the sequence and extending toward the C-terminus. Variations known in the art to eliminate or substantially reduce the activity of the protein are preferably avoided. In some embodiments, the variant lacks an N- and/or C-terminal portion of the full length polypeptide, e.g., up to 10, 20, or 50 amino acids from either terminus is lacking. In some embodiments the polypeptide has the sequence of a mature (full length) polypeptide, by which is meant a polypeptide that has had one or more portions such as a signal peptide removed during normal intracellular proteolytic processing (e.g., during co-translational or post-translational processing). In some embodiments wherein the protein is produced other than by purifying it from cells that naturally express it, the protein is a chimeric polypeptide, by which is meant that it contains portions from two or more different species. In some embodiments wherein a protein is produced other than by purifying it from cells that naturally express it, the protein is a derivative, by which is meant that the protein comprises additional sequences not related to the protein so long as those sequences do not substantially reduce the biological activity of the protein.

The term "functional fragments" as used herein is a polypeptide having an amino acid sequence which is smaller in size than, but substantially homologous to the polypeptide it is a fragment of, and where the functional fragment polypeptide sequence is about at least 50%, or 60% or 70% or 80% or 90% or 100% or greater than 100%, for example 1.5-fold, 2-fold, 3-fold, 4-fold or greater than 4-fold effective biological action as the polypeptide from which it is a fragment of. Functional fragment polypeptides may have additional functions that can include decreased antigenicity, increased DNA binding (as in transcription factors), or altered RNA binding (as in regulating RNA stability or degradation).

The term "vector" refers to a carrier DNA molecule into which a DNA sequence can be inserted for introduction into a host cell. Preferred vectors are those capable of autonomous replication and/or expression of nucleic acids to which they are linked. Vectors capable of directing the expression of genes to which they are operatively linked are referred to herein as "expression vectors". Thus, an "expression vector" is a specialized vector that contains the necessary regulatory regions needed for expression of a gene of interest in a host cell. In some embodiments the gene of interest is operably linked to another sequence in the vector. Vectors can be viral vectors or non-viral vectors. Should viral vectors be used, it is preferred the viral vectors are replication defective, which can be achieved for example by removing all viral nucleic acids that encode for replication. A replication defective viral vector will still retain its infective properties and enters the cells in a similar manner as a replicating adenoviral vector, however once admitted to the cell a replication defective viral vector does not reproduce or multiply. Vectors also encompass liposomes and nanoparticles and other means to deliver DNA molecule to a cell.

The term "operably linked" means that the regulatory sequences necessary for expression of the coding sequence are placed in the DNA molecule in the appropriate positions relative to the coding sequence so as to effect expression of the coding sequence. This same definition is sometimes applied to the arrangement of coding sequences and transcription control elements (e.g. promoters, enhancers, and termination elements) in an expression vector. The term "operatively linked" includes having an appropriate start signal (e.g., ATG) in front of the polynucleotide sequence to be expressed, and maintaining the correct reading frame to permit expression of the polynucleotide sequence under the control of the expression control sequence, and production of the desired polypeptide encoded by the polynucleotide sequence.

The term "viral vectors" refers to the use of viruses, or virus-associated vectors as carriers of a nucleic acid construct into a cell. Constructs may be integrated and packaged into non-replicating, defective viral genomes like Adenovirus, Adeno-associated virus (AAV), or Herpes simplex virus (HSV) or others, including retroviral and lentiviral vectors, for infection or transduction into cells. The vector may or may not be incorporated into the cell's genome. The constructs may include viral sequences for transfection, if desired. Alternatively, the construct may be incorporated into vectors capable of episomal replication, e.g EPV and EBV vectors.

The terms "regulatory sequence" and "promoter" are used interchangeably herein, and refer to nucleic acid sequences, such as initiation signals, enhancers, and promoters, which induce or control transcription of protein coding sequences with which they are operatively linked. In some examples, transcription of a recombinant gene is under the control of a promoter sequence (or other transcriptional regulatory sequence) which controls the expression of the recombinant gene in a cell-type in which expression is intended. It will also be understood that the recombinant gene can be under the control of transcriptional regulatory sequences which are the same or which are different from those sequences which control transcription of the naturally-occurring form of a protein. In some instances the promoter sequence is recognized by the synthetic machinery of the cell, or introduced synthetic machinery, required for initiating transcription of a specific gene.

As used herein, the term "transcription factor" refers to a protein that binds to specific parts of DNA using DNA binding domains and is part of the system that controls the transfer (or transcription) of genetic information from DNA to RNA. As used herein, "proliferating" and "proliferation" refer to an increase in the number of cells in a population (growth) by means of cell division. Cell proliferation is generally understood to result from the coordinated activation of multiple signal transduction pathways in response to the environment, including growth factors and other mitogens. Cell proliferation may also be promoted by release from the actions of intra- or extracellular signals and mechanisms that block or negatively affect cell proliferation.

The term "selectable marker" refers to a gene, RNA, or protein that when expressed, confers upon cells a selectable phenotype, such as resistance to a cytotoxic or cytostatic agent (e.g., antibiotic resistance), nutritional prototrophy, or expression of a particular protein that can be used as a basis to distinguish cells that express the protein from cells that do not. Proteins whose expression can be readily detected such as a fluorescent or luminescent protein or an enzyme that acts on a substrate to produce a colored, fluorescent, or luminescent substance ("detectable markers") constitute a subset of selectable markers. The presence of a selectable marker linked to expression control elements native to a gene that is normally expressed selectively or exclusively in pluripotent cells makes it possible to identify and select somatic cells that have been reprogrammed to a pluripotent state. A variety of selectable marker genes can be used, such as neomycin resistance gene (neo), puromycin resistance gene (puro), guanine phosphoribosyl transferase (gpt), dihydrofolate reductase (DHFR), adenosine deaminase (ada), puromycin-N-acetyltransferase (PAC), hygromycin resistance gene (hyg), multidrug resistance gene (mdr), thymidine kinase (TK), hypoxanthine-guanine phosphoribosyltransferase (HPRT), and hisD gene. Detectable markers include green fluorescent protein (GFP) blue, sapphire, yellow, red, orange, and cyan fluorescent proteins and variants of any of these. Luminescent proteins such as *luciferase* (e.g., firefly or *Renilla luciferase*) are also of use. As will be evident to one of skill in the art, the term "selectable marker" as used herein can refer to a gene or to an expression product of the gene, e.g., an encoded protein.

In some embodiments the selectable marker confers a proliferation and/or survival advantage on cells that express it relative to cells that do not express it or that express it at significantly lower levels. Such proliferation and/or survival advantage typically occurs when the cells are maintained under certain conditions, i.e., "selective conditions." To ensure an effective selection, a population of cells can be maintained under conditions and for a sufficient period of time such that cells that do not express the marker do not proliferate and/or do not survive and are eliminated from the population or their number is reduced to only a very small fraction of the population. The process of selecting cells that express a marker that confers a proliferation and/or survival advantage by maintaining a population of cells under selective conditions so as to largely or completely eliminate cells that do not express the marker is referred to herein as "positive selection", and the marker is said to be "useful for positive selection". Negative selection and markers useful for negative selection are also of interest in certain of the methods described herein. Expression of such markers confers a proliferation and/or survival disadvantage on cells that express the marker relative to cells that do not express the marker or express it at significantly lower levels (or, considered another way, cells that do not express the marker have a proliferation and/or survival advantage relative to cells that express the marker). Cells that express the marker can therefore be largely or completely eliminated from a population of cells when maintained in selective conditions for a sufficient period of time.

A "reporter gene" as used herein encompasses any gene that is genetically introduced into a cell that adds to the phenotype of the stem cell. Reporter genes as disclosed in this invention are intended to encompass fluorescent, luminescent, enzymatic and resistance genes, but also other genes which can easily be detected by persons of ordinary skill in the art. In some embodiments of the invention, reporter genes are used as markers for the identification of particular stem cells, cardiovascular stem cells and their differentiated progeny. A reporter gene is generally operatively linked to sequences that regulate its expression in a manner dependent upon one or more conditions which are monitored by measuring expression of the reporter gene. In some cases, expression of the reporter gene may be determined in live cells. Where live cell reporter gene assays are used, reporter gene expression may be monitored at multiple time points, e.g., 2, 3, 4, 5, 6, 8, or 10 or more time points. In some cases, where a live cell reporter assay is used, reporter gene expression is monitored with a frequency of at least about 10 minutes to about 24 hours, e.g., 20 minutes, 1 hour, 2 hours, 3 hours, 4 hours, 5 hours, 6 hours, 7 hours, 8 hours, 9 hours, 10 hours, 12 hours, 18 hours, or another frequency from any integer between about 10 minutes to about 24 hours.

The terms "subject" and "individual" are used interchangeably herein, and refer to an animal, for example, a human from whom cells can be obtained and/or to whom treatment, including prophylactic treatment, with the cells as described herein, is provided. For treatment of those infections, conditions or disease states which are specific for a specific animal such as a human subject, the term subject refers to that specific animal. The "non-human animals" and "non-human mammals" as used interchangeably herein, includes mammals such as rats, mice, rabbits, sheep, cats, dogs, cows, pigs, and non-human primates. The term "subject" also encompasses any vertebrate including but not limited to mammals, reptiles, amphibians and fish. However, advantageously, the subject is a mammal such as a human, or other mammals such as a domesticated mammal, e.g. dog, cat, horse, and the like, or production mammal, e.g. cow, sheep, pig, and the like.

The terms "treat", "treating", "treatment", etc., as applied to an isolated cell, include subjecting the cell to any kind of process or condition or performing any kind of manipulation or procedure on the cell. As applied to a subject, the terms refer to providing medical or surgical attention, care, or management to an individual. The individual is usually ill or injured, or at increased risk of becoming ill relative to an average member of the population and in need of such attention, care, or management.

As used herein, the term "treating" and "treatment" refers to administering to a subject an effective amount of a composition so that the subject as a reduction in at least one symptom of the disease or an improvement in the disease, for example, beneficial or desired clinical results. For purposes of this invention, beneficial or desired clinical results include, but are not limited to, alleviation of one or more symptoms, diminishment of extent of disease, stabilized (i.e., not worsening) state of disease, delay or slowing of disease progression, amelioration or palliation of the disease state, and remission (whether partial or total), whether detectable or undetectable. Treating can refer to prolonging survival as compared to expected survival if not receiving treatment. Thus, one of skill in the art realizes that a treatment may improve the disease condition, but may not be a complete cure for the disease. As used herein, the term "treatment" includes prophylaxis. Alternatively, treatment is "effective" if the progression of a disease is reduced or halted. "Treatment" can also mean prolonging survival as compared to expected survival if not receiving treatment.

As used herein, the terms "administering," "introducing" and "transplanting" are used interchangeably in the context of the placement of cells of the invention into a subject, by a method or route which results in at least partial localization of the introduced cells at a desired site. The cells can be implanted directly to the pancreas or gastrointestinal tract, or alternatively be administered by any appropriate route which results in delivery to a desired location in the subject where at least a portion of the implanted cells or components of the cells remain viable. The period of viability of the cells after administration to a subject can be as short as a few hours, e.g. twenty-four hours, to a few days, to as long as several years. In some instances, the cells can also be administered subcutaneously, for example, in a capsule (e.g., microcapsule) to maintain the implanted cells at the implant location and avoid migration of the implanted cells.

The phrases "parenteral administration" and "administered parenterally" as used herein means modes of administration other than enteral and topical administration, usually by injection, and includes, without limitation, intravenous, intramuscular, intraarterial, intrathecal, intraventricular, intracapsular, intraorbital, intracardiac, intradermal, intraperitoneal, transtracheal, subcutaneous, subcuticular, intraarticular, subcapsular, subarachnoid, intraspinal, intracerebro spinal, and intrasternal injection and infusion. The phrases "systemic administration," "administered systemically", "peripheral administration" and "administered peripherally" as used herein mean the administration of stem cell-derived cells and/or their progeny and/or compound and/or other material other than directly into the central nervous system, such that it enters the animal's system and, thus, is subject to metabolism and other like processes, for example, subcutaneous administration.

The term "tissue" refers to a group or layer of specialized cells which together perform certain special functions. The term "tissue-specific" refers to a source of cells from a specific tissue.

The terms "decrease", "reduced", "reduction", "decrease" or "inhibit" are all used herein generally to mean a decrease by a statistically significant amount. However, for avoidance of doubt, "reduced", "reduction" or "decrease" or "inhibit" means a decrease by at least 10% as compared to a reference level, for example a decrease by at least about 20%, or at least about 30%, or at least about 40%, or at least about 50%, or at least about 60%, or at least about 70%, or at least about 80%, or at least about 90% or up to and including a 100% decrease (i.e. absent level as compared to a reference sample), or any decrease between 10-100% as compared to a reference level.

The terms "increased", "increase" or "enhance" or "activate" are all used herein to generally mean an increase by a statically significant amount; for the avoidance of any doubt, the terms "increased", "increase" or "enhance" or "activate" means an increase of at least 10% as compared to a reference level, for example an increase of at least about 20%, or at least about 30%, or at least about 40%, or at least about 50%, or at least about 60%, or at least about 70%, or at least about 80%, or at least about 90% or up to and including a 100% increase or any increase between 10-100% as compared to a reference level, or at least about a 2-fold, or at least about a 3-fold, or at least about a 4-fold, or at least about a 5-fold or at least about a 10-fold increase, or any increase between 2-fold and 10-fold or greater as compared to a reference level.

The term "statistically significant" or "significantly" refers to statistical significance and generally means a two standard deviation (2SD) below normal, or lower, concentration of the marker. The term refers to statistical evidence that there is a difference. It is defined as the probability of making a decision to reject the null hypothesis when the null hypothesis is actually true. The decision is often made using the p-value.

As used herein the term "comprising" or "comprises" is used in reference to compositions, methods, and respective component(s) thereof, that are essential to the invention, yet open to the inclusion of unspecified elements, whether essential or not.

As used herein the term "consisting essentially of' refers to those elements required for a given embodiment. The term permits the presence of additional elements that do not materially affect the basic and novel or functional characteristic(s) of that embodiment of the invention.

The term "consisting of" refers to compositions, methods, and respective components thereof as described herein, which are exclusive of any element not recited in that description of the embodiment.

As used in this specification and the appended claims, the singular forms "a," "an," and "the" include plural references unless the context clearly dictates otherwise. Thus for example, references to "the method" includes one or more methods, and/or steps of the type described herein and/or which will become apparent to those persons skilled in the art upon reading this disclosure and so forth.

### Stem Cells

Stem cells are cells that retain the ability to renew themselves through mitotic cell division and can differentiate into a diverse range of specialized cell types. The two broad types of mammalian stem cells are: embryonic stem (ES) cells that are found in blastocysts, and adult stem cells that are found in adult tissues. In a developing embryo, stem cells can differentiate into all of the specialized embryonic tissues. In adult organisms, stem cells and progenitor cells act as a repair system for the body, replenishing specialized cells, but also maintain the normal turnover of regenerative organs, such as blood, skin or intestinal tissues. Pluripotent stem cells can differentiate into cells derived from any of the three germ layers.

While certain embodiments are described below in reference to the use of stem cells, germ cells may be used in place of, or with, the stem cells to provide at least one differentiated cell, using similar protocols as the illustrative protocols described herein. Suitable germ cells can be prepared, for example, from primordial germ cells present in human fetal material taken about 8-11 weeks after the last menstrual period. Illustrative germ cell preparation methods are described, for example, in Shamblott et al., Proc. Natl. Acad. Sci. USA 95:13726, 1998 and U.S. Pat. No. 6,090,622.

ES cells, e.g., human embryonic stem cells (hESCs) or mouse embryonic stem cells (mESCs), with a virtually endless replication capacity and the potential to differentiate into most cell types, present, in principle, an unlimited starting material to generate the differentiated cells for clinical therapy (stemcells.nih.gov/info/scireport/2006report.htm, 2006).

hESC cells, are described, for example, by Cowan et al. (N Engl. J. Med. 350:1353, 2004) and Thomson et al. (Science 282:1145, 1998); embryonic stem cells from other primates, Rhesus stem cells (Thomson et al., Proc. Natl. Acad. Sci. USA 92:7844, 1995), marmoset stem cells (Thomson et al., Biol. Reprod. 55:254, 1996) and human embryonic germ (hEG) cells (Shamblott et al., Proc. Natl. Acad. Sci. USA 95:13726, 1998) may also be used in the methods disclosed herein. mESCs, are described, for example, by Tremml et al. (Curr Protoc Stem Cell Biol. Chapter 1:Unit 1C.4, 2008). The stem cells may be, for example, unipotent, totipotent, multipotent, or pluripotent. In some examples, any cells of primate origin that are capable of producing progeny that are derivatives of at least one germinal layer, or all three germinal layers, may be used in the methods disclosed herein.

In certain examples, ES cells may be isolated, for example, as described in Cowan et al. (N Engl. J. Med. 350:1353, 2004) and U.S. Pat. No. 5,843,780 and Thomson et al., Proc. Natl. Acad. Sci. USA 92:7844, 1995. For example, hESCs cells can be prepared from human blastocyst cells using the techniques described by Thomson et al. (U.S. Pat. No. 6,200,806; Science 282:1145, 1998; Curr. Top. Dev. Biol. 38:133 ff., 1998) and Reubinoff et al, Nature Biotech. 18:399, 2000. Equivalent cell types to hESCs include their pluripotent derivatives, such as primitive ectoderm-like (EPL) cells, as outlined, for example, in WO 01/51610 (Bresagen). hESCs can also be obtained from human pre-implantation embryos. Alternatively, *in vitro* fertilized (IVF) embryos can be used, or one-cell human embryos can be expanded to the blastocyst stage (Bongso et al., Hum Reprod 4: 706, 1989). Embryos are cultured to the blastocyst stage in G1.2 and G2.2 medium (Gardner et al., Fertil. Steril. 69:84, 1998). The zona pellucida is removed from developed blastocysts by brief exposure to pronase (Sigma). The inner cell masses can be isolated by immunosurgery, in which blastocysts are exposed to a 1:50 dilution of rabbit anti-human spleen cell antiserum for 30 min, then washed for 5 min three times in DMEM, and exposed to a 1:5 dilution of Guinea pig complement (Gibco) for 3 min (Solter et al., Proc. Natl. Acad. Sci. USA 72:5099, 1975). After two further washes in DMEM, lysed trophectoderm cells are removed from the intact inner cell mass (ICM) by gentle pipetting, and the ICM plated on mEF feeder layers. After 9 to 15 days, inner cell mass-derived outgrowths can be dissociated into clumps, either by exposure to calcium and magnesium-free phosphate-buffered saline (PBS) with 1 mM EDTA, by exposure to dispase or trypsin, or by mechanical dissociation with a micropipette; and then replated on mEF in fresh medium. Growing colonies having undifferentiated morphology can be individually selected by micropipette, mechanically dissociated into clumps, and replated. ES-like morphology is characterized as compact colonies with apparently high nucleus to cytoplasm ratio and prominent nucleoli. Resulting hESCs can then be routinely split every 1-2 weeks, for example, by brief trypsinization, exposure to Dulbecco's PBS (containing 2 mM EDTA), exposure to type IV collagenase (about 200 U/mL; Gibco) or by selection of individual colonies by micropipette. In some examples, clump sizes of about 50 to 100 cells are optimal. mESCs cells can be prepared from using the techniques described by e.g., Conner et al. (Curr. Prot. in Mol. Biol. Unit 23.4, 2003).

Embryonic stem cells can be isolated from blastocysts of members of the primate species (U.S. Pat. No. 5,843,780; Thomson et al., Proc. Natl. Acad. Sci. USA 92:7844, 1995). Human embryonic stem (hES) cells can be prepared from human blastocyst cells using the techniques described by Thomson et al. (U.S. Pat. No. 6,200,806; Science 282:1145, 1998; Curr. Top. Dev. Biol. 38:133 ff., 1998) and Reubinoff et al, Nature Biotech. 18:399, 2000. Equivalent cell types to hES cells include their pluripotent derivatives, such as primitive ectoderm-like (EPL) cells, as outlined in WO 01/51610 (Bresagen).

Alternatively, in some embodiments, hES cells can be obtained from human preimplantation embryos. Alternatively, *in vitro* fertilized (IVF) embryos can be used, or one-cell human embryos can be expanded to the blastocyst stage (Bongso et al., Hum Reprod 4: 706, 1989). Embryos are cultured to the blastocyst stage in G1.2 and G2.2 medium (Gardner et al., Fertil. Steril. 69:84, 1998). The zona pellucida is removed from developed blastocysts by brief exposure to pronase (Sigma). The inner cell masses are isolated by immunosurgery, in which blastocysts are exposed to a 1:50 dilution of rabbit anti-human spleen cell antiserum for 30 min, then washed for 5 min three times in DMEM, and exposed to a 1:5 dilution of Guinea pig complement (Gibco) for 3 min (Solter et al., Proc. Natl. Acad. Sci. USA 72:5099, 1975). After two further washes in DMEM, lysed trophectoderm cells are removed from the intact inner cell mass (ICM) by gentle pipetting, and the ICM plated on mEF feeder layers.

After 9 to 15 days, inner cell mass-derived outgrowths are dissociated into clumps, either by exposure to calcium and magnesium-free phosphate-buffered saline (PBS) with 1 mM EDTA, by exposure to dispase or trypsin, or by mechanical dissociation with a micropipette; and then replated on mEF in fresh medium. Growing colonies having undifferentiated morphology are individually selected by micropipette, mechanically dissociated into clumps, and replated. ES-like morphology is characterized as compact colonies with apparently high nucleus to cytoplasm ratio and prominent nucleoli. Resulting ES cells are then routinely split every 1-2 weeks by brief trypsinization, exposure to Dulbecco's PBS (containing 2 mM EDTA), exposure to type IV collagenase (~200 U/mL; Gibco) or by selection of individual colonies by micropipette. Clump sizes of about 50 to 100 cells are optimal.

In some embodiments, human Embryonic Germ (hEG) cells are pluripotent stem cells which can be used in the methods as disclosed herein to differentiate into primitive endoderm cells. hEG cells can be prepared from primordial germ cells present in human fetal material taken about 8-11 weeks after the last menstrual period. Suitable preparation methods are described in Shamblott et al., Proc. Natl. Acad. Sci. USA 95:13726, 1998 and U.S. Pat. No. 6,090,622, which is incorporated herein in its entirety by reference.

Briefly, genital ridges are processed to form disaggregated cells. EG growth medium is DMEM, 4500 mg/L D-glucose, 2200 mg/L mM NaHCO₃; 15% ES qualified fetal calf serum (BRL); 2 mM glutamine (BRL); 1 mM sodium pyruvate (BRL); 1000-2000 U/mL human recombinant leukemia inhibitory factor (LIF, Genzyme); 1-2 ng/mL human recombinant bFGF (Genzyme); and 10 µM forskolin (in 10% DMSO). Ninety-six well tissue culture plates are prepared with a sub-confluent layer of feeder cells (e.g., STO cells, ATCC No. CRL 1503) cultured for 3 days in modified EG growth medium free of LIF, bFGF or forskolin, inactivated with 5000 rad γ-irradiation ~0.2 mL of primary germ cell (PGC) suspension is added to each of the wells. The first passage is done after 7-10 days in EG growth medium, transferring each well to one well of a 24-well culture dish previously prepared with irradiated STO mouse fibroblasts. The cells are cultured with daily replacement of medium until cell morphology consistent with EG cells is observed, typically after 7-30 days or 1-4 passages.

In certain examples, the stem cells can be undifferentiated (e.g. a cell not committed to a specific lineage) prior to exposure to at least one maturation factor according to the methods as disclosed herein, whereas in other examples it may be desirable to differentiate the stem cells to one or more intermediate cell types prior to exposure of the at least one maturation factor (s) described herein. For example, the stem cells may display morphological, biological or physical characteristics of undifferentiated cells that can be used to distinguish them from differentiated cells of embryo or adult origin. In some examples, undifferentiated cells may appear in the two dimensions of a microscopic view in colonies of cells with high nuclear/cytoplasmic ratios and prominent nucleoli. The stem cells may be themselves (for example, without substantially any undifferentiated cells being present) or may be used in the presence of differentiated cells. In certain examples, the stem cells may be cultured in the presence of suitable nutrients and optionally other cells such that the stem cells can grow and optionally differentiate. For example, embryonic fibroblasts or fibroblast-like cells may be present in the culture to assist in the growth of the stem cells. The fibroblast may be present during one stage of stem cell growth but not necessarily at all stages. For example, the fibroblast may be added to stem cell cultures in a first culturing stage and not added to the stem cell cultures in one or more subsequent culturing stages.

Stem cells used in all aspects of the present invention can be any cells derived from any kind of tissue (for example embryonic tissue such as fetal or pre-fetal tissue, or adult tissue), which stem cells have the characteristic of being capable under appropriate conditions of producing progeny of different cell types, e.g. derivatives of all of at least one of the 3 germinal layers (endoderm, mesoderm, and ectoderm). These cell types may be provided in the form of an established cell line, or they may be obtained directly from primary embryonic tissue and used immediately for differentiation. Included are cells listed in the NIH Human Embryonic Stem Cell Registry, e.g. hESBGN-01, hESBGN-02, hESBGN-03, hESBGN-04 (BresaGen, Inc.); HES-1, HES-2, HES-3, HES-4, HES-5, HES-6 (ES Cell International); Miz-hES1 (MizMedi Hospital-Seoul National University); HSF-1, HSF-6 (University of California at San Francisco); and H1, H7, H9, H13, H14 (Wisconsin Alumni Research Foundation (WiCell Research Institute)). In some embodiments, the source of human stem cells or pluripotent stem cells used for chemically-induced differentiation into stem cell-derived cells did not involve destroying a human embryo.

In another embodiment, the stem cells can be isolated from tissue including solid tissue. In some embodiments, the tissue is skin, fat tissue (e.g. adipose tissue), muscle tissue, heart or cardiac tissue. In other embodiments, the tissue is for example but not limited to, umbilical cord blood, placenta, bone marrow, or chondral.

Stem cells of interest also include embryonic cells of various types, exemplified by human embryonic stem (hES) cells, described by Thomson et al. (1998) Science 282:1145; embryonic stem cells from other primates, such as Rhesus stem cells (Thomson et al. (1995) Proc. Natl. Acad. Sci. USA 92:7844); marmoset stem cells (Thomson et al. (1996) Biol. Reprod. 55:254); and human embryonic germ (hEG) cells (Shambloft et al., Proc. Natl. Acad. Sci. USA 95:13726, 1998). Also of interest are lineage committed stem cells, such as mesodermal stem cells and other early cardiogenic cells (see Reyes et al. (2001) Blood 98:2615-2625; Eisenberg & Bader (1996) Circ Res. 78(2):205-16; etc.) The stem cells may be obtained from any mammalian species, e.g. human, equine, bovine, porcine, canine, feline, rodent, e.g. mice, rats, hamster, primate, etc. In some embodiments, a human embryo was not destroyed for the source of pluripotent cell used on the methods and compositions as disclosed herein.

ES cells are considered to be undifferentiated when they have not committed to a specific differentiation lineage. Such cells display morphological characteristics that distinguish them from differentiated cells of embryo or adult origin. Undifferentiated ES cells are easily recognized by those skilled in the art, and typically appear in the two dimensions of a microscopic view in colonies of cells with high nuclear/cytoplasmic ratios and prominent nucleoli. Undifferentiated ES cells express genes that may be used as markers to detect the presence of undifferentiated cells, and whose polypeptide products may be used as markers for negative selection. For example, see U.S. application Ser. No. 2003/0224411 A1; Bhattacharya (2004) Blood 103(8):2956-64; and Thomson (1998), supra., each herein incorporated by reference. Human ES cell lines express cell surface markers that characterize undifferentiated nonhuman primate ES and human EC cells, including stage-specific embryonic antigen (SSEA)-3, SSEA-4, TRA-1-60, TRA-1-81, and alkaline phosphatase. The globo-series glycolipid GL7, which carries the SSEA-4 epitope, is formed by the addition of sialic acid to the globo-series glycolipid GbS, which carries the SSEA-3 epitope. Thus, GL7 reacts with antibodies to both SSEA-3 and SSEA-4. The undifferentiated human ES cell lines did not stain for SSEA-1, but differentiated cells stained strongly for SSEA-I. Methods for proliferating hES cells in the undifferentiated form are described in WO 99/20741, WO 01/51616, and WO 03/020920.

A mixture of cells from a suitable source of endothelial, muscle, and/or neural stem cells can be harvested from a mammalian donor by methods known in the art. A suitable source is the hematopoietic microenvironment. For example, circulating peripheral blood, preferably mobilized (i.e., recruited), may be removed from a subject. Alternatively, bone marrow may be obtained from a mammal, such as a human patient, undergoing an autologous transplant. In some embodiments, stem cells can be obtained from the subjects adipose tissue, for example using the CELUTION^{™} SYSTEM from Cytori, as disclosed in U.S. Pat. Nos. 7,390,484 and 7,429,488 which is incorporated herein in its entirety by reference.

In some embodiments, human umbilical cord blood cells (HUCBC) are useful in the methods as disclosed herein. Human UBC cells are recognized as a rich source of hematopoietic and mesenchymal progenitor cells (Broxmeyer et al., 1992 Proc. Natl. Acad. Sci. USA 89:4109-4113). Previously, umbilical cord and placental blood were considered a waste product normally discarded at the birth of an infant. Cord blood cells are used as a source of transplantable stem and progenitor cells and as a source of marrow repopulating cells for the treatment of malignant diseases (i.e. acute lymphoid leukemia, acute myeloid leukemia, chronic myeloid leukemia, myelodysplastic syndrome, and nueroblastoma) and non-malignant diseases such as Fanconi's anemia and aplastic anemia (Kohli-Kumar et al., 1993 Br. J. Haematol. 85:419-422; Wagner et al., 1992 Blood 79; 1874-1881; Lu et al., 1996 Crit. Rev. Oncol. Hematol 22:61-78; Lu et al., 1995 Cell Transplantation 4:493-503). A distinct advantage of HUCBC is the immature immunity of these cells that is very similar to fetal cells, which significantly reduces the risk for rejection by the host (Taylor & Bryson, 1985J. Immunol. 134:1493-1497). Human umbilical cord blood contains mesenchymal and hematopoietic progenitor cells, and endothelial cell precursors that can be expanded in tissue culture (Broxmeyer et al., 1992 Proc. Natl. Acad. Sci. USA 89:4109-4113; Kohli-Kumar et al., 1993 Br. J. Haematol. 85:419-422; Wagner et al., 1992 Blood 79; 1874-1881; Lu et al., 1996 Crit. Rev. Oncol. Hematol 22:61-78; Lu et al., 1995 Cell Transplantation 4:493-503; Taylor & Bryson, 1985J. Immunol. 134:1493-1497 Broxmeyer, 1995 Transfusion 35:694-702; Chen et al., 2001 Stroke 32:2682-2688; Nieda et al., 1997 Br. J. Haematology 98:775-777; Erices et al., 2000 Br. J. Haematology 109:235-242). The total content of hematopoietic progenitor cells in umbilical cord blood equals or exceeds bone marrow, and in addition, the highly proliferative hematopoietic cells are eightfold higher in HUCBC than in bone marrow and express hematopoietic markers such as CD14, CD34, and CD45 (Sanchez-Ramos et al., 2001 Exp. Neur. 171:109-115; Bicknese et al., 2002 Cell Transplantation 11:261-264; Lu et al., 1993 J. Exp Med. 178:2089-2096).

In another embodiment, pluripotent cells are cells in the hematopoietic microenvironment, such as the circulating peripheral blood, preferably from the mononuclear fraction of peripheral blood, umbilical cord blood, bone marrow, fetal liver, or yolk sac of a mammal. The stem cells, especially neural stem cells, may also be derived from the central nervous system, including the meninges.

In another embodiment, pluripotent cells are present in embryoid bodies are formed by harvesting ES cells with brief protease digestion, and allowing small clumps of undifferentiated human ESCs to grow in suspension culture. Differentiation is induced by withdrawal of conditioned medium. The resulting embryoid bodies are plated onto semi-solid substrates. Formation of differentiated cells may be observed after around about 7 days to around about 4 weeks. Viable differentiating cells from *in vitro* cultures of stem cells are selected for by partially dissociating embryoid bodies or similar structures to provide cell aggregates. Aggregates comprising cells of interest are selected for phenotypic features using methods that substantially maintain the cell to cell contacts in the aggregate.

In an alternative embodiment, the stem cells can be reprogrammed stem cells, such as stem cells derived from somatic or differentiated cells. In such an embodiment, the de-differentiated stem cells can be for example, but not limited to, neoplastic cells, tumor cells and cancer cells or alternatively induced reprogrammed cells such as induced pluripotent stem cells or iPS cells.

### Cloning and Cell Culture

Illustrative methods for molecular genetics and genetic engineering that may be used in the technology described herein may be found, for example, in current editions of Molecular Cloning: A Laboratory Manual, (Sambrook et al., Cold Spring Harbor); Gene Transfer Vectors for Mammalian Cells (Miller & Calos eds.); and Current Protocols in Molecular Biology (F. M. Ausubel et al. eds., Wiley & Sons). Cell biology, protein chemistry, and antibody techniques can be found, for example, in Current Protocols in Protein Science (J. E. Colligan et al. eds., Wiley & Sons); Current Protocols in Cell Biology (J. S. Bonifacino et al., Wiley & Sons) and Current protocols in Immunology (J. E. Colligan et al. eds., Wiley & Sons.). Illustrative reagents, cloning vectors, and kits for genetic manipulation may be commercially obtained, for example, from BioRad, Stratagene, Invitrogen, ClonTech, and Sigma-Aldrich Co.

Suitable cell culture methods may be found, for example, in Cell culture methods are described generally in the current edition of Culture of Animal Cells: A Manual of Basic Technique (R. I. Freshney ed., Wiley & Sons); General Techniques of Cell Culture (M. A. Harrison & I. F. Rae, Cambridge Univ. Press), and Embryonic Stem Cells: Methods and Protocols (K. Turksen ed., Humana Press). Suitable tissue culture supplies and reagents are commercially available, for example, from Gibco/BRL, Nalgene-Nunc International, Sigma Chemical Co., and ICN Biomedicals.

Pluripotent stem cells can be propagated by one of ordinary skill in the art and continuously in culture, using culture conditions that promote proliferation without promoting differentiation. Exemplary serum-containing ES medium is made with 80% DMEM (such as Knock-Out DMEM, Gibco), 20% of either defined fetal bovine serum (FBS, Hyclone) or serum replacement (WO 98/30679), 1% non-essential amino acids, 1 mM L-glutamine, and 0.1 mM β-mercaptoethanol. Just before use, human bFGF is added to 4 ng/mL (WO 99/20741, Geron Corp.). Traditionally, ES cells are cultured on a layer of feeder cells, typically fibroblasts derived from embryonic or fetal tissue.

Scientists at Geron have discovered that pluripotent SCs can be maintained in an undifferentiated state even without feeder cells. The environment for feeder-free cultures includes a suitable culture substrate, particularly an extracellular matrix such as Matrigel^{®} or laminin. Typically, enzymatic digestion is halted before cells become completely dispersed (say, ~5 min with collagenase IV). Clumps of ~10 to 2,000 cells are then plated directly onto the substrate without further dispersal.

Feeder-free cultures are supported by a nutrient medium containing factors that support proliferation of the cells without differentiation. Such factors may be introduced into the medium by culturing the medium with cells secreting such factors, such as irradiated (-4,000 rad) primary mouse embryonic fibroblasts, telomerized mouse fibroblasts, or fibroblast-like cells derived from pPS cells. Medium can be conditioned by plating the feeders at a density of ~5-6×10⁴ cm⁻² in a serum free medium such as KO DMEM supplemented with 20% serum replacement and 4 ng/mL bFGF. Medium that has been conditioned for 1-2 days is supplemented with further bFGF, and used to support pluripotent SC culture for 1-2 days. Features of the feeder-free culture method are further discussed in International Patent Publication WO 01/51616; and Xu et al., Nat. Biotechnol. 19:971, 2001.

Under the microscope, ES cells appear with high nuclear/cytoplasmic ratios, prominent nucleoli, and compact colony formation with poorly discernable cell junctions. Primate ES cells express stage-specific embryonic antigens (SSEA) 3 and 4, and markers detectable using antibodies designated Tra-1-60 and Tra-1-81 (Thomson et al., Science 282:1145, 1998). Mouse ES cells can be used as a positive control for SSEA-1, and as a negative control for SSEA-4, Tra-1-60, and Tra-1-81. SSEA-4 is consistently present human embryonal carcinoma (hEC) cells. Differentiation of pluripotent SCs *in vitro* results in the loss of SSEA-4, Tra-1-60, and Tra-1-81 expression, and increased expression of SSEA-1, which is also found on undifferentiated hEG cells.

### Methods of Controlling the Differentiation of Stem Cell-Derived Cells

Aspects of the disclosure relate to generating stem cell-derived cells (e.g., progenitor cells or endocrine cells (e.g., SC-β cells, SC-α cells, non-pancreatic endocrine cells)). Generally, the at least one stem cell-derived cell or precursor thereof, e.g., polyhormonal cells produced according to the methods disclosed herein can comprise a mixture or combination of different cells, for example a mixture of cells such as endocrine progenitor cells, pancreatic progenitor cells, gut tube endoderm cells, definitive endoderm cells, and/or other pluripotent or stem cells.

The at least one stem cell-derived cell or precursor thereof can be produced according to any suitable culturing protocol to differentiate a stem cell or pluripotent cell to a desired stage of differentiation. In some embodiments, the at least one stem cell-derived cell or the precursor thereof are produced by culturing at least one pluripotent cell for a period of time and under conditions suitable for the at least one pluripotent cell to differentiate into the at least one stem cell-derived cell or the precursor thereof.

In some embodiments, the at least one stem cell-derived cell or precursor thereof is a substantially pure population of stem cell-derived cells or precursors thereof. In some embodiments, a population of stem cell-derived cells or precursors thereof comprises a mixture of pluripotent cells or differentiated cells (e.g., a mixture of stem cell-derived cells). In some embodiments, a population of stem cell-derived cells or precursors thereof are substantially free or devoid of embryonic stem cells or pluripotent cells or iPS cells.

In some embodiments, a somatic cell, e.g., fibroblast can be isolated from a subject, for example as a tissue biopsy, such as, for example, a skin biopsy, and reprogrammed into an induced pluripotent stem cell for further differentiation to produce the at least one stem cell-derived cell or precursor thereof for use in the compositions and methods described herein. In some embodiments, a somatic cell, e.g., fibroblast is maintained in culture by methods known by one of ordinary skill in the art, and in some embodiments, propagated prior to being converted into stem cell-derived cells by the methods as disclosed herein.

In some embodiments, the at least one stem cell-derived cell or precursor thereof is maintained in culture by methods known by one of ordinary skill in the art, and in some embodiments, propagated prior to being converted into stem cell-derived cells by the methods as disclosed herein.

Further, at least one stem cell-derived cell or precursor thereof, e.g., pancreatic progenitor can be from any mammalian species, with non-limiting examples including a murine, bovine, simian, porcine, equine, ovine, or human cell. For clarity and simplicity, the description of the methods herein refers to a mammalian stem cell-derived cell or precursor thereof but it should be understood that all of the methods described herein can be readily applied to other cell types of at least one stem cell-derived cell or precursor thereof. In some embodiments, the at least one stem cell-derived cell or precursor thereof is derived from a human individual.

In some embodiments stem cell-derived cells may be produced using the methods disclosed in WO 2015/002724 and WO 2014/201167, both of which are incorporated herein by reference.

In certain embodiments a method for generating stem cell-derived cells comprises contacting a cell population comprising pluripotent cells directed to differentiate into stem cell-derived cells, or cell precursors thereof, with an effective amount of agents or factors capable of directing the differentiation of such cells into stem cell-derived cells. In some aspects the various stages of the differentiation protocol include embryonic stem cells, definitive endoderm cells, foregut cells, early pancreatic bud cells, epithelial cord progenitor cells, endocrine precursor cells, α-like cells, β-like cells, and non-pancreatic endocrine cells.

A differentiation protocol may include six stages. In some aspects the first three stages of the differentiation protocol proceed linearly (e.g., hESCs differentiate to endoderm cells (stage 1), which differentiate to foregut cells (stage 2), which differentiate to pancreatic bud cells (stage 3)). In some aspects, at stage 4, the pancreatic bud cells differentiate into one or more cell types. The one or more cells types may be classified into two cell groups, epithelial cord progenitor cells and endocrine cells. In some aspects endocrine cells may comprise endocrine precursor cells, pancreatic endocrine cells (e.g., α-like cells, β-like cells, etc.) and non-pancreatic endocrine cells. In some aspects pancreatic bud cells differentiate to epithelial cord progenitor cells (stage 4). In some aspects pancreatic bud cells differentiate to endocrine precursor cells (stage 4). The endocrine precursor cells may begin to differentiate to endocrine cells at stage 4 and/or at stage 5. In some aspects the endocrine cells beginning to form in stage 4 comprise α-like cells. In some aspects the endocrine cells beginning to form in stage 5 comprise β-like cells and non-pancreatic endocrine cells. In some aspects the endocrine cells at stage 6 comprise α-like cells, β-like cells, and/or α and β-like cells.

In some embodiments the differentiation protocol may be directed to produce a higher yield of epithelial progenitor cells or a higher yield of endocrine cells. Modifications may be made to the differentiation protocol at stages 4 and/or 5 to direct the differentiation protocol towards a specific cell fate. In some aspects one or more agents may be administered to a cell during stage 4 and/or stage 5 to direct differentiation of the cell fate. For example, the differentiation protocol may be directed towards producing an increased yield of SC-α like cells. Alternatively, the differentiation protocol may be directed towards producing an increased yield of SC-β like cells.

In some embodiments the differentiation protocol may be directed by inhibiting one or more pathways at stage 4 and/or stage 5 of the protocol. In some embodiments the protocol may be directed by activating and/or inhibiting one or more pathways at stage 4 and/or stage 5. In some aspects the one or more pathways activated and/or inhibited include the notch signaling pathway, the WNT signaling pathway, the BMP signaling pathway, the hedgehog signaling pathway, the TGF-β signaling pathway, the Rap1 signaling pathway, the PI3K-Akt signaling pathway, the FoxO signaling pathway, the FGF signaling pathway, and the Hippo signaling pathway. In some aspects the pathways targeted include the WNT pathway and the BMP pathway. In some aspects the differentiation protocol may be directed by modifying the factors applied at the different stages of the protocol. In some embodiments methods of directing a differentiation protocol include applying a WNT pathway modulator and/or a BMP pathway modulator during the differentiation protocol.

In some aspects a stem cell is contacted with a WNT pathway inhibitor. A WNT pathway inhibitor may be a tankyrase inhibitor. In some aspects a WNT pathway inhibitor is selected from the group consisting of IWR1-endo, XAV939, AZ6102, JW55, MN64, TC-E5001, WIKI4, and combinations thereof. In certain aspects the WNT pathway inhibitor is IWR1-endo or XAV939. In some aspects a WNT pathway inhibitor is a WNT secretion inhibitor, e.g., IWP2. In some aspects the stem cell is a pancreatic bud cell. In some aspects the stem cell is an endocrine precursor cell. In some aspects the stem cell is contacted with the WNT pathway inhibitor at stage 4 and/or stage 5 of a differentiation protocol. In some aspects, applying a WNT pathway inhibitor to cells at stages 4 and 5 of a differentiation protocol results in increased expression of endocrine markers and downregulated expression of progenitor markers. For example, contacting the cells at stage 4 with a WNT pathway inhibitor results in increased expression of one or more of PAX6, NEUROD1, ALDH1A1, CHGB, SCG5, CHGA, TTR, NEUROG3, and ERO1LB, and downregulated expression of one or more of FN1, HOXA3, HES1, ONECUT1, SOX9, RARB, VSIG1, NKX6-1, CPA2. Contacting cells at stage 5 with a WNT pathway inhibitor may result in increased expression of one or more of CHGA, SCG5, NEUROD1, PAX6, ABCC8, MAFB, TTR, CHGB, ALDH1A1, NKX6-1, and NEUROG3, and downregulated expression of one or more of RARRES2, SHH, HOX13, ONECUT1, VSIG1, FN1, SOX2, SMO, RARB, and HES1. In addition, contacting the cells at stage 4 and/or stage 5 with a WNT pathway inhibitor results in increased expression of one or more of INS, SST, and GCG.

The contacting of a stem cell with a WNT pathway inhibitor at stage 4 of a differentiation protocol results in an increase in the number of CHGA⁺ cells and/or a decrease in the number of PDX⁺NKX6.1⁺ progenitor cells. In some aspects the contacting of a stem cell with a WNT pathway inhibitor at stage 4 increases the number of CHGA+ cells by about 5%, 10%, 15%, 20%, 25%, 30%, or 35%, on average. In certain aspects the contacting of a stem cell with a WNT pathway inhibitor at stage 4 increases the number of CHGA+ cells by about 20% on average. In some aspects the contacting of a stem cell with a WNT pathway inhibitor at stage 4 reduces the number of PDX+NKX6.1+ progenitors by more than 10%, 15%, 20%, 25%, 30%, 35%, 40%, or 45%, on average. In certain aspects the contacting of a stem cell with a WNT pathway inhibitor at stage 4 reduces the number of PDX+NKX6.1+ progenitors by more than 30% on average. In some aspects the contacting of a stem cell with a WNT pathway inhibitor at stage 4 increases the number of CHGA+ cells by about 20% on average and reduces the number of PDX+NKX6.1+ progenitors by more than 30% on average. In some aspects the contacting of a stem cell with a WNT pathway inhibitor at stage 5 increases the number of C-PEP+ cells (e.g., C-PEP+/NKX6.1+ β-like cells) by more than 25%, 30%, 35%, 40%, 45%, 50%, 55%, 60%, or 65%, on average. In certain aspects the contacting of a stem cell with a WNT pathway inhibitor at stage 5 increases the number of C-PEP+ cells by more than 50%. In some aspects the contacting of a stem cell with a WNT pathway inhibitor at stage 5 does not affect the number of GLP2+ α-like cells.

In some aspects the contacting of a stem cell with a WNT pathway inhibitor at stage 4 of a differentiation protocol results in an increased yield of stem cell-derived alpha cells. In some aspects the contacting of a stem cell with a WNT pathway inhibitor at stage 5 of a differentiation protocol results in an increased yield of stem cell-derived beta cells. In alternative aspects a stem cell is contacted with a WNT pathway activator at stage 4 and/or stage 5, resulting in a decreased yield of endocrine cells (e.g., SC-α and/or SC-β cells).

In some aspects a differentiation protocol includes contacting a stem cell with a BMP pathway activator. In some aspects a BMP pathway activator is selected from the group consisting of BMP2, BMP7, BMP9, GDF5, BMP2/6, BMP2/7, BMP4/7, and combinations thereof. In certain aspects the BMP pathway activator is BMP2/7. In some aspects the stem cell is a pancreatic bud cell. In some aspects the stem cell is contacted with the BMP pathway activator at stage 4 of a differentiation protocol. In some aspects applying a BMP pathway activator to cells at stage 4 of a differentiation protocol reduces the endocrine compartment in favor of the progenitor domain. In some aspects the addition of a BMP pathway activator at stage 4 of a differentiation protocol results in the downregulation of endocrine genes. For example, contacting the cells at stage 4 with a BMP pathway activator results in downregulated expression of one or more of PAX6, NEUROD1, ALDH1A1, CHGB, SCG5, CHGA, TTR, NEUROG3, and ERO1LB, and increased expression of one or more of FN1, HOXA3, HES1, ONECUT1, SOX9, and RARB. In addition, contacting the cells at stage 4 with a BMP pathway activator results in downregulated expression of one or more of INS, SST, and GCG.

The contacting of a stem cell with a BMP pathway activator at stage 4 of a differentiation protocol results in a decrease in the number of CHGA⁺ endocrine cells and/or an increase in the number of PDX⁺NKX6.1⁺ progenitor cells. In some aspects the contacting of a stem cell with a BMP pathway activator at stage 4 decreases the number of CHGA+ cells by nearly 1.5 fold, 2 fold, 2.5 fold, 3 fold, or 3.5 fold, on average. In certain aspects the contacting of a stem cell with a BMP pathway activator at stage 4 decreases the number of CHGA+ cells by nearly 2 fold on average. In some aspects the contacting of a stem cell with a BMP pathway activator at stage 4 increases the number of PDX+NKX6.1+ progenitors by more than 1.5 fold, 2 fold, 2.5 fold, 3 fold, or 3.5 fold, on average. In certain aspects the contacting of a stem cell with a BMP pathway activator at stage 4 increases the number of PDX+NKX6.1+ progenitors by more than 2 fold. In some aspects the contacting of a stem cell with a BMP pathway activator at stage 4 decreases the number of CHGA+ cells by nearly two fold on average and increases the number of PDX+NKX6.1+ progenitors by more than two fold on average.

In some aspects the contacting of a stem cell with a BMP pathway activator at stage 4 of a differentiation protocol results in an increased yield of epithelial progenitor cells. In some aspects the contacting of a stem cell with a BMP pathway activator at stage 4 of a differentiation protocol results in a decreased yield of endocrine cells. In alternative aspects a stem cell is contacted with a BMP pathway inhibitor at stage 4 of a differentiation protocol, resulting in a decreased yield of epithelial progenitor cells.

In some embodiments a differentiation protocol may be directed by removing a γ-secretase inhibitor from stage 5 of the differentiation protocol described herein. In some aspects the γ-secretase inhibitor is XXI. The removal of the γ-secretase inhibitor from the differentiation protocol reduces the proportion of endocrine cells that co-express NKX6.1 (e.g., the endocrine cells of stage 5 more closely resemble the endocrine cells of stage 4 (e.g., α-like cells)). In some aspects the removal of the γ-secretase inhibitor from the differentiation protocol at stage 5 reduces the number of CPEP+NKX6.1+ β-like cells. In some aspects the removal of the γ-secretase inhibitor from the differentiation protocol at stage 5 results in a population of endocrine cells that includes a greater yield of α-like cells.

In some aspects any combination of pathway activators and/or inhibitors are used to direct a differentiation protocol to a desired fate. For example, an increased yield of SC-β like cells may be obtained by combining WNT pathway inhibition at stage 5 of the differentiation protocol with BMP activation at stage 4 of the differentiation protocol.

In some aspects of the disclosure a population of epithelial progenitor cells is provided, where the population comprises an increased yield of progenitor cells by directing the differentiation of stem cells to form the epithelial progenitor cells.

In other aspects of the disclosure a population of stem cell derived endocrine cells comprising stem cell-derived α cells and stem cell-derived β cells is provided, where the ratio of SC-α cells and SC-β cells in the population is controlled by directing the differentiation of stem cells to form the endocrine cells. In some aspects, the disclosure provides a cell line comprising endocrine cells described herein. In some aspects, the disclosure provides an artificial islet or pancreas (e.g., an SC-islet) comprising the endocrine cells described herein (e.g. SC-α cells and/or SC-β cells). In some embodiments the SC-islet further comprises SC-δ cells.

An artificial pancreas is a device that encapsulates and nurtures islets of Langerhans to replace the islets and α, β and/or δ cells destroyed by type 1 diabetes. An artificial pancreas may contain a million islets or more, and may be implanted in the peritoneal cavity or under the skin where it can respond to changing blood glucose levels by releasing hormones, such as glucagon, somatostatin, and/or insulin. An artificial pancreas may be made using living and non-living components (e.g., to shield the islets from the diabetic subject's body and its destructive immune mechanism while permitting the islets to thrive).

The present invention contemplates using stem cell-derived endocrine cells (e.g., SC-α cells, SC-β cells, and/or SC-δ cells), in any artificial pancreas. In some aspects, the artificial pancreas comprises microencapsulated or coated islets comprising stem cell derived endocrine cells generated according to the methods described herein. In some aspects the ratio of specific endocrine cells utilized in the artificial pancreas is controlled using the methods described herein. In some aspects, the artificial pancreas comprises a macroencapsulation device into which islet cells comprising stem cell-derived endocrine cells generated according to the methods herein are grouped together and encapsulated. In some aspects, the macroencapsulation device comprises a PVA hydrogel sheet for an artificial pancreas of the present invention. In some aspects, the artificial islet comprises stem cell-derived endocrine cells generated according to the methods herein in the form of an islet sheet. The islet sheet comprises a layer of artificial human islets comprising the stem cell-derived endocrine cells macroencapsulated within a membrane (e.g., of ultra-pure alginate). The sheet membrane is reinforced with mesh and may be coated on the surface to prevent or minimize contact between the cells encapsulated inside and the transplantation recipient's host immune response. Oxygen, glucose, and other nutrients readily diffuse into the sheet through the membrane nurturing the islets, and hormones, such as insulin, readily diffuse out. Additional examples of membranes designed for macroencapsulation/implantation of an artificial islet or pancreas are known to those of skill in the art.

In some embodiments, the cells described herein, e.g. a population of stem cell-derived endocrine cells are transplantable, e.g., a population of stem cell-derived endocrine cells can be administered to a subject. In some embodiments, the subject who is administered a population of stem cell-derived endocrine cells is the same subject from whom a pluripotent stem cell used to differentiate into a stem cell-derived endocrine cell was obtained (e.g. for autologous cell therapy). In some embodiments, the subject is a different subject. In some embodiments, a subject is suffering from diabetes such as type I diabetes, or is a normal subject. For example, the cells for transplantation (e.g. a composition comprising a population of stem cell-derived endocrine cells) can be a form suitable for transplantation, e.g., organ transplantation.

The method can further include administering the cells to a subject in need thereof, e.g., a mammalian subject, e.g., a human subject. The source of the cells can be a mammal, preferably a human. The source or recipient of the cells can also be a non-human subject, e.g., an animal model. The term "mammal" includes organisms, which include mice, rats, cows, sheep, pigs, rabbits, goats, horses, monkeys, dogs, cats, and preferably humans. Likewise, transplantable cells can be obtained from any of these organisms, including a non-human transgenic organism. In one embodiment, the transplantable cells are genetically engineered, e.g., the cells include an exogenous gene or have been genetically engineered to inactivate or alter an endogenous gene.

A composition comprising a population of stem cell-derived endocrine cells can be administered to a subject using an implantable device. Implantable devices and related technology are known in the art and are useful as delivery systems where a continuous, or timed-release delivery of compounds or compositions delineated herein is desired. Additionally, the implantable device delivery system is useful for targeting specific points of compound or composition delivery (e.g., localized sites, organs). Negrin et al., Biomaterials, 22(6):563 (2001). Timed-release technology involving alternate delivery methods can also be used in this invention. For example, timed-release formulations based on polymer technologies, sustained-release techniques and encapsulation techniques (e.g., polymeric, liposomal) can also be used for delivery of the compounds and compositions delineated herein.

For administration to a subject, a cell population produced by the methods as disclosed herein, e.g. a population of stem cell-derived endocrine cells can be administered to a subject, for example in pharmaceutically acceptable compositions. These pharmaceutically acceptable compositions comprise a therapeutically-effective amount of a population of stem cell-derived endocrine cells as described above, formulated together with one or more pharmaceutically acceptable carriers (additives) and/or diluents.

As described in detail below, the pharmaceutical compositions of the present invention can be specially formulated for administration in solid or liquid form, including those adapted for the following: (1) oral administration, for example, drenches (aqueous or non-aqueous solutions or suspensions), lozenges, dragees, capsules, pills, tablets (e.g., those targeted for buccal, sublingual, and systemic absorption), boluses, powders, granules, pastes for application to the tongue; (2) parenteral administration, for example, by subcutaneous, intramuscular, intravenous or epidural injection as, for example, a sterile solution or suspension, or sustained-release formulation; (3) topical application, for example, as a cream, ointment, or a controlled-release patch or spray applied to the skin; (4) intravaginally or intrarectally, for example, as a pessary, cream or foam; (5) sublingually; (6) ocularly; (7) transdermally; (8) transmucosally; or (9) nasally. Additionally, compounds can be implanted into a patient or injected using a drug delivery system. See, for example, Urquhart, et al., Ann. Rev. Pharmacol. Toxicol. 24: 199-236 (1984); Lewis, ed. "Controlled Release of Pesticides and Pharmaceuticals" (Plenum Press, New York, 1981); U.S. Pat. No. 3,773,919; and U.S. Pat. No. 35 3,270,960.

As used here, the term "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used here, the term "pharmaceutically-acceptable carrier" means a pharmaceutically-acceptable material, composition or vehicle, such as a liquid or solid filler, diluent, excipient, manufacturing aid (e.g., lubricant, talc magnesium, calcium or zinc stearate, or steric acid), or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, methylcellulose, ethyl cellulose, microcrystalline cellulose and cellulose acetate; (4) powdered tragacanth; (5) malt; (6) gelatin; (7) lubricating agents, such as magnesium stearate, sodium lauryl sulfate and talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol (PEG); (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; (22) bulking agents, such as polypeptides and amino acids (23) serum component, such as serum albumin, HDL and LDL; (22) C₂-C₁₂ alcohols, such as ethanol; and (23) other non-toxic compatible substances employed in pharmaceutical formulations. Wetting agents, coloring agents, release agents, coating agents, sweetening agents, flavoring agents, perfuming agents, preservative and antioxidants can also be present in the formulation. The terms such as "excipient", "carrier", "pharmaceutically acceptable carrier" or the like are used interchangeably herein.

The phrase "therapeutically-effective amount" as used herein in respect to a population of cells means that amount of relevant cells in a population of cells, e.g., stem cell-derived endocrine cells (e.g., SC-α cells and/or SC-β cells), or composition comprising stem cell-derived endocrine cells of the present invention which is effective for producing some desired therapeutic effect in at least a sub-population of cells in an animal at a reasonable benefit/risk ratio applicable to any medical treatment. For example, an amount of a population of stem cell-derived endocrine cells administered to a subject that is sufficient to produce a statistically significant, measurable change in at least one symptom of Type 1, Type 1.5 or Type 2 diabetes, such as glycosylated hemoglobin level, fasting blood glucose level, hypoinsulinemia, etc. Determination of a therapeutically effective amount is well within the capability of those skilled in the art. Generally, a therapeutically effective amount can vary with the subject's history, age, condition, sex, as well as the severity and type of the medical condition in the subject, and administration of other pharmaceutically active agents.

As used herein, the term "administer" refers to the placement of a composition into a subject by a method or route which results in at least partial localization of the composition at a desired site such that desired effect is produced. A compound or composition described herein can be administered by any appropriate route known in the art including, but not limited to, oral or parenteral routes, including intravenous, intramuscular, subcutaneous, transdermal, airway (aerosol), pulmonary, nasal, rectal, and topical (including buccal and sublingual) administration.

Exemplary modes of administration include, but are not limited to, injection, infusion, instillation, inhalation, or ingestion. "Injection" includes, without limitation, intravenous, intramuscular, intraarterial, intrathecal, intraventricular, intracapsular, intraorbital, intracardiac, intradermal, intraperitoneal, transtracheal, subcutaneous, subcuticular, intraarticular, sub capsular, subarachnoid, intraspinal, intracerebro spinal, and intrasternal injection and infusion. In preferred embodiments, the compositions are administered by intravenous infusion or injection.

By "treatment", "prevention" or "amelioration" of a disease or disorder is meant delaying or preventing the onset of such a disease or disorder, reversing, alleviating, ameliorating, inhibiting, slowing down or stopping the progression, aggravation or deterioration the progression or severity of a condition associated with such a disease or disorder. In one embodiment, the symptoms of a disease or disorder are alleviated by at least 5%, at least 10%, at least 20%, at least 30%, at least 40%, or at least 50%.

Treatment of diabetes is determined by standard medical methods. A goal of diabetes treatment is to bring sugar levels down to as close to normal as is safely possible. Commonly set goals are 80-120 milligrams per deciliter (mg/dl) before meals and 100-140 mg/dl at bedtime. A particular physician may set different targets for the patient, depending on other factors, such as how often the patient has low blood sugar reactions. Useful medical tests include tests on the patient's blood and urine to determine blood sugar level, tests for glycosylated hemoglobin level (HbA1c; a measure of average blood glucose levels over the past 2-3 months, normal range being 4-6%), tests for cholesterol and fat levels, and tests for urine protein level. Such tests are standard tests known to those of skill in the art (see, for example, American Diabetes Association, 1998). A successful treatment program can also be determined by having fewer patients in the program with complications relating to diabetes, such as diseases of the eye, kidney disease, or nerve disease.

Delaying the onset of diabetes in a subject refers to delay of onset of at least one symptom of diabetes, e.g., hyperglycemia, hypoinsulinemia, diabetic retinopathy, diabetic nephropathy, blindness, memory loss, renal failure, cardiovascular disease (including coronary artery disease, peripheral artery disease, cerebrovascular disease, atherosclerosis, and hypertension), neuropathy, autonomic dysfunction, hyperglycemic hyperosmolar coma, or combinations thereof, for at least 1 week, at least 2 weeks, at least 1 month, at least 2 months, at least 6 months, at least 1 year, at least 2 years, at least 5 years, at least 10 years, at least 20 years, at least 30 years, at least 40 years or more, and can include the entire lifespan of the subject.

In certain embodiments, the subject is a mammal, e.g., a primate, e.g., a human. The terms, "patient" and "subject" are used interchangeably herein. Preferably, the subject is a mammal. The mammal can be a human, non-human primate, mouse, rat, dog, cat, horse, or cow, but are not limited to these examples. Mammals other than humans can be advantageously used as subjects that represent animal models of Type 1 diabetes, Type 2 Diabetes Mellitus, or pre-diabetic conditions. In addition, the methods described herein can be used to treat domesticated animals and/or pets. A subject can be male or female. A subject can be one who has been previously diagnosed with or identified as suffering from or having diabetes (e.g., Type 1 or Type 2), one or more complications related to diabetes, or a pre-diabetic condition, and optionally, but need not have already undergone treatment for diabetes, the one or more complications related to diabetes, or the pre-diabetic condition. A subject can also be one who is not suffering from diabetes or a pre-diabetic condition. A subject can also be one who has been diagnosed with or identified as suffering from diabetes, one or more complications related to diabetes, or a pre-diabetic condition, but who show improvements in known diabetes risk factors as a result of receiving one or more treatments for diabetes, one or more complications related to diabetes, or the pre-diabetic condition. Alternatively, a subject can also be one who has not been previously diagnosed as having diabetes, one or more complications related to diabetes, or a pre-diabetic condition. For example, a subject can be one who exhibits one or more risk factors for diabetes, complications related to diabetes, or a pre-diabetic condition, or a subject who does not exhibit diabetes risk factors, or a subject who is asymptomatic for diabetes, one or more diabetes-related complications, or a pre-diabetic condition. A subject can also be one who is suffering from or at risk of developing diabetes or a pre-diabetic condition. A subject can also be one who has been diagnosed with or identified as having one or more complications related to diabetes or a pre-diabetic condition as defined herein, or alternatively, a subject can be one who has not been previously diagnosed with or identified as having one or more complications related to diabetes or a pre-diabetic condition.

As used herein, the phrase "subject in need of stem cell-derived endocrine cells" refers to a subject who is diagnosed with or identified as suffering from, having or at risk for developing diabetes (e.g., Type 1, Type 1.5 or Type 2), one or more complications related to diabetes, or a pre-diabetic condition.

A subject in need of a population of stem cell-derived endocrine cells can be identified using any method used for diagnosis of diabetes. For example, Type 1 diabetes can be diagnosed using a glycosylated hemoglobin (A1C) test, a random blood glucose test and/or a fasting blood glucose test. Parameters for diagnosis of diabetes are known in the art and available to skilled artisan without much effort.

In some embodiments, the methods of the invention further comprise selecting a subject identified as being in need of additional stem cell-derived endocrine cells (e.g., SC-α cells and/or SC-β cells). A subject in need of a population of stem cell-derived endocrine cells can be selected based on the symptoms presented, such as symptoms of type 1, type 1.5 or type 2 diabetes. Exemplary symptoms of diabetes include, but are not limited to, excessive thirst (polydipsia), frequent urination (polyuria), extreme hunger (polyphagia), extreme fatigue, weight loss, hyperglycemia, low levels of insulin, high blood sugar (e.g., sugar levels over 250 mg, over 300 mg), presence of ketones present in urine, fatigue, dry and/or itchy skin, blurred vision, slow healing cuts or sores, more infections than usual, numbness and tingling in feet, diabetic retinopathy, diabetic nephropathy, blindness, memory loss, renal failure, cardiovascular disease (including coronary artery disease, peripheral artery disease, cerebrovascular disease, atherosclerosis, and hypertension), neuropathy, autonomic dysfunction, hyperglycemic hyperosmolar coma, and combinations thereof.

In some embodiments, a composition comprising a population of stem cell-derived endocrine cells for administration to a subject can further comprise a pharmaceutically active agent, such as those agents known in the art for treatment of diabetes and or for having anti-hyperglycemic activities, for example, inhibitors of dipeptidyl peptidase 4 (DPP-4) (e.g., Alogliptin, Linagliptin, Saxagliptin, Sitagliptin, Vildagliptin, and Berberine), biguanides (e.g., Metformin, Buformin and Phenformin), peroxisome proliferator-activated receptor (PPAR) modulators such as thiazolidinediones (TZDs) (e.g., Pioglitazone, Rivoglitazone, Rosiglitazone and Troglitazone), dual PPAR agonists (e.g., Aleglitazar, Muraglitazar and Tesaglitazar), sulfonylureas (e.g., Acetohexamide, Carbutamide, Chlorpropamide, Gliclazide, Tolbutamide, Tolazamide, Glibenclamide (Glyburide), Glipizide, Gliquidone, Glyclopyramide, and Glimepiride), meglitinides ("glinides") (e.g., Nateglinide, Repaglinide and Mitiglinide), glucagon-like peptide-1 (GLP-1) and analogs (e.g., Exendin-4, Exenatide, Liraglutide, Albiglutide), insulin and insulin analogs (e.g., Insulin lispro, Insulin aspart, Insluin glulisine, Insulin glargine, Insulin detemir, Exubera and NPH insulin), alpha-glucosidase inhibitors (e.g., Acarbose, Miglitol and Voglibose), amylin analogs (e.g. Pramlintide), Sodium-dependent glucose cotransporter T2 (SGLT T2) inhibitors (e.g., Dapgliflozin, Remogliflozin and Sergliflozin) and others (e.g. Benfluorex and Tolrestat).

A composition comprising stem cell-derived endocrine cells can be administrated to the subject in the same time, of different times as the administration of a pharmaceutically active agent or composition comprising the same. When administrated at different times, the compositions comprising a population of stem cell-derived endocrine cells and/or pharmaceutically active agent for administration to a subject can be administered within 5 minutes, 10 minutes, 20 minutes, 60 minutes, 2 hours, 3 hours, 4, hours, 8 hours, 12 hours, 24 hours of administration of the other. When a composition comprising a population of stem cell-derived endocrine cells and a composition comprising a pharmaceutically active agent are administered in different pharmaceutical compositions, routes of administration can be different. In some embodiments, a subject is administered a composition comprising stem cell-derived endocrine cells. In other embodiments, a subject is administered a composition comprising a pharmaceutically active agent. In another embodiment, a subject is administered a compositions comprising a population of stem cell-derived endocrine cells mixed with a pharmaceutically active agent. In another embodiment, a subject is administered a composition comprising a population of stem cell-derived endocrine cells and a composition comprising a pharmaceutically active agent, where administration is substantially at the same time, or subsequent to each other.

Toxicity and therapeutic efficacy of administration of a compositions comprising a population of stem cell-derived endocrine cells can be determined by standard pharmaceutical procedures in cell cultures or experimental animals, e.g., for determining the LD50 (the dose lethal to 50% of the population) and the ED50 (the dose therapeutically effective in 50% of the population). Compositions comprising a population of stem cell-derived endocrine cells that exhibit large therapeutic indices are preferred.

The amount of a composition comprising a population of stem cell-derived endocrine cells can be tested using several well-established animal models.

The non-obese diabetic (NOD) mouse carries a genetic defect that results in insulitis showing at several weeks of age (Yoshida et al., Rev. Immunogenet. 2:140, 2000). 60-90% of the females develop overt diabetes by 20-30 weeks. The immune-related pathology appears to be similar to that in human Type I diabetes. Other models of Type I diabetes are mice with transgene and knockout mutations (Wong et al., Immunol. Rev. 169:93, 1999). A rat model for spontaneous Type I diabetes was recently reported by Lenzen et al. (Diabetologia 44:1189, 2001). Hyperglycemia can also be induced in mice (>500 mg glucose/dL) by way of a single intraperitoneal injection of streptozotocin (Soria et al., Diabetes 49:157, 2000), or by sequential low doses of streptozotocin (Ito et al., Environ. Toxicol. Pharmacol. 9:71, 2001). To test the efficacy of implanted islet cells, the mice are monitored for return of glucose to normal levels (<200 mg/dL).

Larger animals provide a good model for following the sequelae of chronic hyperglycemia. Dogs can be rendered insulin-dependent by removing the pancreas (J. Endocrinol. 158:49, 2001), or by feeding galactose (Kador et al., Arch. Opthalmol. 113:352, 1995). There is also an inherited model for Type I diabetes in keeshond dogs (Am. J. Pathol. 105:194, 1981). Early work with a dog model (Banting et al., Can. Med. Assoc. J. 22:141, 1922) resulted in a couple of Canadians making a long ocean journey to Stockholm in February of 1925.

In some embodiments, data obtained from the cell culture assays and in animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized.

The therapeutically effective dose of a composition comprising a population of stem cell-derived endocrine cells can also be estimated initially from cell culture assays. A dose may be formulated in animal models *in vivo* to achieve a secretion of glucagon at a concentration which is appropriate in response to circulating glucose in the plasma. Alternatively, the effects of any particular dosage can be monitored by a suitable bioassay.

With respect to duration and frequency of treatment, it is typical for skilled clinicians to monitor subjects in order to determine when the treatment is providing therapeutic benefit, and to determine whether to increase or decrease dosage, increase or decrease administration frequency, discontinue treatment, resume treatment or make other alteration to treatment regimen. The dosing schedule can vary from once a week to daily depending on a number of clinical factors, such as the subject's sensitivity to the stem cell-derived endocrine cells. In addition, the ratio of SC-α cells and/or SC-β cells within the stem-cell derived endocrine cells may be adjusted. The desired dose can be administered at one time or divided into subdoses, e.g., 2-4 subdoses and administered over a period of time, e.g., at appropriate intervals through the day or other appropriate schedule. Such sub-doses can be administered as unit dosage forms. In some embodiments, administration is chronic, e.g., one or more doses daily over a period of weeks or months. Examples of dosing schedules are administration daily, twice daily, three times daily or four or more times daily over a period of 1 week, 2 weeks, 3 weeks, 4 weeks, 1 month, 2 months, 3 months, 4 months, 5 months, or 6 months or more.

In another aspect of the invention, the methods provide use of an isolated population of stem cell-derived endocrine cells as disclosed herein. In one embodiment of the invention, an isolated population of stem cell-derived endocrine cells as disclosed herein may be used for the production of a pharmaceutical composition, for the use in transplantation into subjects in need of treatment, e.g. a subject that has, or is at risk of developing diabetes, for example but not limited to subjects with congenital and acquired diabetes. In one embodiment, an isolated population of stem cell-derived endocrine cells may be genetically modified. In another aspect, the subject may have or be at risk of diabetes and/or metabolic disorder. In some embodiments, an isolated population of stem cell-derived endocrine cells as disclosed herein may be autologous and/or allogeneic. In some embodiments, the subject is a mammal, and in other embodiments the mammal is a human.

The use of an isolated population of stem cell-derived endocrine cells as disclosed herein provides advantages over existing methods because the population of stem cell-derived endocrine cells can be differentiated from pancreatic bud cells or precursors thereof derived from stem cells, e.g. iPS cells obtained or harvested from the subject administered an isolated population of stem cell-derived endocrine cells. The isolated population of stem cell-derived endocrine cells may also be directed during the differentiation process to comprise predominantly SC-α cells or predominantly SC-β cells. This is highly advantageous as it provides a renewable source of stem cell-derived endocrine cells which can be differentiated from stem cells to endocrine cells by methods commonly known by one of ordinary skill in the art, and then further differentiated by the methods described herein to pancreatic α-like cells or pancreatic β-like cells or cells with pancreatic α or β cell characteristics, for transplantation into a subject, in particular a substantially pure population of mature pancreatic α-like cells or mature pancreatic β-like cells that do not have the risks and limitations of cells derived from other systems.

One embodiment of the invention relates to a method of treating diabetes or a metabolic disorder in a subject comprising administering an effective amount of a composition comprising a population of stem cell-derived endocrine cells as disclosed herein to a subject with diabetes and/or a metabolic disorder. In a further embodiment, the invention provides a method for treating diabetes, comprising administering a composition comprising a population of stem cell-derived endocrine cells as disclosed herein to a subject that has, or has an increased risk of developing diabetes.

In one embodiment of the above methods, the subject is a human and a population of stem cell-derived endocrine cells as disclosed herein are human cells. In some embodiments, the invention contemplates that a population of stem cell-derived endocrine cells as disclosed herein are administered directly to the pancreas of a subject, or is administered systemically. In some embodiments, a population of stem cell-derived endocrine cells as disclosed herein can be administered to any suitable location in the subject, for example in a capsule in the blood vessel or the liver or any suitable site where administered the population of stem cell-derived endocrine cells can secrete insulin and/or glucagon in response to increased glucose levels in the subject.

The present invention is also directed to a method of treating a subject with diabetes or a metabolic disorder which occurs as a consequence of genetic defect, physical injury, environmental insult or conditioning, bad health, obesity and other diabetes risk factors commonly known by a person of ordinary skill in the art. Efficacy of treatment of a subject administered a composition comprising a population of stem cell-derived endocrine cells can be monitored by clinically accepted criteria and tests, which include for example, (i) Glycated hemoglobin (A1C) test, which indicates a subjects average blood sugar level for the past two to three months, by measuring the percentage of blood sugar attached to hemoglobin, the oxygen-carrying protein in red blood cells. The higher your blood sugar levels, the more hemoglobin has sugar attached. An A1C level of 6.5 percent or higher on two separate tests indicates the subject has diabetes. A test value of 6-6.5% suggest the subject has prediabetes. (ii) Random blood sugar test. A blood sample will be taken from the subject at a random time, and a random blood sugar level of 200 milligrams per deciliter (mg/dL)-11.1 millimoles per liter (mmol/L), or higher indicated the subject has diabetes, (iii) Fasting blood sugar test. A blood sample is taken from the subject after an overnight fast. A fasting blood sugar level between 70 and 99 mg/dL (3.9 and 5.5 mmol/L) is normal. If the subjects fasting blood sugar levels is 126 mg/dL (7 mmol/L) or higher on two separate tests, the subject has diabetes. A blood sugar level from 100 to 125 mg/dL (5.6 to 6.9 mmol/L) indicates the subject has prediabetes. (iv) Oral glucose tolerance test. A blood sample will be taken after the subject has fasted for at least eight hours or overnight and then ingested a sugary solution, and the blood sugar level will be measured two hours later. A blood sugar level less than 140 mg/dL (7.8 mmol/L) is normal. A blood sugar level from 140 to 199 mg/dL (7.8 to 11 mmol/L) is considered prediabetes. This is sometimes referred to as impaired glucose tolerance (IGT). A blood sugar level of 200 mg/dL (11.1 mmol/L) or higher may indicate diabetes.

In some embodiments, the effects of administration of a population of stem cell-derived endocrine cells as disclosed herein to a subject in need thereof is associated with improved exercise tolerance or other quality of life measures, and decreased mortality. The effects of cellular therapy with a population of stem cell-derived endocrine cells can be evident over the course of days to weeks after the procedure. However, beneficial effects may be observed as early as several hours after the procedure, and may persist for several years. In some embodiments, the effects of cellular therapy with a population of stem cell-derived endocrine cells occurs within two weeks after the procedure.

In some embodiments, a population of stem cell-derived endocrine cells as disclosed herein may be used for tissue reconstitution or regeneration in a human patient or other subject in need of such treatment. In some embodiments compositions of populations of stem cell-derived endocrine cells can be administered in a manner that permits them to graft or migrate to the intended tissue site and reconstitute or regenerate the functionally deficient area. Special devices are available that are adapted for administering cells capable of reconstituting a population of endocrine cells (e.g., α and/or β cells) in the pancreas or at an alternative desired location. Accordingly, the stem cell-derived endocrine cells may be administered to a recipient subject's pancreas by injection, or administered by intramuscular injection.

In some embodiments, compositions comprising a population of stem cell-derived endocrine cells as disclosed herein have a variety of uses in clinical therapy, research, development, and commercial purposes.

### EXAMPLES

### Example 1: Wnt signaling separates the progenitor and endocrine compartments during pancreas development

*In vitro* differentiation of pluripotent cells into β cells is a promising alternative to cadaveric-islet transplantation as a cure for type 1diabetes (T1D). During the directed differentiation of human embryonic stem cells (hESCS) by exogenous factors, numerous genes that affect the differentiation process are turned on and off autonomously. Manipulating these reactions could increase the efficiency of differentiation and provide a more complete control over the final composition of cell populations. To uncover *in-vitro* autonomous responses, single-cell RNA sequencing was performed on hESCs as they differentiate in spherical clusters. It was observed that endocrine cells and their progenitors exist beside one another in separate compartments that activate distinct genetic pathways. WNT pathway inhibition in the endocrine domain of the differentiating clusters revealed a necessary role for the WNT inhibitor APC during islet formation *in-vivo.* Accordingly, WNT inhibition *in vitro* causes an increase in the proportion of differentiated endocrine cells.

### Single cell RNA-sequencing of differentiating β cells

hESCs were differentiated into stem-cell-derived β cells (SC-β) as clusters in suspension using a six-stage protocol³⁷ (FIG. 1A). scRNA-seq was performed on undifferentiated cells and on ten consecutive time points, representing the end of each of the differentiation stages and select intermediate points (FIGS. 5A-5B). To analyze the relationships between the cells, SIMLR analysis (single-cell interpretation via multi kernel learning) was combined with topic modeling (TM). SIMLR is a method that groups cells based on cell-to-cell similarity and then displays them in lower dimensional space⁶⁰ (FIG. 1B). TM is a probabilistic unsupervised learning algorithm that, in the context of gene expression analysis, identifies groups of genes that are frequently expressed together in the same cell, and gathers them into "expression profiles" (EPs)^{4, 13, 55}. For each EP, every gene receives a "relevance value" which describes the gene's weight in the identification of this particular EP. While establishing which genes comprise an EP, the TM algorithm simultaneously quantifies how active each EP is within a particular cell via a "usage value". Cells that tend to use genes from the same EPs (have high usage values for similar EPs) can be grouped together. Whereas customarily used clustering methods such as hierarchical clustering assume that the relationships between genes are strict (e.g. Euclidean distance, correlation), TM analyzes these relationships as probability distributions. This allows the clustering of cells and genes in a flexible arrangement. Rather than forcing each gene to one expression module, with TM a gene can be relevant to several EPs, reflecting its possible expression in the context of different biological processes. Similarly, since each cell uses several biological processes, a single cell may use several EPs, to varying extents. Furthermore, since conventional clustering methods allow a gene to belong only to a single expression module, many genes can be lost to artificial modules caused by technical noise. However, the inherent flexibility of TM allows these genes to appear in biologically meaningful EPs as well. Altogether, the advantages of TM analysis over conventional clustering methods are especially relevant for discovering hidden structures in highly complex datasets, including scRNA-seq of heterogeneous populations.

TM identified 36 EPs in the data (FIG. 6). Of these, 8 EPs contain markers related to cell-type identity and, combined, are used by all differentiating cells (excluding undifferentiated hESCs). The identity of each cell could now be determined according to the EP it uses most (FIGS. 1C-1D, FIG. 5C and FIG. 5F). Cells collected in the first three stages correspond with the first three cell types, whereas cells collected at the 4th stage and beyond are heterogeneous and can be divided into five cell types (FIG. 1E). An interactive data base that combines SIMLR with topic modeling and describes the major cell populations and their relative similarity to each other can be found at ifx.rc.fas.harvard.edu/invitrobetacells/.

### Early in vitro differentiation is linear and uniform, whereas late differentiation gives rise to heterogeneous populations

At the end of the first stage, marked by SOX17 expression³⁷, all cells analyzed primarily use an EP comprised of other definitive endoderm genes including CER1 and LEFTY2, whereas by the end of the second stage they primarily use an EP corresponding to foregut endoderm (HHEX, TTR) (FIGS. 1D-1E, FIG. 5C, FIG. 5F, and Table 1)^{5, 56}. Almost all cells analyzed at the end of the third stage, when PDX1 is activated³⁷, use an EP that contains additional pancreatic-bud markers such as GATA4, ONECUT1 and ONECUT2 (FIGS. 1D-1E, FIG. 5F, and Table 1)^{11,19}. The absence of NKX6.1 and expression of the foregut markers HOXA1, SHISA2 and HHEX, suggest that these cells represent an early stage in pancreatic bud development (FIG. 1D, Table 1).^{11, 14, 20, 46}. Unlike the first two EPs, the early pancreatic-bud EP is also used by cells from later stages, albeit with low usage values and not as the primary EP (FIG. 1E and FIGS. 7A-7B). This suggests that many of the genes expressed at this point remain activated in later stages of development.

From stage 4 onward, the clusters contain heterogeneous populations that can be divided into two major cell groups (FIG. 1E). The cells in one group express genes from an EP containing epithelial-cord markers, including SOX9 and ONECUT1 (HNF6), together with cell-cycle related genes such as MCM3 and CCND2 (FIGS. 1E-1G, Table 1). A second group expresses genes from several EPs containing endocrine markers including CHGA, ISL1 and NEUROD1 (FIGS. 1D-1E, FIG. 1H, Table 1). The latter also express lower levels of genes associated with cell proliferation (FIGS. 1G-1H). It was previously shown that treatment with Activin A and the ROCK inhibitor Y-27632 during stage 4 induces the appearance of buds on the clusters' surface⁵². Immunostaining shows that NEUROD1⁺ non-proliferating endocrine cells reside primarily in peninsula-like buds that formed in stage 4, whereas SOX9⁺ proliferating epithelial-progenitors are found in the cluster core (FIGS. 1I-1J). Altogether, it is concluded that the uniform population that formed in the first three stages of the protocol differentiates and splits into epithelial-cord-like progenitors and endocrine cells. These two cell types are physically separated within differentiating clusters.

### The endocrine population includes 4 distinct cell types

The endocrine EPs demarcate four major endocrine cell types (FIGS. 1C-1E). One group represents endocrine precursors and includes cells that express the endocrine initiating gene NEUROG3 (FIG. 1D), with some cells co-expressing both progenitor markers (SOX11) and early-onset endocrine genes (RUNX1T1)³⁶ (FIG. 5F). The hormones INS and GCG are expressed at low levels, and some cells express GHRL or SST. These endocrine precursors can be further classified into an early sub-group, collected mostly in the middle of stage 4, and a late sub-group, collected at the end of stages 4 and 5 (EP 28 and 22, respectively, in FIG. 6 and Table 1).

The remaining endocrine cells form three types of hormonal cells. The first express α-cell markers such as GCG, ETV1 and ARX, together with the non-α marker INS, and occasionally SST and PPY (FIG. 1D, FIG. 5F, Table 1). Flow cytometry analysis indicates that as cells progress into stage 6 these polyhormonal cells resolve into GCG⁺INS⁻ α cells (FIGS. 5D-5E). A second group are β cells, identified by high expression of INS, NKX6.1, PDX1 and LMO1, and scarcity of GCG transcripts (FIG. 1D, FIG. 5F, Table 1). Finally, a group of CHGA⁺ endocrine cells express low levels of the pan-islet marker ISL1, serotoninergic genes such as TPH1, and non-islet genes^{50, 58} such as ADH6 and LMX1A (FIGS. 1D-1E, FIG. 5F, Table 1). These non-pancreatic endocrine cells may represent byproducts of the *in vitro* differentiation process that resemble enteroendocrine populations^{16, 59}.

### Two waves of endocrine differentiation

The presence of epithelial-progenitors side-by-side with endocrine cells observed from stage 4 onwards is reminiscent of normal embryonic pancreas formation, wherein new endocrine cells emerge from epithelial-cord progenitors in a continuous, asynchronous flux^{22, 29}. To see if such a flux occurs *in vitro,* the dynamics of endocrine differentiation during stages 4 and 5 was investigated.

Flow cytometry reveals a wave of newly formed NEUROG3⁺ endocrine precursors during the first days of stage 4. A few precursors appear at the last day of stage 3, and their numbers peak to about 10% of the entire population during the first day of stage 4 (FIG. 2A). In the following days fewer NEUROG3⁺ cells appear, and as they differentiate NEUROG3 is gradually replaced by the pan-endocrine gene CHGA. Less than 2% of cells are NEUROG3⁺CHGA⁻ by the end of stage 4 (FIG. 2A). During stage 5 a second wave of endocrine precursor formation was observed. Here too, the wave peaks on the first day of the stage, when ~20% of the cells are NEUROG3⁺. Similar to stage 4, the initial rise is followed by a daily decline in the number of precursors as NEUROG3⁺ is replaced with CHGA, until less than 3% of the cells are NEUROG3⁺ by the last day of stage 5 (FIG. 2A).

These data show that unlike embryonic development, *in vitro* generated endocrine cells form in two waves, one in stage 4 and the other in stage 5 of the protocol.

It should be noticed that during stage 6 the proportion of endocrine cells continues to increase, but scRNA-seq shows that this is accompanied with a decline in the number of endocrine precursors detected (FIG. 1E and FIG. 5C). Thus, this is most likely not the result of a third wave of endocrine differentiation, but rather the result of existing endocrine precursors completing their differentiation, accompanied by a decline in the numbers of epithelial progenitors.

### α-cells start forming in the first wave of endocrine differentiation, whereas β-cells form in the second wave

Several findings suggest that the two waves of endocrine formation differ in the populations generated: (1) The α cell EP is utilize by cells already in the middle of stage 4, whereas the β cell and the non-pancreatic-endocrine programs are only employed by cells from the end of stage 5 onwards (FIG. 2C). (2) NKX6.1, which is expressed together with INS in β cells (FIG. 1D), is rarely detected together with either NEUROG3 or CHGA during stage 4. However, at the first day of stage 5, most of the newly emerging NEUROG3⁺ cells express NKX6.1 at medium levels, and then gradually turn into CHGA⁺NKX6.1^{high} endocrine cells (FIG. 2B and FIG. 7C). (3) The GCG⁺ INS⁺ polyhormonal cells that appear at stage 4 express ARX (Table 1), indicative of an α cell state. Conversely, bona fide CPEP⁺NKX6.1⁺ β cells appear almost exclusively during stage 5 (FIG. 2A).

To test this hypothesis, XXI was removed from the media. XXI serves to inhibit the notch pathway during stage 5, and induce general endocrine differentiation ^{2, 3, 34}. Its removal is therefore expected to hamper endocrine differentiation during stage 5, without affecting cells that had differentiated earlier. Indeed, XXI removal during stage 5 dramatically reduced the number CPEP⁺NKX6.1⁺ β cells, but did not significantly affect the number of GLP2⁺ α cells (FIG. 2D). Thus, interference with endocrine differentiation at stage 5 limits β cell differentiation, but does not effect the earlier formation of α cells. This confirms that the first wave of endocrine differentiation, during stage 4, produces primarily α cells, whereas the second wave, at stage 5, gives rise to β cells. This is reminiscent of normal mouse embryonic development, where α cell differentiation precedes that of β cells^{22, 52}.

### Identifying pathways that affect pancreas compartmentalization

The distinct fates of endocrine cells differentiating in two waves suggests that controlling the timing of differentiation could affect the composition of the clusters. To control the timing of differentiation, it was desired to find molecular mechanisms that act in the clusters autonomously, rather than through the direct effect of factors already included in the protocol. Ligand-receptor interaction analysis suggested multiple potential pairs that could either act in an autoregulatory process or between different cell types (FIGS. 7D-7E, Table 3).

To focus on genetic pathways that control the overall balance between undifferentiated progenitors and endocrine cells, cells with high usage values of the epithelial-progenitors EP were compared with cells that use the general endocrine EP (EPs 19 and 6, respectively) (FIG. 3A). The expression of more than 4,800 genes is significantly different between the two groups. (FIG. 7F). As expected, the endocrine population is enriched for transcripts related to islet function and with maturity onset diabetes of the young (MODY), including PAX4, PDX1, PAX6 and NEUROD1; and express higher levels of transcripts related to Insulin or Glucagon signaling (INS, GCG, ABCC8, GCK, FOXO1, G6PC2) (FIG. 3C, FIG. 7F, FIG. 8A). Additional genes enriched in these cells are related with general mechanisms of secretion including the endoplasmic reticulum, endocytosis and calcium reabsorption (Table 2). The mechanisms shared by endocrine cells and neurons are also evident with enrichment of transcripts related with synaptic transport (GABRB3, CAMK2B). Lastly, *in vitro* generated endocrine cells are enriched for transcripts related to several molecular pathways that have a role in endocrine function, including cAMP (RYR2, AKT3), cGMP/PKG (MAPK1, MAPK3), AMPK (RAB2A, CREB3), mTOR (PTEN, PRKCA), VEGF (VEGFA, SHC2) and PPAR (PPARA) signaling (Table 2).

Conversely, the epithelial progenitors are enriched for transcripts related to cell adhesion (CDH1, CDC42, ERBB2) and cell division (MCM7, CCND2, CHEK1, PCNA). They also express genes that indicate high activity of several signaling pathways known to have a role in progenitor-to-endocrine differentiation, including notch² (NOTCH2, NOTCH3, JAG1, HES1), hippo-Yap^{12, 47} (YAP1, AMOT), activin (SMAD2/3, TGFBR1, ACVR2A) and BMP (BMP2, BMP7, BMPR1B)⁸ (FIGS. 8C-8F). The progenitor population also expressed higher levels of transcripts related with the Pi3k-AKT pathway (PIK3R1/3), possibly in context with FGF activity and the enrichment of FGFR1 and 2 in these cells (FIG. 8G).

Unexpectedly, in spite of its known role in pancreas development, enrichment of hedgehog pathway activity was not observed in either cell type²⁴⁻²¹. Upon examination of specific components of the pathway it was noticed that progenitors express significantly higher levels of both SHH and its signal transducer SMO¹, but neither of the pathway's inhibitors PTCH1 or PTCH2 show a significant difference in RNA levels (FIG. 8H). Accordingly, in-situ hybridization showed that transcripts of PTCH1 are spread uniformly across the cluster, whereas SMO mRNA is dense in the non-endocrine region of the differentiating cluster, and sparse in the endocrine bud (FIG. 8I). Surprisingly, unlike its mRNA transcripts, high levels of PTCH1 protein were confined to the endocrine region both in clusters (FIG. 8J) and in developing mouse islets (FIG. 8K). This suggests that a post-translational mechanism acts to inhibit hedgehog signaling specifically in the endocrine compartment.

A similar mechanism whereby the pathway's inhibitor is confined to the endocrine compartment is observed for the WNT pathway. Transcripts upregulated in progenitors are enriched for factors related with the active form of WNT, including the ligand WNT3, the receptors FZD5 and 7, and the major pathway transducer β-catenin (CTNNB1), which shows a similar pattern on the protein level (FIG. 3E). Conversely, APC, which acts as part of the destruction complex that destabilizes CTNNB1 and inhibits WNT activity, is upregulated in the endocrine cells (FIG. 3D). In mouse embryos, Apc protein is detected exclusively in the endocrine peninsula area, where β-catenin is detected at lower levels than in the epithelial cords (FIG. 3F).

Finally, transcripts upregulated in progenitors are enriched for factors related to the active form of WNT, including the ligand WNT3, the receptors FZD5 and 7, and the major pathway transducer β-catenin (CTNNB1), which shows a similar pattern on the protein level (FIG. 3B and FIGS. 3D-3E). Conversely, APC which inhibits WNT activity, is upregulated in endocrine cells (FIG. 3D). In mouse embryos, Apc protein is detected exclusively in the endocrine peninsula area, whereas β-catenin levels are high in the epithelial cords (FIG. 3F).

### WNT pathway inhibition is necessary for endocrine differentiation in mice

High WNT pathway activity in progenitors and the opposing expression of APC, the WNT inhibitor, in the endocrine compartment, suggest WNT inhibition plays a role in endocrine differentiation. Wnt pathway has a role in early murine pancreas formation and in the later expansion of the acinar compartment, but previous reports are inconclusive about a possible role for the WNT pathway in endocrine differentiation^{10, 17, 32-33, 53}. Since the described protocol does not involve manipulation of the pathway at this advanced stage, it was decided to examine WNT pathway's role in endocrine differentiation.

First, the effect of Apc deletion on islet formation *in vivo* was examined. Apc is a central component of the destruction mechanism that degrades Ctnnb1, and in its absence, excess Ctnnb1 molecules accumulate in the nucleus, and constitutively activate WNT pathway targets²⁸. In agreement with the importance of WNT inactivation during early pancreas development³¹, induced deletion of Apc in the pancreatic bud of mouse embryos resulted in the formation of a rudimentary pancreas (FIGS. 8L-8N). To examine WNT's later role in the formation of the endocrine compartment, mice carrying a conditional knockout of Apc (APC^{MIN}, Apc^{loxP/loxP})²⁷ were crossed with mice expressing CRE recombinase specifically in cells expressing Neurog3⁴⁹. Cells that efficiently deleted Apc presented nuclear Ctnnb 1 accumulation (FIG. 4A), and failed to express either the pan-endocrine marker Chga (FIG. 4B) or the hormones Insulin and Glucagon (FIG. 8O). Since Neurog3 is also active during enteroendocrine cell differentiation²¹, APC was deleted in gut-cells as well, leading to hyperplastic lesions (FIG. 8P). Altogether, this shows that WNT pathway inhibition is necessary for proper endocrine differentiation in the mouse pancreas.

### WNT inhibition and BMP activation modulate the ratio between progenitors and endocrine cells

Finally, the potential of compartmentalizing pathways to regulate the ratio between progenitors and endocrine cells *in vitro* was examined. The focus was on WNT and BMP pathways, since they are not directly manipulated in the critical stages of the current protocol.

BMP is active in the epithelial-progenitors pool (FIG. 3B, FIG. 8F), and it was previously shown to attenuate endocrine differentiation^{35, 48}. Accordingly, addition of recombinant BMP2-7 at stage 4 resulted in downregulation of endocrine genes (FIG. 9D), and nearly twofold decrease in the number of CHGA⁺ endocrine cells (FIGS. 4C-4D). Concomitantly, the number of PDX1⁺/NKX6.1⁺ progenitors increased by more than twofold on average (FIGS. 4C-4D). It was confirmed that this in an independent cell line (FIG. 9C), but a similar effect when BMP2-7 was added during stage 5 was not observed.

Conversely, the role found for WNT inhibition in islet formation suggests it could be used to increase the number of endocrine cells generated *in vitro.* Indeed, treatment with the wnt-tankyrase inhibitor IWR1-endo⁶ during both stages 4 and 5 increased expression of endocrine markers and downregulated progenitor markers (FIG. 9D). Similar results were obtained using XAV939, another wnt-tankyrase inhibitor (FIG. 9D)¹⁸. To estimate the effect of WNT pathway activity on the actual yield of the protocol, the effect of its manipulation on cell proportions *in vitro* was measured. Activation of the WNT pathway using the GSK3 inhibitor CHIR99021⁴⁵ decreased the overall number of endocrine cells at stage 4 (FIG. 9A). In contrast, inhibition of the pathway with IWR1-endo during stage 4 significantly raised the proportion of CHGA⁺ endocrine cells and reduced the proportion of PDX⁺NKX6.1⁺ progenitors (FIGS. 4C-4D). IWR treatment during stage 5 caused an even higher relative increase in the proportion of CHGA⁺ endocrine cells (FIG. 4E and FIG. 9C), and specifically, the proportion of C-PEP⁺/NKX6.1⁺ β cells increased by more than 50% on average, relative to controls (FIGS. 4F-4G). The higher proportion of β cells upon IWR addition in stage 5 was maintained during stage 6 of the protocol (FIG. 4H), and overall cell numbers were slightly higher following treatment (FIG. 9E), indicating that IWR increases the overall yield of *in vitro* generated β cells. IWR treated cells maintain their ability to secrete insulin in response to high glucose levels (FIG. 9F), but upon consecutive stimulations with high glucose, IWR treated cells release less insulin in the second challenge, compared to controls (FIG. 9G).

These experiments show that the ratio between the progenitor pool and the differentiated endocrine cells can be regulated by the activity of either WNT or BMP pathways. The exogenous manipulation of the ratio between the two compartments at the right stage in the differentiation protocol affects β cell yield. Specifically, expanding the endocrine compartment through inhibition of the WNT pathway during β cell differentiation at stage 5, improves the yield of *in vitro* generated β cells.

### Discussion

Protocols for *in vitro* directed differentiation of hESCs into β cells are commonly described as linear processes that homogenously drive cells from one step to the next^{37, 39, 44, 48}. Using scRNA-seq it was found that differentiation is homogenous only at the first three stages of the protocol, after which epithelial progenitors and endocrine cells reside side by side. A wave of endocrine differentiation at stage 4 tends to produce α cells, and a second wave, at stage 5, gives rise to β cells and non-pancreatic endocrine cells (FIG. 4J).

These findings indicate that the *in vitro* differentiation protocol recapitulates multiple aspects of embryonic pancreas development. In both cases the epithelial cord progenitors and the endocrine peninsulas that emerge from them exist side by side, and in both cases α cell differentiation precedes β cells^{22, 11, 52}. However, differences were also noticed between the protocol and embryonic development, including the absence of early Cpa1⁺ multipotent progenitors that give rise to acinar, ductal and endocrine cells⁶¹, and the formation of non-enteroendocrine-like cells. It should be noted that the differentiating clusters may contain additional rare cell types, which have not been detected by analysis. Cell heterogeneity may be attributed to physical constraints such as the effect of cluster thickness on diffusion, but even planar differentiation protocols are often not homogenous²⁶. Here it is shown that cells initiate autonomous molecular programs, and in the case of WNT and hedgehog pathways, for example, opposing activity levels in the progenitor and endocrine compartments is observed, suggesting a regulatory cross-talk between the various cell types. By intervening with potential regulatory mechanisms the outcome of the differentiation process can be manipulated. Exogenous BMP activation increases the progenitor compartment in stage 4, whereas small molecule WNT inhibitors increase the opposite, endocrine population.

The ultimate goal of *in vitro* differentiation protocols is to provide diabetic patients with β cells derived from pluripotent stem cells instead of cadaveric islets. However, it should be noticed that the currently practiced medical protocol involves whole islet transplantation, rather than β cells alone. In fact, there is no certainty that transplanting a single cell type will be able to regain the function of the entire islet. It is therefore sensible to aim efforts at generating whole islets *in vitro,* rather than pure populations of β cells. This work joins others^{39, 59} in the attempt to characterize protocols for *in vitro* production of pancreatic endocrine cells at the single cell level. The use of scRNA-seq generates a large, unbiased dataset, which enabled the thorough characterization of the various cell types generated, the timing of their differentiation and the identification of molecular pathways that affect cell fate. Looking ahead, the ability to finely tune the timing and balance of progenitor vs. endocrine fates can be used to control the ratio between α and β cells, and promote the *in vitro* formation of islets with proper cell composition.

### Materials and Methods

### Animals and animal handling

To generate mice lacking APC in the pancreatic bud, heterozygote C57BL/6j-Apc*^{Min}*/J (Moser et al., 1990) (Jackson laboratory, Bar Harbor, ME) mice, carrying a mutation in the gene (APC^{min/+}), were crossed with B6.Cg-*Apc^{tm2Rak}*/Nci (NCI/NIH) mice²⁷ (APC^{loxp}) carrying a conditional knock out of Apc, and with Tg(Pdx1-cre)^{89.1Dam} mice¹⁵ that express CRE recombinase under a Pdx1 regulatory element. To delete Apc in endocrine cells, Tg(Neurog3-cre)^{C1Able/}J (Jackson laboratory, Bar Harbor, ME)⁴⁹ mice expressing CRE recombinase under a Neurog3 regulatory element were used with the previous Apc mice, to generate either heterozygotes that carry one mutated allele and one conditional allele, or mice that are homozygotes for the conditional allele. To validate CRE activity, these mice were also crossed with B6.129X1*-Gt(ROSA)26Sor^{tm1(EYFP)Cos}*/J(Jackson laboratory, Bar Harbor, ME) mice, that express YFP form the ROSA26 locus upon CRE mediated recombination. Both male and female mice were used for breeding all strains, except for C57BL/6j-Apc*^{Min}*/J, where only heterozygote males were used for breeding and crossed with females from other strains. Sections of adult mice are from 1 and 2 month old females. Sex of the newborns analyzed was not determined. Mice were euthanized through CO₂ inhalation prior to tissue isolation.

Animal studies were performed in strict accordance with the recommendations in the Guide for the Care and Use of Laboratory Animals of the National Institutes of Health. All of the animals were handled according to approved institutional animal care and use committee (IACUC) protocol number [16-05-269]. The protocol was approved by the Committee on the Use of Animals in Research and Teaching of Harvard University Faculty of Arts & Sciences (HU/FAS). The HU/FAS animal care and use program is AAALAC International accredited, has a PHS Assurance (A3593-01) on file with NIH's Office of Laboratory Animal Welfare, and is registered with the USDA (14-R-0128).

### HESC culture and differentiation

HUES8 (male) human embryonic stem cells and human induced pluripotent stem cells (hiPSCs) from line 1016 (male) were obtained from the Human Embryonic Stem Cell Facility and iPS Core Facility of the Harvard Stem Cell Institute. Cells were differentiated into beta like cells in suspension as described previously³⁷. Briefly, 150Xe⁶ cells were suspended in 300ml of supplemented mTeSR1 and grown in spinning flasks (70rpm, 37°C, 5%CO₂) for 48 hours prior to the beginning of a stepwise differentiation protocol:
**Stage 1** - 24 hours in S1 medium supplemented with ActivinA (100ng/ml) and CHIR99021 (1.4µg/ml), followed by 48 hours without CHIR99021.
**Stage 2** - 72 hours in S2 medium supplemented with KGF (50ng/ml).
**Stage 3** - 48 hours in S3 medium supplemented with KGF (50ng/ml), LDN193189 (200nM), Sant1 (0.25µM), retinoic acid (2µM), and PDBU (500nM).
**Stage 4** - 5 days in S3 medium supplemented with KGF (50ng/ml), Sant1 (0.25µM) and retinoic acid (0.1µM).
**Stage 5** - 7 days in BE5 medium supplemented with Beta Cellulin (20ng/ml). XXI (1µM), Alk5iII(10 µM) and T3 (1µM). Sant1 (0.25µM) was added in the first three days, and retinoic acid was added at 0.1µM in the first three days, then at 0.025µM.
**Stage 6** - CMRLS medium supplemented with Alk5iII (10µM) and T3 (1µM).

In many of the experiments following cell collection for sc-RNAseq, the protocol was slightly modified to add Y-27632 (10µM) at stage 3-5, and ActivinA (5ng/ml) in stage 4. In some cases, an extra day at stage 4 was also added. For the presented functional and flow cytometry analysis in stage 6, CMRLS based media was replaced with S3 medium, without supplements.

Factors were tested in either 6-well plates placed on a rocker, or in Biott ABLE Bioreactors (Stemgent). IWR1-endo (Sigma-Aldrich) was used at 5µM. XAV939 (Santa Cruz, SC-296704A) was used at 5 µM. Recombinant human BMP2/BMP7 (Thermo-Fisher) was used at 100µg/ml. when added during stage 4, CHIR99021 was used at a concentration of 1.4-1.55µg/ml.

### Preparation of single-cell suspension

Clusters were collected prior to commencement of differentiation, and at the last day of stages 1-5. In addition, on the 3^{rd} day of stages 4 and 5, and on the 2^{nd}, 7^{th} and 14^{th} days of stage 6. The clusters were allowed to settle, washed in PBS and dissociated by 5min incubation in TrypLE Express (GIBCO) at 37°C, followed by mechanical agitation through pipetting. TrypLE activity was terminated by washing with PBS+1% FCS.

### Single cell library preparation and sequencing

Single-cell capture, cell lysis, reverse transcription of full-length mRNA and cDNA amplification were performed using the Fluidigm C1 system and the Smart-seq protocol⁴³. Sequencing libraries were prepared using the Illumina Nextera XT DNA library preparation kit and samples were sequenced on an Illumina HiSeq.

### Data Analysis

### Alignment and processing of sequencing reads, data conversion and cell filtering

Reads were aligned to the human reference genome build hg19 with TopHat (v2.0.13), which was provided with gene annotations from GENCODE (v17). Gene expression profiles for each cell were computed using Cufflinks (2.2.0) as previously described in Trapnell et al.⁵⁷ Per-cell fragments per kilobase million (FPKM) measures were converted into relative transcript counts using the Census algorithm from the Monocle package^{40-41, 57}. Cells with <1k detected genes or with >45k total mRNA were removed. Additional potential outliers were removed by defining lower and upper thresholds based on the appearance of the per-cell total mRNA distribution curve, separately for each sample. This approach automatically accounted for all cells lying outside of 2 standard deviations of each sample's mean log-transformed per-cell total mRNA count and resulted in final number of 773 single-cell expression profiles (949 pre-filtering), with a median of 898,228 (mean: 945,907) usable paired end reads per cell (FIG. 5B).

### Inference of cellular gene expression programs via topic modeling

The topic model (TM) is a probabilistic unsupervised learning algorithm which is designed to discover latent topics present in a set of text documents, based solely on the observed usage of each word in each document⁴. For text data, a topic is defined as set of (a priori unspecified) related words which probabilistically tend to co-occur. For example, statistical co-occurrence of words such as "banks", "market", and "GDP" within the same document provides strong evidence that a topic such as finance is being discussed. Once the topics have been inferred, a document can be more interpretably represented in terms of the relative presence of each topic rather than a massive collection of word frequencies. In the analysis, the topic model and inference algorithm used in the GeneProgram software tool¹³ were utilized, which employs a Hierarchical Dirichlet Process (HDP) prior. This enables automatic inference of the number of latent topics (along with the topics themselves) from the data⁵⁵.

TM is adapted to scRNA-seq data by treating each cell as a document and each gene as a word. Within the cell, the unit amount of mRNA from the gene is therefore the genomic equivalent of a word's frequency within a document. In this context, referral is made to a latent topic as an Expression Program (EP), which represents a set of genes that behave coordinately in particular cells. Unlike traditional clustering techniques, this TM analysis allows many EPs to be active within a given cell, and a single gene may also be used by different EPs (one gene can regulate multiple different biological processes depending on the context in which it is expressed). For each EP, a gene receives a relevance value (quantifying how active the gene is within the EP) and a cell is assigned a usage value (quantifying how active the EP is within the cell). After inferring the EPs underlying the scRNA-seq measurements via Markov-Chain Monte Carlo¹³, each cell can be can interpretably represented and classified in terms of its relative usage of different EPs, rather than the raw expression values of many thousand genes. Similarly, each EP is itself represented as a collection of genes along with their "relevance" in defining the EP. The biological significance of an EP can thus be elucidated by investigating which genes are the most actively used¹³.

The TM software used to infer the EPs in this analysis is available at groups.csail.mit.edu/cgs/geneprogram.html, and was run with the following settings: 4 levels of discretization for the expression values, 100 posterior samples (taken every 1,000 steps of the MCMC-chain with an initial burn-in of 30,000 steps), minimum of 2 genes per EP with a minimum usage value of 0.1, with the default prior HDP concentration and minimum-similarity/merging parameters used in Gerber et al.¹³

### SIMLR analysis

HESCs were removed from the 773 cleaned cells, retaining 720 cells for all subsequent analyses. A polynomial trend curve was fitted to the mean-variance plot of gene FPKM counts, and used as an estimate for technical variability. Altogether 3453 genes showed higher variance, and were used to cluster the cells using the SIMLR method implemented in the SIMLR R library⁶⁰. SIMLR calculates a cell-cell similarity metric based on parametrized Gaussian Kernels and uses the similarity values as input to the stochastic neighbor embedding method to plot the cell data in reduced dimensions. K-means clustering was subsequently used to assign the cells to different numbers of clusters, eventually settling on 10 predetermined clusters.

Differential expression was determined using the ROTS algorithm⁵⁴. P-values were corrected for multiple testing using the false discovery rate (FDR), and 4863 genes with FDR of less or equal to 0.05 were further characterized using Enrichr pathway analysis⁷. The KEGG database²³ was used as a source of curated lists of pathway genes.

### Ligand-receptor interaction analysis

Ligands and receptors from Ramilowski et al.⁴² were introduced into the stage-specific analysis if expressed in 20% or more of the cells analyzed from that stage. The expression of these genes was compared between the cells belonging to the EPs analyzed on a given stage, and those that were significantly differentially expressed between EPs were retained and assigned to the EP where their average expression level is highest. This analysis was performed separately for stages 4 and 5.

### Flow cytometry analysis

Differentiated cell clusters were dispersed into single-cell suspension by incubation in TrypLE Express at 37°C, quenched with PBS+2% FBS, fixed with 4% PFA for 30 min at 4°C, washed once in PBS, and resuspended in PBS+ 2% FBS. Staining was done in 96-well plates. Cells were incubated in blocking buffer (PBS+0.1% Triton X-100+5% donkey serum) at 4°C for 30-60 min. Cells were then re-suspended in blocking buffer with primary antibodies and incubated at room temperature for 1 hour or at 4°C overnight. Cells were washed twice in blocking buffer and incubated in blocking buffer with secondary antibodies at 4°C for 1 hour. Cells were then washed three times in PBS+5% Donkey serum and analyzed using either the BD Accuri c6 flow cytometer or the LSR-II flow cytometer (BD Biosciences). Analysis of the results was performed using FlowJo software.

### Nano-string gene expression analysis

Cells were collected for RNA extraction in RLT Lysis Buffer (Qiagen) and frozen at -80°C. RNA was extracted using the RNeasy Mini Kit (Qiagen), according the manufacturer's instructions - including DNA digestion, and 100ng of RNA was used for each reaction. Nanostring set up was performed according to the protocol for the nCounter XT Gene Expression Assay. Data was analyzed in the nSolver 2.5 Software. All genes were normalized to housekeeping genes (ITCH, RPL15, RPL19, TCEB1, UBE2D3). Analysis of changes in gene expression of markers for endocrine and progenitor cells was done using R.

### Immunofluorescence staining and In-situ hybridization

Isolated tissue was fixed overnight in 4% paraformaldehyde, washed in PBS, embedded in paraffin and sectioned on a microtome. Paraffin was removed using Histo-Clear (National Diagnostics) and serial washes in declining ETOH concentrations. After antigen retrieval, samples were blocked in PBS with 5% donkey serum and 0.1% Triton x-100 for 1 hour in room temperature. Primary antibody was diluted in the blocking solution and incubated in room temperature for 1hr, followed by washing. Secondary antibody was incubated for 1 hour in room temperature, washed, and DAPI staining was added.

In situ hybridization was performed using ViewRNA ISH tissue assay kit. For detection of human SMO and PTCH1 transcripts, VA1-14719 and VA6-20367 probes were used, respectively.

### Glucose Stimulated Insulin Secretion (GSIS)

Differentiated cells were washed twice in Krebs buffer (Krb) containing 2.8mM glucose, and then distributed into 24-well plates containing Millicell Cell culture inserts for easy transfer between conditions. Each well received 0.5ml buffer with approximately 10⁶ cells. After fasting in 2.8mM glucose for 1 hour in 37°C, the analysis of the cells' response to changing glucose levels was performed by a series of 1 hour incubations in 37°C, in the following order: 1) 2.8mM (low) glucose. 2) 20mM (high) glucose. 3) brief wash in 2.8mM glucose. 4) 1 hour incubation in 2.8mM glucose. 5) 20mM glucose. 6) 2.8mM glucose with 30mM KCL. At the end of each step (excluding the wash), the incubation buffer was collected and the amount of insulin secreted during the hour's incubation was determined using human insulin ELISA kit (ALPCO diagnostics; 80-INSHUU-E01.1). Clusters were then dispersed into single cells using TrypLE Express (Life Technologies), and cell number was counted automatically by a Vi-Cell (Beckman Coulter).

### Quantification and Statistical Analysis

All statistical methods used are described in the legend to the relevant figures. n marks the number of times an experiment was performed on independent differentiation batches, except for FIG. 9C, which describes a triplicate of samples taken from the same differentiation batch. Data was analyzed and plotted using Prism software from GraphPad. When box plots are presented, the bottom and top box borders represent the 25^{th} and 75^{th} percentiles, respectively. The line inside the box represents the median, and the whiskers represent the minimum and maximum values observed.

### Resources

| REAGENT or RESOURCE | SOURCE | IDENTIFIER |
|---|---|---|
| Antibodies | | |
| Rat anti C-peptide | DSHB | Cat#GN-ID4 |
| Rabbit anti Chaga | Abeam | Cat#Ab15160 |
| Chicken anti GFP | Aves Labs inc. | Cat#GFP-1020 |
| Goat anti glp2 | Santa Cruz Biotechnology | Cat#sc-7781 |
| Mouse anti Glucagon | Santa Cruz Biotechnology | Cat#sc-514592 |
| Goat anti Neurod1 | R&D systems | Cat#AF2746 |
| Sheep anti Neurogenin3 | R&D systems | Cat#AF3444 |
| Mouse anti Nkx6.1 | DSHB | Cat#F55A12 |
| Rabbit anti Sox9 | Abeam | Cat#Ab-5535 |
| Goat anti PDX1 | R&D systems | Cat#AF2419 |
| Mouse anti somatostatin | Santa Cruz Biotechnology | Cat#sc-55565 |
| Rabbit anti MCM3 | Cell Signaling Technology | Cat#4012s |
| Mouse anti Ctnnb1 | ThermoFisher Scientific | Cat#MA1-2001 |
| Goat anti Patched1 | Santa Cruz Biotechnology | Cat#sc-6147 |
| Rabbit anti APC | Abeam | Cat#Ab-15270 |
| Mouse anti E-cadherin | BD Transduction Laboratories | Cat#610181 |

| Chemicals, Peptides, and Recombinant Proteins | | |
|---|---|---|
| ActivinA | R&D Systems | Cat#338-AC |
| Rock Inhibitor Y-27632 | DNSK | Cat#DNSK-KI15-02 |
| Chir99021 | Stemgent | Cat#04-0004-10 |
| KGF | Peprotech | Cat#100-19 |
| Sant1 | Sigma-Aldrich | Cat#S4572 |
| PDBu | EMD Millipore | Cat#524390 |
| XXI | EMD Millipore | Cat#565790 |
| Alk5i II | Axxora | Cat#ALX-270-445 |
| T3 | EMD Millipore | Cat#642511 |
| Betacellulin | ThermoFisher Scientific | |
| IWR1-endo | Sigma-Aldrich | Cat#10161 |
| XAV939 | Santa Cruz Biotechnology | Cat#sc-296704A |
| Recombinant Human BMP2/BMP-7 Heterodimer | ThermoFisher Scientific | Cat#3229-BM |

| Critical Commercial Assays | | |
|---|---|---|
| ViewRNA ISH Tissue Assay Kit (2-plex) | ThermoFisher | Cat#QVT0012 |
| Anti human SMO ViewRNA probeset | ThermoFisher | Cat#VA1-14719 |
| Anti human PTCH1 ViewRNA probeset | ThermoFisher | Cat#VA6-20367 |
| SMARTer^{®} Ultra Low Kit for the Fluidigm^{®} C1 System | Clontech | Cat#634833 |
| C1 Single-Cell Auto Prep Array for mRNA Seq (17-25µm) | Fluidigm | Cat#100-5761 |

| Deposited Data | | |
|---|---|---|
| Raw and analyzed single cell sequencing data | This paper | SRA BioProject PRJNA532884 |

| Experimental Models: Cell Lines | | |
|---|---|---|
| HUES8 hESCS | HSCI | hES Cell line: HUES-8 |
| hiPSC 1016 | HSCI | hiPSC 1016 |

| Experimental Models: Organisms/Strains | | |
|---|---|---|
| Mouse: C57BL/6j-Apc*^{Min}*/J | Jackson Laboratories | RRID:IMSR_JAX:00 2020 |
| Mouse: B6.Cg-Apc*^{tm2Rak}*/Nci | NCI Mouse Repository | RRID:IMSR_NCIMR: 01XAA |
| Mouse: Tg(Pdx1-cre)^{89.1Dam} | In house | RRID:MGI:5902759 |
| Mouse: Tg(Neurog3-cre)^{C1Able/}J | Jackson Laboratories | RRID:IMSR_JAX:005 667 |
| Mouse: B6.129X1-*Gt(ROSA)26Sor^{tm1(EYFP)Cos}*/J | Jackson Laboratories | RRID:IMSR_JAX:006 148 |

| Software and Algorithms | | |
|---|---|---|
| Topic modeling | This paper | groups.csail.mit.edu/ cgs/geneprogram.ht ml |
| Interactive database | This paper | ifx.rc.fas.harvard.ed u/invitrobetacells/ |

### References

1. Alcedo, J., Zou, Y., and Noll, M. (2000). Posttranscriptional regulation of smoothened is part of a self-correcting mechanism in the Hedgehog signaling system. Mol Cell 6, 457-465.
2. Apelqvist, A., Li, H., Sommer, L., Beatus, P., Anderson, D.J., Honjo, T., Hrabe de Angelis, M., Lendahl, U., and Edlund, H. (1999). Notch signalling controls pancreatic cell differentiation. Nature 400, 877-881.
3. Beher, D., Wrigley, J.D., Nadin, A., Evin, G., Masters, C.L., Harrison, T., Castro, J.L., and Shearman, M.S. (2001). Pharmacological knock-down of the presenilin 1 heterodimer by a novel gamma -secretase inhibitor: implications for presenilin biology. J Biol Chem 276, 45394-45402.
4. Blei, D.M. (2012). Probabilistic topic models. Communications of the ACM 55, 77-84.
5. Bort, R., Martinez-Barbera, J.P., Beddington, R.S., and Zaret, K.S. (2004). Hex homeobox gene-dependent tissue positioning is required for organogenesis of the ventral pancreas. Development 131, 797-806.
6. Chen, B., Dodge, M.E., Tang, W., Lu, J., Ma, Z., Fan, C.W., Wei, S., Hao, W., Kilgore, J., Williams, N.S., et al. (2009). Small molecule-mediated disruption of Wnt-dependent signaling in tissue regeneration and cancer. Nat Chem Biol 5, 100-107.
7. Chen, E.Y., Tan, C.M., Kou, Y., Duan, Q., Wang, Z., Meirelles, G.V., Clark, N.R., and Ma'ayan, A. (2013). Enrichr: interactive and collaborative HTML5 gene list enrichment analysis tool. BMC Bioinformatics 14, 128.
8. Chung, W.S., Andersson, O., Row, R., Kimelman, D., and Stainier, D.Y. (2010). Suppression of Alk8-mediated Bmp signaling cell-autonomously induces pancreatic beta-cells in zebrafish. Proc Natl Acad Sci U S A 107, 1142-1147.
9. Decker, K., Goldman, D.C., Grasch, C.L., and Sussel, L. (2006). Gata6 is an important regulator of mouse pancreas development. Dev Biol 298, 415-429.
10. Dessimoz, J., Bonnard, C., Huelsken, J., and Grapin-Botton, A. (2005). Pancreas-specific deletion of beta-catenin reveals Wnt-dependent and Wnt-independent functions during development. Curr Biol 15, 1677-1683.
11. Filipe, M., Goncalves, L., Bento, M., Silva, A.C., and Belo, J.A. (2006). Comparative expression of mouse and chicken Shisa homologues during early development. Dev Dyn 235, 2567-2573.
12. Gao, T., Zhou, D., Yang, C., Singh, T., Penzo-Mendez, A., Maddipati, R., Tzatsos, A., Bardeesy, N., Avruch, J., and Stanger, B.Z. (2013). Hippo signaling regulates differentiation and maintenance in the exocrine pancreas. Gastroenterology 144, 1543-1553, 1553 e1541.
13. Gerber, G.K., Dowell, R.D., Jaakkola, T.S., and Gifford, D.K. (2007). Automated discovery of functional generality of human gene expression programs. PLoS Comput Biol 3, e148.
14. Godwin, A.R., Stadler, H.S., Nakamura, K., and Capecchi, M.R. (1998). Detection of targeted GFP-Hox gene fusions during mouse embryogenesis. Proc Natl Acad Sci U S A 95, 13042-13047.
15. Gu, G., Dubauskaite, J., and Melton, D.A. (2002). Direct evidence for the pancreatic lineage: NGN3+ cells are islet progenitors and are distinct from duct progenitors. Development 129, 2447-2457.
16. Gunawardene, A.R., Corfe, B.M., and Staton, C.A. (2011). Classification and functions of enteroendocrine cells of the lower gastrointestinal tract. Int J Exp Pathol 92, 219-231.
17. Heiser, P.W., Lau, J., Taketo, M.M., Herrera, P.L., and Hebrok, M. (2006). Stabilization of beta-catenin impacts pancreas growth. Development 133, 2023-2032.
18. Huang, S.M., Mishina, Y.M., Liu, S., Cheung, A., Stegmeier, F., Michaud, G.A., Charlat, O., Wiellette, E., Zhang, Y., Wiessner, S., et al. (2009). Tankyrase inhibition stabilizes axin and antagonizes Wnt signalling. Nature 461, 614-620.
19. Jacquemin, P., Pierreux, C.E., Fierens, S., van Eyll, J.M., Lemaigre, F.P., and Rousseau, G.G. (2003). Cloning and embryonic expression pattern of the mouse Onecut transcription factor OC-2. Gene Expr Patterns 3, 639-644.
20. Jennings, R.E., Berry, A.A., Kirkwood-Wilson, R., Roberts, N.A., Hearn, T., Salisbury, R.J., Blaylock, J., Piper Hanley, K., and Hanley, N.A. (2013). Development of the human pancreas from foregut to endocrine commitment. Diabetes 62, 3514-3522.
21. Jenny, M., Uhl, C., Roche, C., Duluc, I., Guillermin, V., Guillemot, F., Jensen, J., Kedinger, M., and Gradwohl, G. (2002). Neurogenin3 is differentially required for endocrine cell fate specification in the intestinal and gastric epithelium. EMBO J 21, 6338-6347.
22. Johansson, K.A., Dursun, U., Jordan, N., Gu, G., Beermann, F., Gradwohl, G., and Grapin-Botton, A. (2007). Temporal control of neurogenin3 activity in pancreas progenitors reveals competence windows for the generation of different endocrine cell types. Dev Cell 12, 457-465.
23. Kanehisa, M., Furumichi, M., Tanabe, M., Sato, Y., and Morishima, K. (2017). KEGG: new perspectives on genomes, pathways, diseases and drugs. Nucleic Acids Res 45, D353-D361.
24. Kawahira, H., Ma, N.H., Tzanakakis, E.S., McMahon, A.P., Chuang, P.T., and Hebrok, M. (2003). Combined activities of hedgehog signaling inhibitors regulate pancreas development. Development 130, 4871-4879.
25. Kawahira, H., Scheel, D.W., Smith, S.B., German, M.S., and Hebrok, M. (2005). Hedgehog signaling regulates expansion of pancreatic epithelial cells. Dev Biol 280, 111-121.
26. Kelly, O.G., Chan, M.Y., Martinson, L.A., Kadoya, K., Ostertag, T.M., Ross, K.G., Richardson, M., Carpenter, M.K., D'Amour, K.A., Kroon, E., et al. (2011). Cell-surface markers for the isolation of pancreatic cell types derived from human embryonic stem cells. Nat Biotechnol 29, 750-756.
27. Kuraguchi, M., Wang, X.P., Bronson, R.T., Rothenberg, R., Ohene-Baah, N.Y., Lund, J.J., Kucherlapati, M., Maas, R.L., and Kucherlapati, R. (2006). Adenomatous polyposis coli (APC) is required for normal development of skin and thymus. PLoS Genet 2, e146.
28. MacDonald, B.T., Tamai, K., and He, X. (2009). Wnt/beta-catenin signaling: components, mechanisms, and diseases. Dev Cell 17, 9-26.
29. Miyatsuka, T., Li, Z., and German, M.S. (2009). Chronology of islet differentiation revealed by temporal cell labeling. Diabetes 58, 1863-1868.
30. Moser, A.R., Pitot, H.C., and Dove, W.F. (1990). A dominant mutation that predisposes to multiple intestinal neoplasia in the mouse. Science 247, 322-324.
31. Munoz-Bravo, J.L., Flores-Martinez, A., Herrero-Martin, G., Puri, S., Taketo, M.M., Rojas, A., Hebrok, M., and Cano, D.A. (2016). Loss of Pancreas upon Activated Wnt Signaling Is Concomitant with Emergence of Gastrointestinal Identity. PLoS One 11, e0164714.
32. Murtaugh, L.C. (2008). The what, where, when and how of Wnt/beta-catenin signaling in pancreas development. Organogenesis 4, 81-86.
33. Murtaugh, L.C., Law, A.C., Dor, Y., and Melton, D.A. (2005). Beta-catenin is essential for pancreatic acinar but not islet development. Development 132, 4663-4674.
34. Murtaugh, L.C., Stanger, B.Z., Kwan, K.M., and Melton, D.A. (2003). Notch signaling controls multiple steps of pancreatic differentiation. Proc Natl Acad Sci U S A 100, 14920-14925.
35. Nostro, M.C., Sarangi, F., Ogawa, S., Holtzinger, A., Corneo, B., Li, X., Micallef, S.J., Park, I.H., Basford, C., Wheeler, M.B., et al. (2011). Stage-specific signaling through TGFbeta family members and WNT regulates patterning and pancreatic specification of human pluripotent stem cells. Development 138, 861-871.
36. Osipovich, A.B., Long, Q., Manduchi, E., Gangula, R., Hipkens, S.B., Schneider, J., Okubo, T., Stoeckert, C.J., Jr., Takada, S., and Magnuson, M.A. (2014). Insm1 promotes endocrine cell differentiation by modulating the expression of a network of genes that includes Neurog3 and Ripply3. Development 141, 2939-2949.
37. Pagliuca, F.W., Millman, J.R., Gurtler, M., Segel, M., Van Dervort, A., Ryu, J.H., Peterson, Q.P., Greiner, D., and Melton, D.A. (2014). Generation of functional human pancreatic beta cells in vitro. Cell 159, 428-439.
38. Pan, F.C., and Wright, C. (2011). Pancreas organogenesis: from bud to plexus to gland. Dev Dyn 240, 530-565.
39. Petersen, M.B.K., Azad, A., Ingvorsen, C., Hess, K., Hansson, M., Grapin-Botton, A., and Honore, C. (2017). Single-Cell Gene Expression Analysis of a Human ESC Model of Pancreatic Endocrine Development Reveals Different Paths to beta-Cell Differentiation. Stem Cell Reports 9, 1246-1261.
40. Qiu, X., Hill, A., Packer, J., Lin, D., Ma, Y.A., and Trapnell, C. (2017). Single-cell mRNA quantification and differential analysis with Census. Nat Methods 14, 309-315.
41. Qiu X, M.Q., Tang Y, Wang L, Chawla R, Pliner H, Trapnell C (2017). Reversed graph embedding resolves complex single-cell developmental trajectories. Nature Methods.
42. Ramilowski, J.A., Goldberg, T., Harshbarger, J., Kloppmann, E., Lizio, M., Satagopam, V.P., Itoh, M., Kawaji, H., Carninci, P., Rost, B., et al. (2015). A draft network of ligand-receptor-mediated multicellular signalling in human. Nat Commun 6, 7866.
43. Ramskold, D., Luo, S., Wang, Y.C., Li, R., Deng, Q., Faridani, O.R., Daniels, G.A., Khrebtukova, I., Loring, J.F., Laurent, L.C., et al. (2012). Full-length mRNA-Seq from single-cell levels of RNA and individual circulating tumor cells. Nat Biotechnol 30, 777-782.
44. Rezania, A., Bruin, J.E., Arora, P., Rubin, A., Batushansky, I., Asadi, A., O'Dwyer, S., Quiskamp, N., Mojibian, M., Albrecht, T., et al. (2014). Reversal of diabetes with insulin-producing cells derived in vitro from human pluripotent stem cells. Nat Biotechnol 32, 1121-1133.
45. Ring, D.B., Johnson, K.W., Henriksen, E.J., Nuss, J.M., Goff, D., Kinnick, T.R., Ma, S.T., Reeder, J.W., Samuels, I., Slabiak, T., et al. (2003). Selective glycogen synthase kinase 3 inhibitors potentiate insulin activation of glucose transport and utilization in vitro and in vivo. Diabetes 52, 588-595.
46. Rodriguez-Seguel, E., Mah, N., Naumann, H., Pongrac, I.M., Cerda-Esteban, N., Fontaine, J.F., Wang, Y., Chen, W., Andrade-Navarro, M.A., and Spagnoli, F.M. (2013). Mutually exclusive signaling signatures define the hepatic and pancreatic progenitor cell lineage divergence. Genes Dev 27, 1932-1946.
47. Rosado-Olivieri, E.A., Anderson, K., Kenty, J.H., and Melton, D.A. (2019). YAP inhibition enhances the differentiation of functional stem cell-derived insulin-producing beta cells. Nat Commun 10, 1464.
48. Russ, H.A., Parent, A.V., Ringler, J.J., Hennings, T.G., Nair, G.G., Shveygert, M., Guo, T., Puri, S., Haataja, L., Cirulli, V., et al. (2015). Controlled induction of human pancreatic progenitors produces functional beta-like cells in vitro. EMBO J 34, 1759-1772.
49. Schonhoff, S.E., Giel-Moloney, M., and Leiter, A.B. (2004). Neurogenin 3-expressing progenitor cells in the gastrointestinal tract differentiate into both endocrine and non-endocrine cell types. Dev Biol 270, 443-454.
50. Segerstolpe, A., Palasantza, A., Eliasson, P., Andersson, E.M., Andreasson, A.C., Sun, X., Picelli, S., Sabirsh, A., Clausen, M., Bjursell, M.K., et al. (2016). Single-Cell Transcriptome Profiling of Human Pancreatic Islets in Health and Type 2 Diabetes. Cell Metab 24, 593-607.
51. Shapiro, A.M., Lakey, J.R., Ryan, E.A., Korbutt, G.S., Toth, E., Warnock, G.L., Kneteman, N.M., and Rajotte, R.V. (2000). Islet transplantation in seven patients with type 1 diabetes mellitus using a glucocorticoid-free immunosuppressive regimen. N Engl J Med 343, 230-238.
52. Sharon, N., Chawla, R., Mueller, J., Vanderhooft, J., Whitehorn, L.J., Rosenthal, B., Gürtler, M., Estanboulieh, R.R., Shvartsman, D., Gifford, D.K., et al. (2019). A peninsular structure coordinates asynchronous differentiation with morphogenesis to generate pancreatic islets. Cell 176, 790-804.
53. Strom, A., Bonal, C., Ashery-Padan, R., Hashimoto, N., Campos, M.L., Trumpp, A., Noda, T., Kido, Y., Real, F.X., Thorel, F., et al. (2007). Unique mechanisms of growth regulation and tumor suppression upon Ape inactivation in the pancreas. Development 134, 2719-2725.
54. Suomi, T., Seyednasrollah, F., Jaakkola, M.K., Faux, T., and Elo, L.L. (2017). ROTS: An R package for reproducibility-optimized statistical testing. PLoS Comput Biol 13, e1005562.
55. Teh YW, J.M., Beal MJ, Blei DM (2006). Hierarchical Dirichlet Processes. J Am Stat Assoc 101, 1566-1581.
56. Thomas, P.Q., Brown, A., and Beddington, R.S. (1998). Hex: a homeobox gene revealing peri-implantation asymmetry in the mouse embryo and an early transient marker of endothelial cell precursors. Development 125, 85-94.
57. Trapnell, C., Cacchiarelli, D., Grimsby, J., Pokharel, P., Li, S., Morse, M., Lennon, N.J., Livak, K.J., Mikkelsen, T.S., and Rinn, J.L. (2014). The dynamics and regulators of cell fate decisions are revealed by pseudotemporal ordering of single cells. Nat Biotechnol 32, 381-386.
58. Uhlen, M., Fagerberg, L., Hallstrom, B.M., Lindskog, C., Oksvold, P., Mardinoglu, A., Sivertsson, A., Kampf, C., Sjostedt, E., Asplund, A., et al. (2015). Proteomics. Tissue-based map of the human proteome. Science 347, 1260419.
59. Veres, A., Faust, A.L., Bushnell, H.L., Engquist, E.N., Kenty, J.H.-R., Harb, G., Poh, Y.-C., Sintov, E., Gürtler, M., Pagliuca, F.W., et al. (in press). Charting cellular identity during human in vitro beta cell differentiation. Nature.
60. Wang, B., Zhu, J., Pierson, E., Ramazzotti, D., and Batzoglou, S. (2017). Visualization and analysis of single-cell RNA-seq data by kernel-based similarity learning. Nat Methods 14, 414-416.
61. Zhou, Q., Law, A.C., Rajagopal, J., Anderson, W.J., Gray, P.A., and Melton, D.A. (2007). A multipotent progenitor domain guides pancreatic organogenesis. Dev Cell 13, 103-114.

### ADDITIONAL STATEMENTS OF INVENTION

In addition to the earlier statements of invention, the invention provides:
1. A method of increasing differentiation of pluripotent cells into pancreatic endocrine cells, the method comprising contacting a population of stem cells with at least one WNT pathway inhibitor.
2. A method of increasing differentiated endocrine cell yield comprising contacting a population of stem cells with at least one WNT pathway inhibitor.
3. A method of directing differentiation of a population of cells comprising contacting a population of stem cells with at least one WNT pathway inhibitor.
4. The method of any of paragraphs 1-3, wherein the WNT pathway inhibitor comprises a tankyrase inhibitor.
5. The method of any of paragraphs 1-4, wherein the WNT pathway inhibitor is selected from IWR1-endo, XAV939, AZ6102, JW55, MN64, TC-E5001, WIKI4, or combinations thereof.
6. The method of any of paragraphs 1-5, wherein the population of stem cells comprises pancreatic bud cells.
7. The method of any of paragraphs 1-6, wherein the population of stem cells is contacted with the WNT pathway inhibitor at stage 4 of a differentiation protocol.
8. The method of any of paragraphs 1-7, wherein the contacting of the population of stem cells with the WNT pathway inhibitor at stage 4 increases the number of CHGA⁺ cells by about 20%.
9. The method of any of paragraphs 1-8, wherein the contacting of the population of stem cells with the WNT pathway inhibitor at stage 4 decreases the number of PDX⁺NKX6.1⁺ progenitor cells by more than 30%.
10. The method of any of paragraphs 1-9, wherein the contacting of the population of stem cells with the WNT pathway inhibitor at stage 4 increases the yield of stem cell-derived alpha cells.
11. The method of any of paragraphs 1-5, wherein the population of stem cells is contacted with the WNT pathway inhibitor at stage 5 of a differentiation protocol.
12. The method of paragraph 11, wherein the contacting of the population of stem cells with the WNT pathway inhibitor at stage 5 increases the number of CHGA⁺ cells by about 50%.
13. The method of paragraph 11 or paragraph 12, wherein the contacting of the population of stem cells with the WNT pathway inhibitor at stage 5 increases the number of C-PEP⁺ cells by more than 50%.
14. The method of paragraph 13, wherein the C-PEP⁺ cells are C-PEP⁺/NKX6.1⁺ cells.
15. The method of any of paragraphs 11-14, wherein the contacting of the population of stem cells with the WNT pathway inhibitor at stage 5 increases the yield of stem cell-derived beta cells.
16. The method of any of paragraphs 1-15, wherein the population of stem cells is contacted with the WNT pathway inhibitor at stages 4 and 5 of a differentiation protocol.
17. The method of any of paragraphs 1-16, further comprising contacting the population of stem cells with a BMP pathway inhibitor.
16. The method of paragraph 17, wherein the population of stem cells are contacted with the BMP pathway inhibitor at stage 4 of a differentiation protocol.
17. The method of paragraph 17, wherein the population of stem cells are contacted with the BMP pathway inhibitor at stage 5 of a differentiation protocol.
18. The method of paragraph 17, wherein the population of stem cells are contacted with the BMP pathway inhibitor at stages 4 and 5 of a differentiation protocol.
19. A method of increasing differentiation of pluripotent cells into epithelial progenitor cells, the method comprising contacting a population of stem cells with at least one BMP pathway activator.
20. A method of increasing differentiated epithelial progenitor cell yield comprising contacting a population of stem cells with at least one BMP pathway activator.
21. A method of directing differentiation of a population of cells comprising contacting a population of stem cells with at least one BMP pathway activator.
22. The method of any of paragraphs 19-21, wherein the BMP pathway activator is selected from BMP2, BMP7, BMP9, GDF5, BMP2/6, BMP2/7, BMP4/7, or combinations thereof.
23. The method of any of paragraphs 19-22, wherein the population of stem cells comprises pancreatic bud cells.
24. The method of any of paragraphs 19-23, wherein the population of stem cells is contacted with the BMP pathway activator at stage 4 of a differentiation protocol.
25. The method of any of paragraphs 19-24, wherein the contacting of the population of stem cells with the BMP pathway activator at stage 4 decreases the number of CHGA⁺ cells by about two fold.
26. The method of any of paragraphs 19-25, wherein the contacting of the population of stem cells with the BMP pathway activator at stage 4 increases the number of PDX⁺NKX6.1⁺ progenitor cells by more than two fold.
27. The method of any of paragraphs 19-26, wherein the contacting of the population of stem cells with the BMP pathway activator at stage 4 increases the yield of epithelial progenitor cells.
28. The method of any of paragraphs 19-27, further comprising contacting the population of stem cells with a WNT pathway activator.

## Claims

1. A method of increasing differentiation of pluripotent cells into epithelial progenitor cells, the method comprising contacting a population of pancreatic bud cells with at least one BMP pathway activator.

2. A method of increasing differentiated epithelial progenitor cell yield comprising contacting a population of pancreatic bud cells with at least one BMP pathway activator.

3. A method of directing differentiation of a population of cells comprising contacting a population of pancreatic bud cells with at least one BMP pathway activator.

4. The method of any of claims 1-3, wherein the BMP pathway activator is selected from BMP2, BMP7, BMP9, GDF5, BMP2/6, BMP2/7, BMP4/7, or combinations thereof.

5. The method of any of claims 1-4, wherein the contacting of the population of pancreatic bud cells with the BMP pathway activator decreases the number of CHGA⁺ cells by about two fold.

6. The method of any of claims 1-5, wherein the contacting of the population of pancreatic bud cells with the BMP pathway activator increases the number of PDX1⁺NKX6.1⁺ progenitor cells by more than two fold.

7. The method of any of claims 1-6, wherein the contacting of the population of pancreatic bud cells with the BMP pathway activator increases the yield of epithelial progenitor cells.

8. The method of any of claims 1-7, further comprising contacting the population of pancreatic bud cells with a WNT pathway activator.

9. A composition comprising at least one BMP pathway activator and a plurality of PDX1⁺ cells.

10. The composition of claim 9, wherein the composition comprises a plurality of PDX1⁺NKX6.1⁺ cells.

11. The composition of claim 9 or claim 10, wherein the BMP pathway activator is selected from the group consisting of BMP2, BMP7, BMP9, GDF5, BMP2/6, BMP2/7, and BMP4/7.

12. The composition of any one of claims 9-11, wherein the composition further comprises Sant1, retinoic acid, or KGF;
wherein the composition further comprises Sant1 and retinoic acid;
wherein the composition further comprises retinoic acid and KGF; or
wherein the composition further comprises KGF and Sant1.

13. The composition of any one of claims 9-12, wherein the composition further comprises activin A.
